# EUROPEAN PATENT SPECIFICATION

(11) **EP 0 747 346 B1**
(45) Date of publication and mention of the grant of the patent: **16.02.2000**
(21) Application number: 96108764.0
(22) Date of filing: 31.05.1996
(51) Int. Cl.: C07C 233/18, C07C 233/20, C07C 233/60, C07C 233/43, C07C 275/24, A61K 31/165, A61K 31/75, C07C 233/23, C07C 233/13, C07C 233/05

(54) **N-acyl-2 aryl cyclopropylmethylamine derivatives as melatonergics**
N-Acyl-2-arylcyclopropylmethylamin-Derivate als melatonergisches Mittel
Dérivés de la N-acyl-2-aryl cyclopropylméthylamine comme agents mélatonergiques

(30) Priority: 07.06.1995 US 486633; 07.06.1995 US 487306
(43) Date of publication of application: 11.12.1996
(73) Proprietor: BRISTOL-MYERS SQUIBB COMPANY, Princeton, NJ 08543-4000 (US)
(72) Inventor: Keavy, Daniel J., Middletown, CT 06457 (US); Parker, Michael F., Somers, CT 06071 (US); Mattson, Ronald J., Meriden, CT 06450 (US); Johnson, Graham, Madison, CT 06443 (US)
(74) Representative: Josif, Albert, Dr.-Ing.

(56) References cited:
- EP-A- 0 420 064
- EP-A- 0 527 687
- EP-A- 0 706 994
- WO-A-92/09566
- WO-A-93/07111
- FR-A- 1 415 997

## Description

The invention pertains to novel N-acyl 2-arylcyclopropylmethylamine derivatives having drug and bio-affecting properties, to their preparation and to pharmaceutical formulations containing them. These compounds possess melatonergic properties that should make them useful in treating certain medical disorders. The invention therefore also pertains to the use of these compounds for manufacturing a medicament for treating a circadian rhythm - related disorder in a patient.

Melatonin (i; N-acetyl-5-methoxytryptamine) is a hormone which is synthesized and secreted primarily by the pineal gland. In mammals, melatonin levels show a cyclical, circadian pattern, with highest levels occurring during the dark period of a circadian light-dark cycle. Melatonin is involved in the transduction of photoperiodic information and appears to modulate a variety of neural and endocrine functions in vertebrates, including the regulation of reproduction, body weight and metabolism in photoperiodic mammals, the control of circadian rhythms and the modulation of retinal physiology.

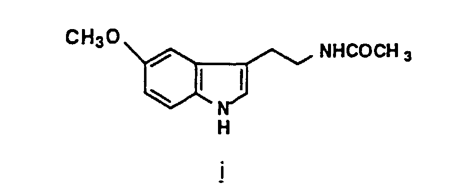

Recent evidence demonstrates that melatonin exerts its biological effects through specific receptors. Use of the biologically active, radiolabelled agonist [¹²⁵I]-2-iodomelatonin has led to the identification of high affinity melatonin receptors in the central nervous systems (CNS) of a variety of species. The sequence of one such high affinity melatonin receptor, cloned from frog melanocytes, has been reported. In mammalian brain, autoradiographic studies have localized the distribution of melatonin receptors to a few specific structures.

Although there are significant differences in melatonin receptor distribution even between closely related species, in general the highest binding site density occurs in discrete nuclei of the hypothalamus. In humans, specific [¹²⁵I]-2-iodomelatonin binding within the hypothalamus is completely localized to the suprachiasmatic nucleus, strongly suggesting that melatonin receptors are located within the human biological clock.

Exogenous melatonin administration has been found to synchronize circadian rhythms in rats (Cassone, et al., J. Biol. Rhythms,1:219-229, 1986). In humans, administration of melatonin has been used to treat jet-lag related sleep disturbances, considered to be caused by desynchronization of circadian rhythms (Arendt, et al., Br. Med. J. 292:1170, 1986). Further, the use of a single dose of melatonin to induce sleep in humans has been claimed by Wurtman in International Patent Application WO 94/07487.

Melatonin binding sites have been found in diverse tissues of the body-i.e., in the retina, superchiasmatic nucleus, spleen, etc. This means that melatonin exerts multiple physiological effects and is not highly selective. The potential for side effects with melatonin use is large. Melatonin agonists should be more selective than melatonin and have fewer side effects. Suitable melatonin agonists could overcome melatonin's drawbacks, resulting in products having more predictable and, possibly, sustained activity.

Melatonin agonists should be particularly useful for the treatment of chronobiological disorders. They would also be useful for the further study of melatonin receptor interactions as well as in the treatment of conditions affected by melatonin activity, such as depression, work-shift syndrome, sleep disorders, glaucoma, reproduction, cancer, immune disorders, neuroendoecine disorders, and a variety of sleep disorders.

Aside from simple indole derivatives of melatonin itself, various amide structures have been prepared and their use as melatonin ligands disclosed. In general these amide structures can be represented as: 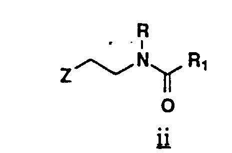 wherein Z is an aryl or heteroaryl system attached by a two carbon chain to the amide group. Some specific examples follow.

Yous, et al. in European Patent Application EPA A 527 687A A disclose, as melatonin ligands, ethylamines having cyclic substituents, 1, 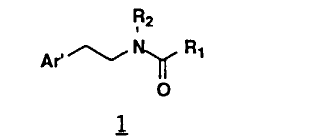 wherein Ar' is, inter alia, a substituted or unsubstituted benzo[b]thiophen-3-yl, benzimidazol-1-yl, benzo[b]furan-3-yl, 1,2-benzisoxazol-3-yl, 1,2-benzisothiazol-3-yl, or indazol-3-yl radical; R₁ is, inter alia, an alkyl or cycloalkyl group; and R₂ is hydrogen or lower alkyl.

Langlois, et al., in Australian Patent Application AU-A-48729/93 disclose arylalkyl(thio)amides 2 as melatonergic ligands, 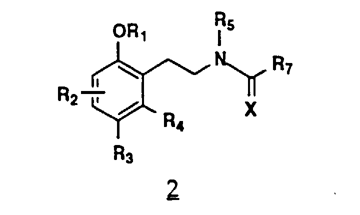 wherein R₁ is hydrogen or lower alkyl; R₂ is hydrogen, halogen, or lower alkyl; R₃ and R₄ are identical or different groups including, inter alia, hydrogen, halogen, or lower alkyl; R₅ is hydrogen or lower alkyl; X is sulfur or oxygen and R₇ is, inter alia, lower alkyl or alkenyl.

However, these references do not teach or suggest the novel melatonergic aryl cyclopropylmethylamine derivatives of the present invention.

A number of compounds containing structural elements common to the compounds of the present invention have been disclosed, although melatonergic properties have not been claimed for any of the compounds within these disclosures.

Matsuda, et al., in international patent application WO95/22521 disclose 1-phenyl-2-(1-aminoalkyl)-N,N-diethylcyclopropanecarboxaamides 3 as N-methyl-D-aspartate (NMDA) receptor antagonists, wherein R₁ represents, inter alia, a C₁-C₅ linear saturated aliphatic, a C₁-C₅ linear unsaturated aliphatic, a branched aliphatic, or a phenyl group which may be substituted with one to three substituents selected independently from the group consisting of halogen, C₁-C₄ alkyl, nitro, amino, hydroxy, and C₁-C₄ alkoxy.

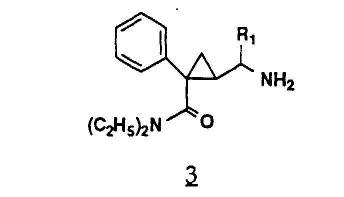

The 1,2-diarylcyclopropane derivatives of type 4 are disclosed in NE 6701256 as having CNS stimulant properties, 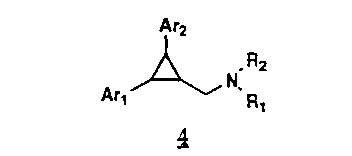 Ar₁ and Ar₂ are independently and optionally substituted phenyl; R₁ is inter alia hydrogen, lower alkyl or acyl; R₂ is inter alia alkyl, cycloalkyl or aralkyl.

The present invention is concerned with compounds of Formula I or II, which possess melatonergic properties and thus have utility in the treatment of conditions affected by melatonin activity: 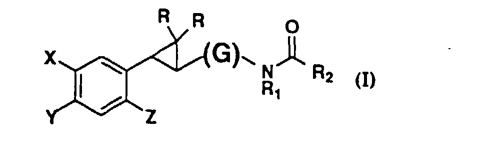 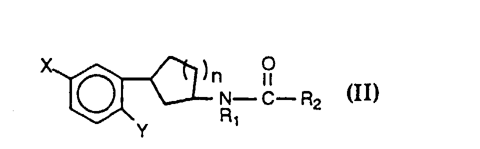 wherein:
X is halogen, hydrogen, C₁₋₄ alkyl or OR₅, wherein R₅ is hydrogen, C₁₋₄ perfluoroalkyl, C₁₋₄ fluoroalkyl, C₁₋₄perdeuteroalkyl, C₁₋₂₀ alkyl, C₄₋₂₀ cycloalkylalkyl, C₂₋₂₀ carbonitriloalkyl, C₃₋₂₂ alkoxycarbonylalkyl, C₃₋₂₀ alkenyl, C₃₋₂₀ alkynyl, optionally substituted C₉₋₂₀ phenylalkyl, optionally substituted C₉₋₂₀ phenylalkenyl, optionally substituted C₉₋₂₀ phenylalkynyl, C₂₋₂₀ hydroxyalkyl, optionally substituted C₈₋₂₀ phenyloxyalkyl, C₇₋₂₀ pyridylalkyl, or C₆₋₂₀ pyrrylalkyl wherein the optional substituents are on the phenyl ring and are selected from halogen, trifluoromethyl and C₁₋₄ alkoxy;
Y is hydrogen or halogen;
Z is hydrogen, halogen, cyano, optionally substituted phenyl, optionally substituted C₇₋₂₀ phenylalkyl, optionally substituted C₈₋₂₀ phenylalkynyl, or C₂₋₂₀ alkamido wherein the optional substituents are on the phenyl ring and are selected from halogen, trifluoromethyl and C₁₋₄ alkoxy;
R, in both cases, is hydrogen, halogen, or C₁₋₄ alkyl;
G is a divalent methylene, ethylene, or C₁₋₄ alkmethylene moiety;
R₁ is hydrogen, C₁₋₄ alkyl or benzyl;
R₂ is C₁₋₆ alkyl, C₂₋₆ alkenyl, C₃₋₆ cycloalkyl, C₂₋₄ alkoxyalkyl, C₁₋₄ trifluoromethylalkyl, C₂₋₈ alkylthioalkyl or NR₃R₄, wherein R₃ and R₄ are independently selected from hydrogen and C₁₋₄ alkyl, but R₃ and R₄ cannot both be hydrogen; and
n is 1 or 2.

This invention deals with compounds of Formula I or II, their preparation and compositions containing them for treating certain conditions.

Formulas I or II are: 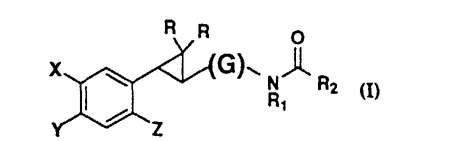 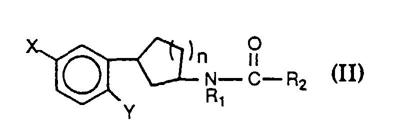 wherein:
X is halogen, hydrogen, C₁₋₄ alkyl or OR₅, wherein R₅ is hydrogen, C₁₋₄ perfluoroalkyl, C₁₋₄ fluoroalkyl, C₁₋₄ perdeuteroalkyl, C₁₋₂₀ alkyl, C₄₋₂₀ cycloalkylalkyl, C₂₋₂₀ carbonitriloalkyl, C₃₋₂₂ alkoxycarbonylalkyl, C₃₋₂₀ alkenyl, C₃₋₂₀ alkynyl, optionally substituted C₉₋₂₀ phenylalkyl, optionally substituted C₉₋₂₀ phenylalkenyl, optionally substituted C₉₋₂₀ phenylalkynyl, C₂₋₂₀ hydroxyalkyl, optionally substituted C₈₋₂₀ phenyloxyalkyl, C₇₋₂₀ pyridylalkyl, or C₆₋₂₀ pyrrylalkyl wherein the optional substituents are on the phenyl ring and are selected from halogen, trifluoromethyl and C₁₋₄ alkoxy;
Y is hydrogen or halogen;
Z is hydrogen, halogen, cyano, optionally substituted phenyl, optionally substituted C₇₋₂₀ phenylalkyl, optionally substituted C₈₋₂₀ phenylalkynyl, or C₂₋₂₀ alkamido wherein the optional substituents are on the phenyl ring and are selected from halogen, trifluoromethyl and C₁₋₄ alkoxy;
R, in both cases, is hydrogen, halogen, or C₁₋₄ alkyl;
G is a divalent methylene, ethylene, or C₁₋₄ alkmethylene moiety;
R₁ is hydrogen, C₁₋₄ alkyl or benzyl;
R₂ is C₁₋₆ alkyl, C₂₋₆ alkenyl, C₃₋₆ cycloalkyl, C₂₋₄ alkoxyalkyl, C₁₋₄ trifluoromethylalkyl, C₂₋₈ alkylthioalkyl or NR₃R₄, wherein R₃ and R₄ are independently selected from hydrogen and C₁₋₄ alkyl, but R₃ and R₄ cannot both be hydrogen; and
n is 1 or 2.

It is to be understood that, as used herein, "halogen" denotes fluorine, chlorine, bromine and iodine; the term "alkyl" refers to straight and branched chain saturated hydrocarbon radicals; "fluoroalkyl" refers to straight and branched chain monofluorosubstituted saturated hydrocarbon radicals; "alkenyl" refers to straight and branched hydrocarbon radicals containing a carbon-carbon double bond; "cycloalkyl" refers to saturated cyclic hydrocarbon radicals; "alkoxy" denotes an alkyl radical connected to a molecule via an oxygen atom; "alkylthioalkyl" refers to an alkyl radical linked to another via a sulfur atom; "phenyloxyalkyl" refers to an alkyl radical linked to an optionally substituted phenyl group via an oxygen atom; "cycloalkylalkyl" refers to an alkyl radical linked to a saturated cyclic hydrocarbon radical; "carbonitriloalkyl" refers to an alkyl radical linked to a nitrilo radical via a carbon atom; "alkoxycarbonylalkyl" refers to an alkyl radical linked directly to an alkoxycarbonyl radical; "hydroxyalkyl" refers to an alkyl radical linked to a hydrogen atom via an oxygen atom; "pyridylalkyl" refers to a pyridyl radical linked directly to the terminal carbon of an alkyl radical; "pyrrylalkyl " refers to a pyrryl radical linked directly to the terminal carbon of an alkyl radical; "trifluoromethylalkyl" refers to a trifluoro-substituted methyl group linked directly to an alkyl radical; "perfluoralkyl" refers to straight and branched chain saturated fluorocarbon radicals; "perdeuteroalkyl" refers to straight and branched chain saturated deuterocarbon radicals; "cyano" refers to a radical containing a carbon-nitrogen triple bond; "alkynyl" refers to straight and branched hydrocarbon radicals containing a carbon-carbon triple bond; "phenylalkyl", "phenylalkenyl", and "phenylalkynyl" refer to radicals in which an optionally substituted phenyl group is appended to the terminal carbon of an alkyl, alkenyl or alkynyl radical respectively; "alkamido" (or "alkylamido") refers to -NC(O)-alkyl groups containing the stated number of carbon atoms; "alkmethylene" (or "alkylmethylene") refers to an alkyl radical attached directly to a methene radical. "Benzyl" or "Bn" refers to the phenylmethyl group, -CH₂-phenyl. Phenyl groups, when present, bear substituents selected from hydrogen, halogen, trifluoromethyl and C₁₋₄ alkoxy moieties.

Numbers recited in subscripts immediately following "C" denote the number of carbon atoms in the moiety.

Based upon biological tests, the following Formula I compounds are preferred. All have binding affinities for the human melatonin receptor with IC₅₀ values of 600 nM or less.

Preferred compounds of formula I are those wherein R₁ is hydrogen, R₂ is C₁₋₄ alkyl, Y and Z are independently hydrogen or halogen, and X is OR₅, wherein R₅ is C₁₋₂₀ alkyl, C₃₋₂₀ alkenyl, C₃₋₂₀ alkynyl, and C₉₋₂₀ aralkyl, aralkenyl or aralkynyl. It is preferred that R₂ be devoid of O, N and S atoms. It is highly preferred that R₂ be C₁₋₄ alkyl.

Preferred compounds of the present invention include those in the following list:
(*trans*)-N-[[2-(3-Methoxyphenyl)cyclopropyl]methyl] butanamide;
(*trans*)-N-[[2-(3-Methoxyphenyl)cyclopropyl]methyl]-2-methoxyacetamide;
(+)-(*trans*)-N-[[2-(3-Methoxyphenyl)cyclopropyl]-methyl] butanamide;
(-)-(*trans*)-N-[[2-(3-Methoxyphenyl)cyclopropyl]methyl] butanamide;
(*trans*)-N-[[2-(3-Methoxyphenyl)cyclopropyl]methyl] propanamide;
(*trans*)-N-[[2-(4-Chloro-3-methoxyphenyl)cyclopropyl]methyl] butanamide;
(-)-(*trans*)-N-[[2-(3-Methoxyphenyl)cyclopropyl]methyl]-2-methylpropanamide;
(*trans*)-N-[[2-[3-(6-Phenylhexyl)oxy]phenyl]cyclopropyl]methyl] butanamide;
(*trans*)-N-[[2-(2-Brom-5-methoxyphenyl)cyclopropyl]methyl] butanamide;
(*trans*)-N-[[2-(3-Methoxyphenyl)cyclopropyl]methyl] acetamide;
(-)-(*trans*)-N-[[2-(2,4-Dibrom-5-methoxyphenyl)-cyclopropyl]methyl] butanamide;
(*trans*)-N-[[2-(2-Iodo-5-methoxyphenyl)cyclopropyl]methyl] butanamide;
(-)-(*trans*)-N-[[2-(2-Iodo-5-methoxyphenyl)cyclopropyl]methyl] butanamide;
(*trans*)-N-[[2-[3-(Heptyloxy)phenyl)cyclopropyl]methyl] butanamide;
(*trans*)-N-[[2-[3-(2-Propenyloxy)phenyl]cyclopropyl]methyl] butanamide;
(*trans*)-N-[[2-[3-[(7-Phenylheptyl)oxy]phenyl]cyclopropyl]methyl] butanamide;
(*trans*)-N-[[2-(3-[(Ethoxyphenyl)cyclopropyl]methyl] butanamide;
(*trans*)-N-[2-[(3-Octyloxy)phenyl]cyclopropyl]methyl] butanamide;
(*trans*)-N-[[2-[3-(Nonyloxy)phenyl]cyclopropyl]methyl] butanamide;
(*trans*)-N-[[2-[(3-Decyloxy)phenyl]cyclopropyl]methyl] butanamide;
(*trans*)-N-[[2-[(3-Undecyloxy)phenyl]cyclopropyl]methyl] butanamide;
(*trans*)-N-[[2-[(3-Dodecyloxy)phenyl]cyclopropyl]methyl] butanamide;
(*trans*)-N-[[2-[3-[(2-Phenylethyl)oxy]phenyl]cyclopropyl]methyl] butanamide;
(*trans*)-N-[[2-[3-[3-(3-Methoxyphenyl)propoxy]phenyl]cyclopropyl]methyl] butanamide;
(-)-(*trans*)-N-[[2-(5-Methoxy-2-(phenylethynyl)phenyl)cycloprop-1-yl]methyl] butanamide;
(*trans*)-N-[[3-(3-Methoxyphenyl)-2,2-difluoro-1-cycloprop-1-yl]methyl] butanamide;
(-)-(*trans*)-N-[[2-(3-Methoxyphenyl)cycloprop-1-yl]methyl]cyclopropane carboxamide;
(-)-(*trans*)-N-[[2-[3-[3-(3-Methoxyphenyl)propoxy]phenyl]cycloprop-1-yl)-methyl] butanamide;
(-)-(*trans*)-N-[[2-(3-Methoxyphenyl)cycloprop-1-yl]methyl]-N'-methyl urea;
(-)-(*trans*)-N-[[2-(3-Methoxyphenyl)cycloprop-1-yl]methyl] propanamide;
(-)-(*trans*)-N-[[2-(3-Methoxyphenyl)cycloprop-1-yl]methyl] acetamide;
(-)-(*trans*)-3,3,3-Trifluor-N-[[2-(3-methoxyphenyl)cycloprop-1-yl]methyl] propanamide;
(*trans*)-N-[[2-[3-[(3-,7,11-Trimethyldodeca-2,6,10-trien-1-yl)oxy]phenyl]cycloprop-1-yl]methyl] butanamide;
(*trans*)-N-[[2-[3-[(4-Phenylbut-1-yl)oxy]phenyl]cycloprop-1-yl]methyl] butanamide;
(*trans*)-N-[[2-[3-[(5-Phenylpent-1-yl)oxy]phenyl]cycloprop-1-yl]methyl] butanamide;
(*trans*)-N-[2-[(3-Trideuteromethoxyphenyl)cycloprop-1-yl]methyl] butanamide;
(*trans*)-N-[[2-[3-[(3-Cyclohexylprop-1-yl)oxy]phenyl]cycloprop-1-yl]methyl] butanamide;
(*trans*)-N-[[2-[3-[(3-Cyclopentylprop-1-yl)oxy]phenyl] cycloprop-1-yl]methyl] butanamide;
(*trans*)-N-[[2-[3-[2-[3-(Trifluoromethyl)phenyl]ethoxy]phenyl]cycloprop-1-yl]methyl] butanamide;
(*trans*)-N-[[2-[3-[[2-(3-Methoxyphenyl)cycloprop-1-yl]methoxy]phenyl] cycloprop-1-yl]methyl] butanamide;
(*trans*)-N-[[2-(4-Chloro-3-methoxyphenyl)cyclopropyl]methyl] butanamide;
(*trans*)-N-[[2-(3-Methoxyphenyl)cyclopropyl]methyl]-2-methylthioacetamide;
(*trans*)-N-[[2-[3-[3-(3-Methoxyphenyl)propoxy]phenyl]cyclopropyl]methyl] butanamide;
(-)-(*trans*)-N-[[2-(4-Iodo-3-methoxyphenyl)cycloprop-1-yl]methyl] butanamide;
(-)-(*trans*)-N-[[2-(5-Methoxy-2(phenylethyl)phenyl)cycloprop-1-yl]methyl] butanamide;
(-)-(*trans*)-N-[[2-(4-Methoxy[1,1'-biphenyl]-2yl-cycloprop-1-yl]methyl] butanamide;
(-)-(*trans*)-N-[[2-[4-Methoxy-4'-(trifluoromethyl) [1,1'-biphenyl]-2yl]cycloprop-1-yl]methyl] butanamide;
(*trans*)-N-[[2-(3-Bromophenyl)cycloprop-1-yl]methyl] butanamide;
(*trans*)-N-[[2-(3-Bromophenyl)cycloprop-1-yl]methyl] acetamide;
(*trans*)-N-[[2-(3-Bromophenyl)cycloprop-1-yl]methyl] propanamide;
(-)-(*trans*)-N-[[2-[2-Iodo-5-[3-(3-methoxyphenyl)propoxy]phenyl]cycloprop-1-yl) methyl] butanamide;
(*trans*)-N-[[2-[3[(3-Phenylprop-1-yl)oxy] phenyl]cycloprop-1-yl]methyl] butanamide;
(*trans*)-N-[[2-[3[(3-Phenoxyprop-1-yl)oxy] phenyl]cycloprop-1-yl]methyl] butanamide;
(*trans*)-N-[[2-[3[(3-Dimethylocta-2,6-dien-1-yl)oxy] phenyl] cycloprop-1-yl]methyl] butanamide;
(*trans*)-N-[[2-[3-(5-Methylhexyloxy)phenyl]cycloprop-1-yl]methyl] butanamide;
(*trans*)-N-[[2-[3-(4-Methyl-3-penten-1-yloxy)phenyl]cycloprop-1-yl]methyl] butanamide;
(*trans*)-N-[[2-[3-[(3-Cyclohexylbut-1-yl)oxy]phenyl]cycloprop-1-yl]methyl] butanamide;
(*trans*)-N-[[2-[3-[2-[2-(Trifluoromethyl)phenyl]ethoxy]phenyl]cycloprop-1-yl]methyl] butanamide;
(*trans*)-N-[[2-[3-[2-(3-Fluorophenyl) ethoxy]phenyl]cycloprop-1-yl]methyl] butanamide;
(*trans*)-N-[[2-[3-[3-(4-Methoxyphenyl)propoxy]phenyl]cycloprop-1-yl]methyl] butanamide;
(*trans*)-N-[[2-[3-[2-(2-Fluorophenyl) ethoxy] phenyl]cycloprop-1-yl]methyl] butanamide;
(*trans*)-N-[[2-[3-[2-(2-Methoxyphenyl) ethoxy] phenyl]cycloprop-1-yl]methyl] butanamide;
(-)-(*trans*)-N-[[2-(3-Fluorophenyl)cyclopropyl]methyl] butanamide;
(-)-(*trans*)-N-[[2-(3-Fluorophenyl)cycloprop-1-yl]methyl]-2-methylpropanamide;
(-)-(*trans*)-N-[[2-(2-Brom-5-fluorophenyl)cycloprop-1-yl]methyl] butanamide;
(-)-(trans)-N-[[2-(4-Bromo-3-fluorophenyl)cycloprop-1-yl]methyl] butanamide;
(-)-(*trans*)-N-[[2-(5-Fluor-2-iodophenyl)cycloprop-1-yl]methyl] butanamide;
(-)-(*trans*)-N-[[2-(3-Fluoro-4-iodophenyl)cycloprop-1-yl]methyl] butanamide;
(*trans*)-N-[[3-(3-Methoxyphenyl)-2,2-difluoro-1-cycloprop-1-yl]methyl] butanamide;
(*trans*)-N-[[3-(3-Methoxyphenyl)-2,2-difluoro-1-cycloprop-1-yl]methyl]-2-methylpropanamide;
(*trans*)-N-[[2-(3-Bromophenyl)cycloprop-1-yl]methyl] butanamide;
(*trans*)-N-[[2-(3-Bromophenyl)cycloprop-1-yl]methyl] acetamide;
(*trans*)-N-[[2-(3-Methylphenyl)cycloprop-1-yl]methyl] butanamide;
(*trans*)-N-[[2-(3-Bromophenyl)cycloprop-1-yl]methyl] propanamide;
(*trans*)-N-[[2-(3-Methylphenyl)cycloprop-1-yl]methyl]-2-methylpropanamide;
(*trans*)-N-[[2-(3-Methylphenyl)cycloprop-1-yl]methyl] acetamide;
(*trans*)-N-[[2-(3-Chlorophenyl)cycloprop-1-yl]methyl] butanamide;
(*trans*)-N-[[2-(3-Chlorophenyl)cycloprop-1-yl]methyl] propanamide;
(*trans*)-N-[[2-(3-Chlorophenyl)cycloprop-1-yl]methyl] acetamide;
(*trans*)-N-[[2-(3-Chlorophenyl)cycloprop-1-yl]methyl] cyclopropane carboxamide;
(*trans*)-N-[[2-(2,5-Difluorophenyl)cycloprop-1-yl]methyl] acetamide;
(*trans*)-N-[[2-(2,5-Difluorophenyl)cycloprop-1-yl]methyl] propanamide;
(*trans*)-N-[[2-(2,5-Difluorophenyl)cycloprop-1-yl]methyl] butanamide;
(*trans*)-N-[[2-(2,5-Difluorophenyl)cycloprop-1-yl]methyl] cyclopropane carboxamide;
(*trans*)-N-[[2-[3-(Pentafluoroethyl)phenyl]cycloprop-1-yl]methyl] butanamide;
(-)-(*trans*)-N-[(2-Phenylcycloprop-1-yl)methyl] butanamide;
(*trans*)-N-[(2-Phenylcycloprop-1-yl) methyl] acetamide;
(*trans*)-N-[(2-Phenylcycloprop-1-yl) methyl] butanamide;
(*trans*)-N-[2-[(3-Ethylphenyl) cycloprop-1-yl]methyl] acetamide;
(*trans*)-N-[2-[(3-Ethylphenyl) cycloprop-1-yl]methyl] propanamide;
(trans)-N-[2-[(3-Ethylphenyl) cycloprop-1-yl]methyl] butanamide;
(*trans*)-N-[2-[(3-Ethylphenyl) cycloprop-1-yl]methyl] cyclopropanecarboxamide;
(*trans*)-N-[[2-[3-(Trifluoromethyl)phenyl]cycloprop-1-yl]methyl] butanamide;
(*trans*)-N-[[2-[3-(Trifluoromethyl)phenyl]cycloprop-1-yl]methyl]-2-methylpropanamide;
(-)-(*trans*)-N-[[2-(2-Bromo-5-fluorophenyl)cycloprop-1-yl]methyl] butanamide;
(*trans*)-N-[[2-(3-Methylphenyl)cycloprop-1-yl]methyl] butanamide;
(*trans*)-N-[2-[(3-Ethylphenyl)cycloprop-1-yl]methyl] butanamide;
(cis)-N-[3-Methoxyphenyl)cyclohexyl]acetamide;
(cis)-N-[3-Methoxyphenyl)cyclohexyl]-2-methyl-propanamide;
(cis)-N-[3-Methoxyphenyl)cyclohexyl]butanamide;
(cis)-N-Ethyl-N'-[3-methoxyphenyl)cyclohexyl]urea; and
N-[3-Methoxyphenyl)cyclopentyl]butanamide.

### Preparation of Formula I Compounds

The compounds of Formula I can be prepared as depicted in the following schemes (Processes 1-8). The groups R, R₁, R₂,R₃,R₄, X, Y, Z, G and R₅ are as defined herein above.

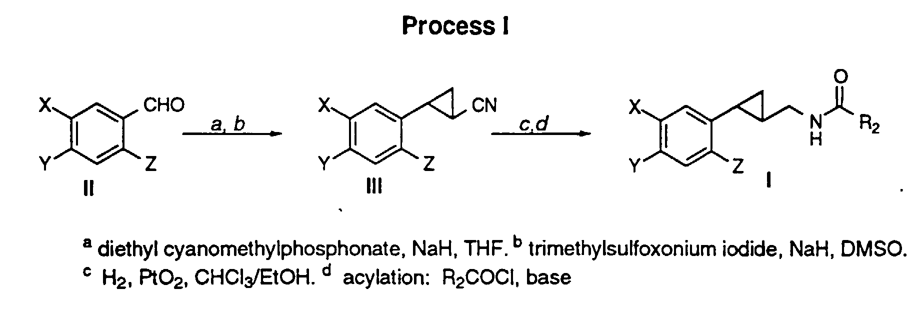

In the resultant Formula I compounds, R₁ and R are H, G is methylene, and the other substitutents are as defined above.

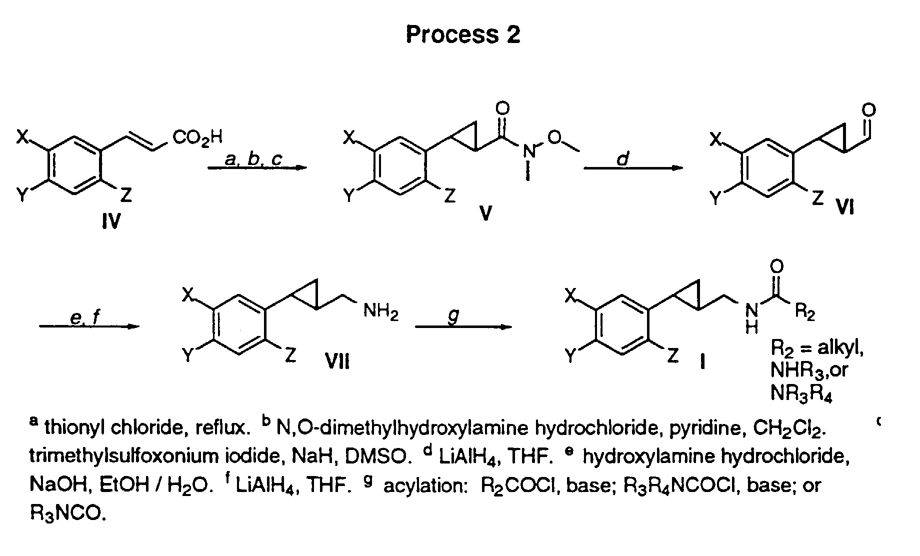

Using Process 2, the R₂ group in the final formula I compound is alkyl, NHR₃, or NR₃R₄ (as defined above). G is methylene; R₁ and R are H.

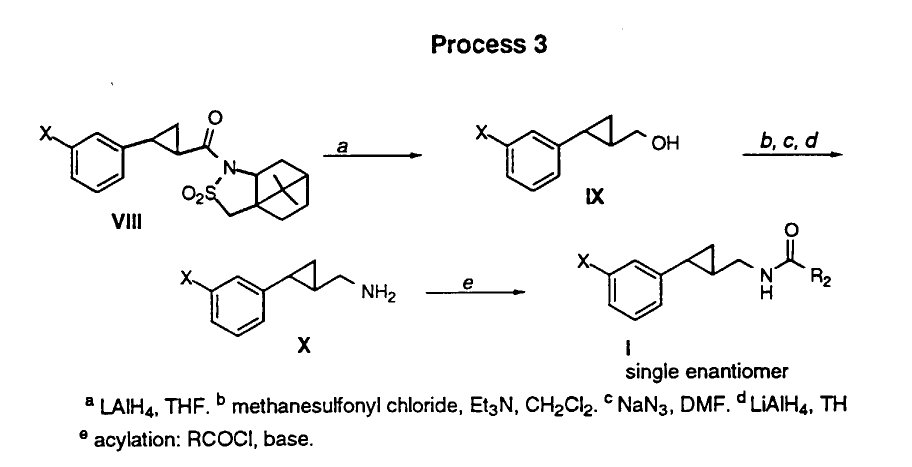

In Formula I compounds made using Process 3, R₁, R, Y and Z are all H, and G is methylene.

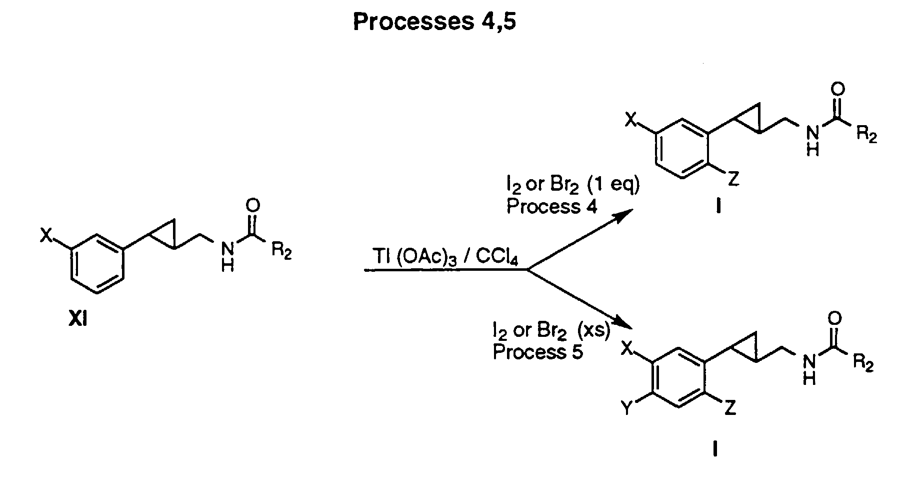

When using Process 4, the Formula I compounds made are those in which G is methylene; Y, R₁, and R are H, and Z is Br or I. Formula I compounds produced via Process 5 are those wherein Y and Z are both Br or I, G is methylene, and R₁ and R are H.

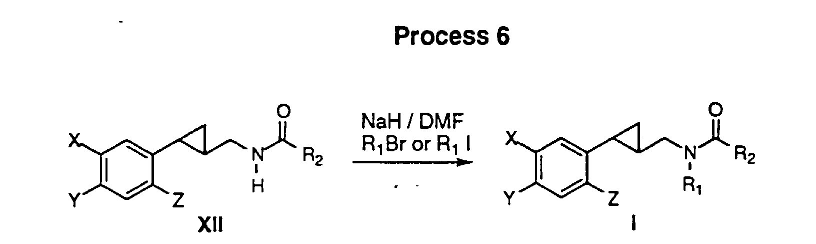

Via Process 6, the Formula I compounds made are those in which R₁ is C₁₋₄ alkyl or benzyl, G is methylene, R is H, and R₂, X, Y, Z are as defined above.

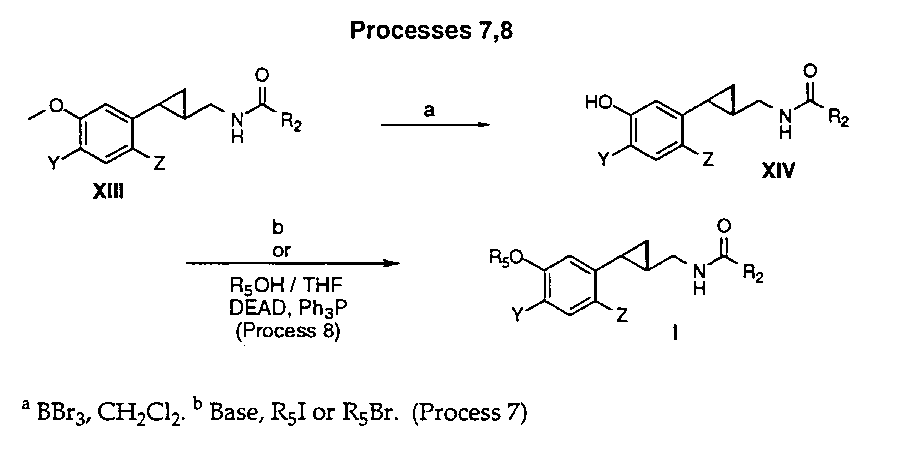

In Processes 7 and 8, the final Formula I compounds are those in which G is methylene, X is OR₅, R₁ and R are H, and Y, Z, R₂ and R₅ have the definitions given above.

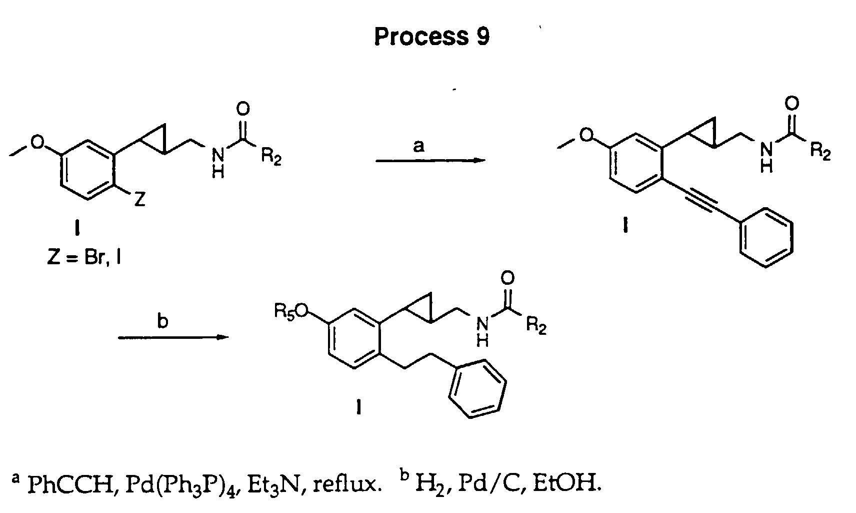

Via Process 9, the formula I compounds wherein X is OCH₃, G is methylene, Z is phenylalkyne or hydrocinnamyl, and R₁, R and Y are hydrogen are produced.

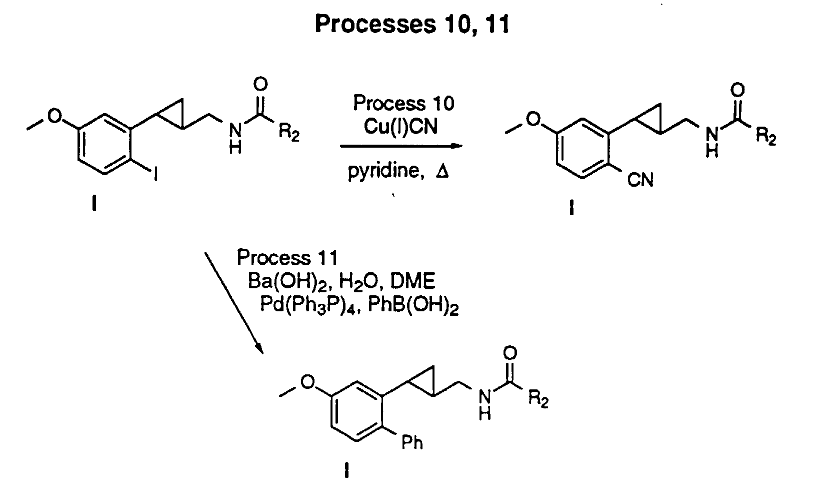

Via Process 10, the formula I compounds wherein X is OCH₃, G is methylene, Z is cyano, and R₁, R and Y are hydrogen are produced. Via Process 11, the formula I compounds wherein X is OCH₃, G is methylene, Z is phenyl, and R₁, R and Y are hydrogen are produced.

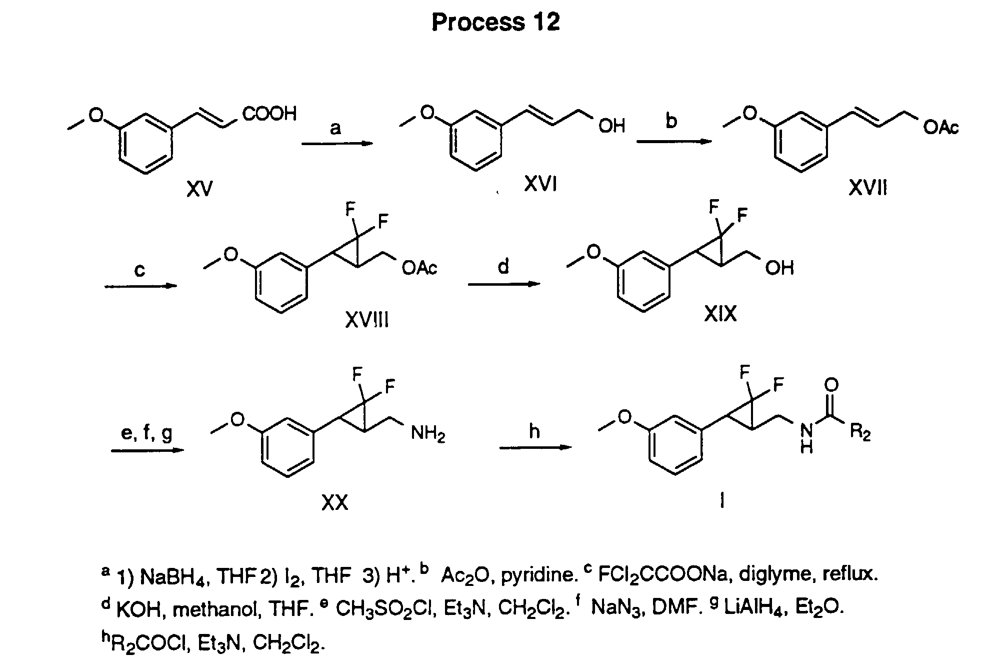

Via Process 12, the formula I compounds wherein X is OCH₃, R is fluorine, G is methylene, and Z, R₁, and Y are hydrogen are produced.

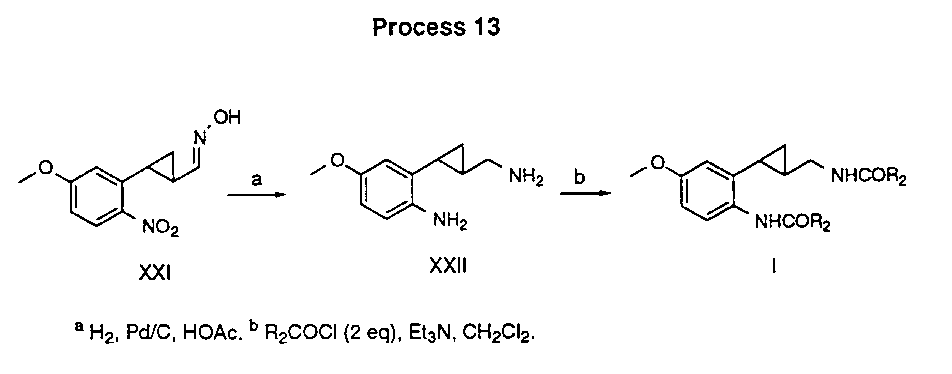

Via Process 13, the formula I compounds in which X is OCH₃, Z is alkamido, G is methylene, and R, R₁, and Y are hydrogen are produced.

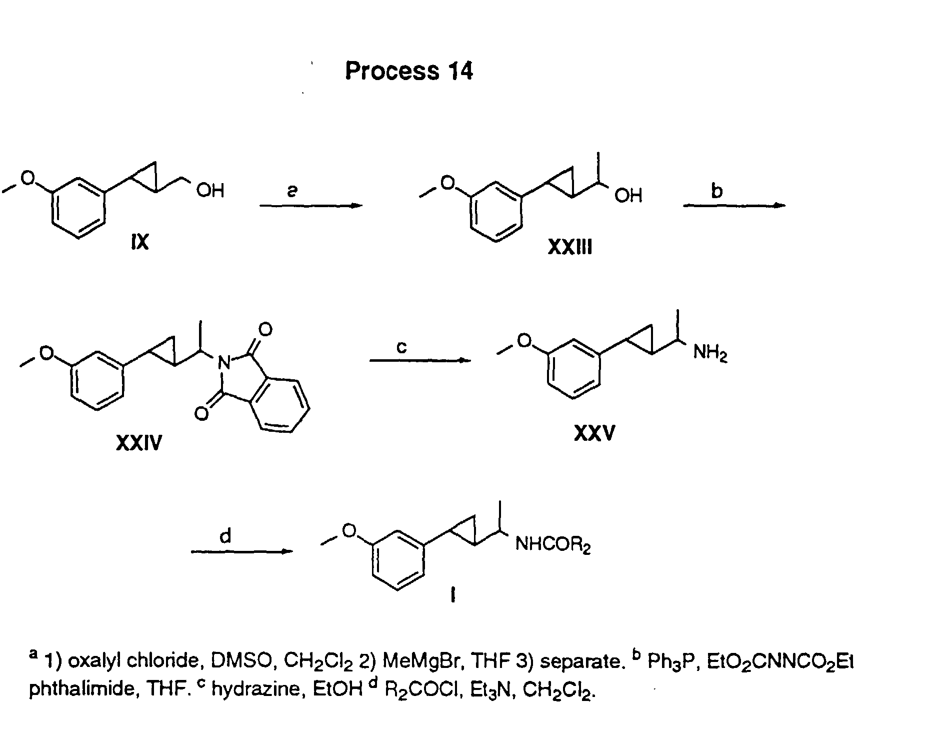

Via Process 14, the formula I compounds wherein X is OCH₃, G is alkmethylene, and R, R₁, Z, and Y are hydrogen are produced.

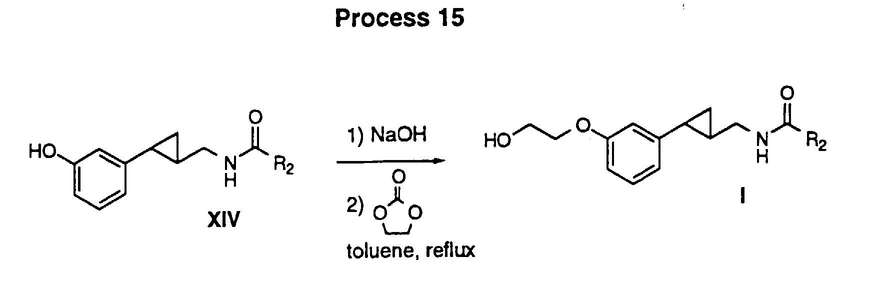

Via Process 15, the formula I compounds wherein X is 2-hydroxyethyl, G is methylene, and R, R₁, Z, and Y are hydrogen are produced.

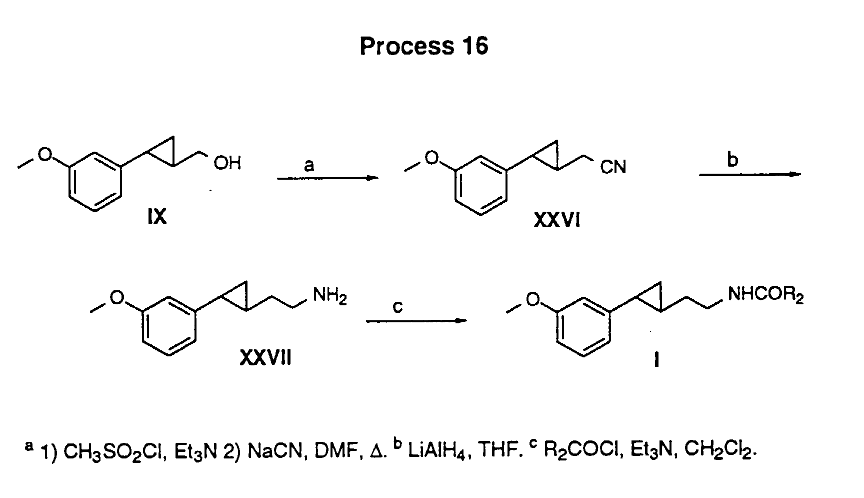

Via Process 16, the formula I compounds wherein X is OCH₃, G is ethylene, and R, R₁, Z, and Y are hydrogen are produced.

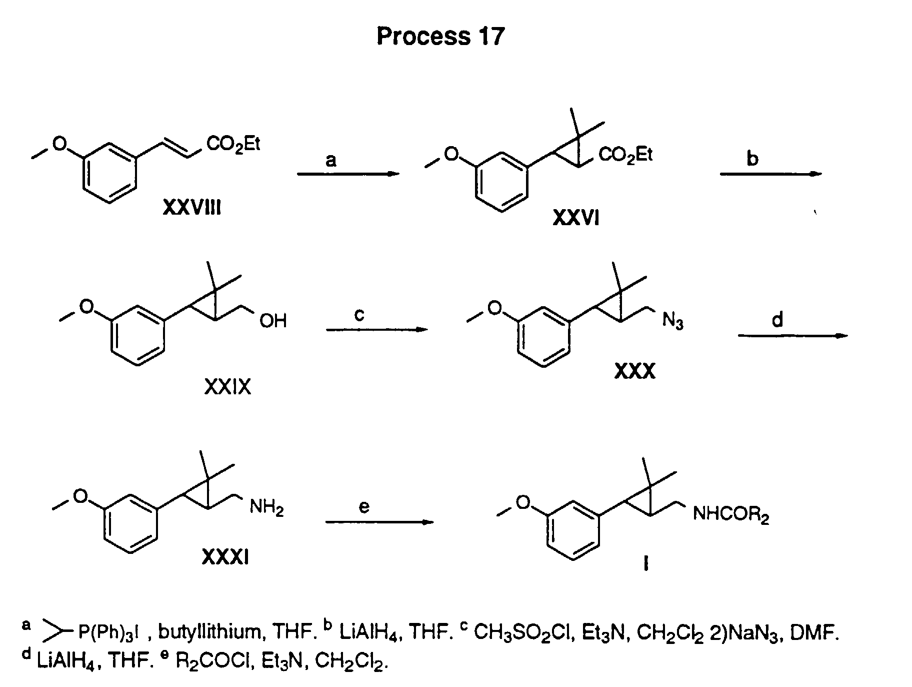

Via Process 17, the formula I compounds wherein X is OCH₃, G is methylene, R is methyl, and R₁, Z, and Y are hydrogen are produced.

### Preparation of Formula II Compounds

The compounds of Formula II were prepared by the method shown in Process 18. An organometallic reagent (**1**), such as a Grignard reagent or an aryl lithium reagent, is condensed with the appropriate cycloalkenone (**2**) in the presence of a copper(I) salt such as CuBr, CuI, CuBr•Me₂S, or the like, to give the 3-aryl-cycloalkanone (**3**) directly. Alternatively, **1** can be condensed with a 3-alkoxy-cycloalkenone (**4**), which gives **3** after subsequent ketalization, catalytic hydrogenation, and deprotection steps. Suitable catalysts for this conversion include palladium on carbon, and the like. Cycloalkanone, **3**, can be converted to the compounds of Formula I by standard methods, such as condensation with hydroxyamine, followed by catalytic hydrogenation, and subsequent acyclation Suitable catalysts for this conversion include Raney Nickel, palladium on carbon, and the like. Suitable acylating agents include carboxylic acid halides, anhydrides, acyl imidazoles, alkyl isocyanates, and carboxylic acids in the presence of condensing agents, such as carbonyl imidazole, carbodiimides, and the like.

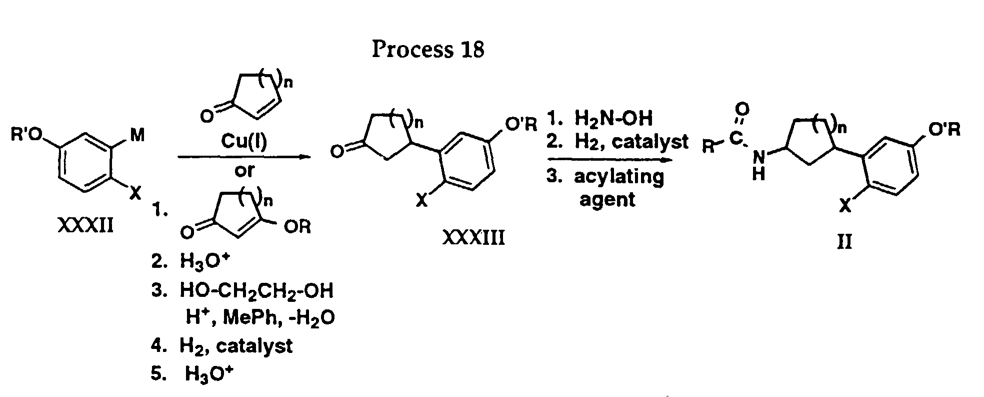

Unless stated otherwise, all substituents on the Formula I compounds produced via Processes 1-17 may have any of the definitions recited in the first discussion of Formula I herein.

The preparation of Compounds of Formula I involves the following steps described for each of Processes 1-17:

### Process 1 (Reactions a-d)

(i) Treatment of commercially available aldehydes of Formula II with sodium hydride and cyanomethylphosphonate, followed by cyclopropanation with dimethyloxosulphonium methylide gave intermediate cyclopropane nitriles of Formula III.
(ii) Catalytic reduction of nitriles III by hydrogen in the presence of platinum oxide, followed by acylation of the resultant amine with an appropriate acyl chloride, gave compounds of Formula I, wherein R₁ is hydrogen.

### Process 2 (Reactions a-g)

(i) Treatment of commercially available cinnamic acids of Formula IV with refluxing thionyl chloride, followed by acylation with N,O-dimethylhydroxylamine hydrochloride in the presence of pyridine gave unsaturated N-methyl,N-methoxy amides which were cyclopropanated with dimethyloxophosphonium methylide to give intermediates of Formula V.
(ii) Reduction of amides V with lithium aluminum hydride gave aldehydes of formula VI.
(iii) Aldehydes VI were converted to oximes with hydroxylamine hydrochloride and sodium hydroxide, and reduced with lithium aluminum hydride to give amines of Formula VII.
(iv) Compounds of formula VII were acylated with appropriate acylating agents, such as acyl chlorides, carbamoyl chlorides, or isocyanates to give compounds of Formula I, wherein R₁ is hydrogen, and R₂ is alkyl, NR₃R₄, or NHR₃ as defined previously.

### Process 3 (Reactions a-e)

(i) Reduction of the known homochiral sultams VIII with lithium aluminum hydride produced alcohols IX as single enantiomers.
(ii) Conversion of alcohols IX to the corresponding methanesulfonate esters with methanesulfonyl chloride and triethylamine, followed by azide formation with sodium azide in dimethylformamide, and lithium aluminum hydride reduction gave enantiopure amines of Formula X.
(iii) Amines X were acylated with appropriate acyl chlorides to give compounds of Formula I wherein R₁ is hydrogen and Y and Z are hydrogen.

### Process 4

Amides XI, produced by Processes I, II or III, were treated with thallium (III) acetate and 1 equivalent of bromine or iodine to give compounds of Formula I wherein Y is hydrogen, Z is bromine or iodine, and R₁ is hydrogen.

### Process 5

Amides XI, produced by Processes I, II or III, were treated with thallium (III) acetate and 2 or more equivalents of bromine or iodine to give compounds of Formula I wherein Y is equal to Z is equal to bromine or iodine, and R₁ is hydrogen.

### Process 6

Amides XII, produced by Processes I, II or III, were treated with sodium hydride and an appropriate alkyl or benzylhalide to give compounds of Formula I wherein R₁ is C₁₋₄ alkyl or benzyl.

### Process 7

(i) Amides XIII, produced by Processes I, II or III, were treated with boron tribromide to produce compounds of Formula XIV.
(ii) Amides XIV were treated with base and the resultant alkoxides were alkylated with appropriate alkyl iodides or alkyl bromides to produce compounds of Formula I wherein X is OR₅ and R₁ is hydrogen.

### Process 8

(i) Amides XIII, produced by Processes I, II or III, were treated with boron tribromide to produce compounds of Formula XIV.
(ii) Amides XIV were added to a prepared solution of an appropriate alcohol, triphenylphosphine, and diethyl azodicarboxylate to give compounds of Formula I wherein X is OR₅ and R₁ is hydrogen.

### Process 9

(i) Amides I, wherein Z is Br or I and produced by Process IV, were coupled with phenylacetylene under the aegis of tetrakis(triphenylphosphine) palladium(0) to give compounds of Formula I wherein Z is phenylacetylene.
(ii) Amides I, produced above, were hydrogenated over palladium on carbon catalyst to produce compounds of Formula I wherein Z is hydrocinnamyl.

### Process 10

(i) Amides I, wherein Z is I and produced by Process IV, were heated at reflux in pyridine in the presence of cuprous cyanide to produce compounds of Formula I wherein Z is cyano.

### Process 11

(i) Amides I, wherein Z is I and produced by Process IV, were subjected to Mitsonobu coupling in the presence of aqueous barium hydroxide, phenylboric acid, tetrakis(triphenylphosphine) palladium(0) and dimethoxyethane to produce Formula I compounds wherein Z is phenyl.

### Process 12 (Reactions a-h)

(i) Reduction via sodium borohydride of 3-methoxy cinnamic acid (XV), followed by sequential treatment with iodine and hydrochloric acid produced allylic alcohol XVI.
(ii) Compound XVI was acylated via acetic anhydride in pyridine to produce allylic acetate XVII.
(iii) Allylic acetate XVII was cyclopropanated via treatment with sodium chlorodifluoroacetate in refluxing diglyme to produce the difluorocyclopropane XVIII.
(iv) Difluorocyclopropane XVIII was treated with potassium hydroxide in methanol to give the corresponding alcohol XIX.
(v) Alcohol XIX was converted to a mesylate via treatment with methanesulfonyl chloride in dichloromethane in the presence of triethylamine. The mesylate was digested with dimethylformamide and treated with sodium azide to give the corresponding azide. The azide was reduced via lithium aluminum hydride to the amine XX.
(vi) Amine XX was converted to Amides I, wherein R is fluorine, by acylation with an acyl chloride in the presence of triethylamine.

### Process 13 (Reactions a,b)

(i) Oxime XXI, produced as in Process II, was converted to diamine XXII via catalytic hydrogenation over palladium on carbon in acetic acid.
(ii) Diamine XXII was diacylated via treatment with two equivalents of an acyl chloride in the presence of triethylamine to give Amides I, wherein Z is alkamido.

### Process 14 (Reactions a-d)

(i) Alcohol IX, produced via Process III, was oxidized via Swern oxidation to give the corresponding carboxaldehyde, which was treated with methyl magnesium bromide to give a mixture of diastereomers. The epimers were separated by chromatography to produce pure alcohols XXIII.
(ii) Alcohol XXIII was converted by treatment with phthalimide, diethylazo dicarboxylate, and triphenylphosphine to the phthalimide XXIV.
(iii) Phthalimide XXIV was treated with hydrazine in ethanol to give amine XXV.
(iv) Acylation of XXV was accomplished with an acyl chloride in the presence of triethylamine to give compounds of Formula I, wherein G is alkmethylene.

### Process 15

(i) Phenol XIV, produced via Process VII, was converted to the sodium salt by treatment with sodium hydroxide and heated at reflux with ethylene carbonate in toluene to give Amides I, wherein R₅ is 2-hydroxyethyl.

### Process 16 (Reactions a-c)

(i) Alcohol IX, produced via Process III, was treated with methanesulfonyl chloride in the presence of triethylamine to give a mesylate which was subsequently transformed, via treatment with sodium cyanide in dimethylformamide, to nitrile XXVI.
(ii) Nitrile XXVI was reduced with lithium aluminum hydride in tetrahydrofuran to give amine XXVII.
(iii) Amine XXVII was acylated with an acid chloride in the presence of triethylamine to give Amides I, wherein G is ethylene.

### Process 17 (Reactions a-e)

(i) Commercially available 3-methoxycinnamic acid ethyl ester was cyclopropanated with the ylide derived from isopropyl triphenylphosphonium iodide and butyllithium to give cyclopropane XXVI.
(ii) Cyclopropane XXVI was converted to alcohol XXIX via reduction with lithium aluminum hydride in tetrahydrofuran.
(iii) Alcohol XXIX was treated with methanesulfonyl chloride and triethylamine in dichloromethane to produce a mesylate which was subsequently converted to azide XXX by treatment with sodium azide in dimethylformamide.
(iv) Azide XXX was reduced via lithium aluminum hydride in tetrahydrofuran to amine XXXI.
(v) Amine XXXI was acylated with an acyl halide and triethylamine to give Amides I, wherein R is methyl.

Reagents, solvents and reaction conditions for the above described preparative steps would be known to one skilled in the art of organic synthesis. All steps are conventional organic reactions having extensive precedent in the scientific literature.

These preparative methods may be varied in order to produce other compounds embraced by this invention but not specifically disclosed.

Additionally compounds of Formula I also encompass all pharmaceutically acceptable solvates, with hydrates being the preferred solvates. The present invention also includes geometrical isomers as well as optical isomers, e.g. mixtures of enantiomers as well as individual enantiomers and diastereomers, which arise as a consequence of structural asymmetry in certain compounds of the instant series. *Trans-*cyclopropane stereoisomers are in general preferred. Separation or stereospecific synthesis of the individual isomers is accomplished by application of various methods which are well known to practitioners in the art.

In addition, the invention includes isotopically labeled variants of the disclosed compounds, particularly radioiodinated compounds into which a radioactive isotope of iodine, such as ¹²²I,¹²³I,¹²⁵I, or ¹³¹I, has been incorporated; such radiolabeled compounds are of utility as specific, high affinity receptors and thus can be employed in receptor binding assays, autoradiographic studies, and in other *in vitro* and *in vivo* biological tests which are used in the discovery and pharmacological characterization of novel melatonergic agents.

The "Description of Specific Embodiments" section hereinbelow provides greater descriptive details of the synthesis of compounds of Formula I and of intermediates of Formulas II - XXXI.

The compounds of the present invention have affinity for receptors of the endogenous pineal hormone, melatonin, as determined in a receptor binding assay, and exhibit agonist activity as determined by a functional assay. The biological tests are described below.

As is discussed above, melatonin is involved in the regulation of a variety of biological rhythms and exerts its biological effects via interaction with specific receptors. There is evidence that the administration of melatonin agonists is of clinical utility in the treatment of various conditions regulated by melatonin activity. Such conditions include depression, jet-lag, work-shift syndrome, sleep disorders, glaucoma, some disorders associated with reproduction, cancer, immune disorders and neuroendocrine disorders.

The systemic administration and dosing regimen of compounds of Formula I can be done in a manner similar to that described for melatonin itself. The dosage and dosage regimen must be adjusted using sound professional judgment and taking into consideration such variables as the age, body weight, sex and physical condition of the recipient, the route of administration and the nature of the illness being treated. Oral, transdermal, subcutaneous, intravenous, intramuscular, rectal, buccal, intranasal, and ocular routes of administration may be used.

One or more of the compounds of the invention is mixed with pharmaceutically acceptable amounts of one or more conventional pharmaceutical excipients to produce a formulation to be administered by the desired route. Generally, such formulations will contain one or several carriers or diluents. Useful carriers include solids, semi-solids and liquids which have miscibility, or other compatability, with the active agent(s) so that they can deliver same to a patient or host.

Suitable carriers include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, methyl cellulose, methyl- and propyl-hydroxybenzoates, talc, magnesium stearate, mineral oil and the like. Mixures are operable.

Other useful excipients include lubricants, wetting agents, gellants, emulsifiers, preservatives, colorants, perfumes, flavor enhancers, drying agents and the like. Mixtures can be employed.

Generally, compositions which include the compounds of the invention will contain from about 0.10 to about 10% of active compound(s) and 99.90 to 90%, or other suitable amounts, of excipient(s).

Dosage levels will be dictated by the patient's needs and by the medical judgment of the treating physician. Generally, however, dosages of about 0.1 mg to about 100 mg per day are useful to treat sleep, circadian rhythm or other medical disorders.

In methods of treatment employing the compounds of the invention, the treatment will involve the step(s) of administering one or more dosages of the compound to a host, preferably a mammalian, e.g. human host in need of such treatment.

### DESCRIPTION OF SPECIFIC EMBODIMENTS

The compounds which constitute the present invention, their methods of preparation and their biological actions will appear more fully after consideration of the following examples, which are given for the purpose of illustration only and are not to be construed as limiting the invention's scope.

In the following examples, temperatures are expressed in degrees Celsius (°C), hours are designated "h" or "hr", and melting points are uncorrected. The proton nuclear magnetic resonance (NMR) spectral characteristics refer to chemical shifts (δ) expressed as parts per million (ppm) versus tetramethylsilane (TMS) as the reference standard. The relative area reported for NMR signals at various chemical shifts corresponds to the number of hydrogen atoms of a particular type in the molecule. The multiplicities of the signals are reported as broad singlet (br s), singlet (s), doublet (d), triplet (t) or multiplet (m). The NMR spectra were obtained using solutions of the compounds in either deuterodimethylsulfoxide (DMSO-d₆) or deuterochloroform (CDCl₃). Infrared (IR) spectral descriptions include only absorption wave numbers (cm-1) having functional group identification value and IR determinations were made using potassium bromide (KBr) as diluent. The elemental analyses are reported as percent by weight.

The following examples describe in detail the preparation of representative examples of compounds of Formula I and of synthetic intermediates. It will be apparent to those skilled in the art that modifications, both of material and methods, will allow preparation of other compounds disclosed herein. From the foregoing description and the following examples it is believed that one skilled in the art would be able to carry out the invention to the fullest extent.

### EXAMPLES

### Examples 1-139 show the preparation and characterization of Formula I compounds. Examples 140-144 present similar information for Formula II compounds.

### Example 1 (Process 1)

### Preparation of (trans)-N-[[2-(2-Fluoro-5-methoxyphenyl)cyclopropyl]methyl]-2-methylpropanamide

### Step i

(a) **(*trans*)-3-(2-Fluoro-5-methoxyphenyl)-2-propenenitrile:** To a suspension of NaH (6.90 g, 60% dispersion in mineral oil, 173 mmol) in THF (500 mL) at 0 °C was added diethyl cyanomethylphosphonate (30.10 g, 170 mmol) dropwise. This was followed by the dropwise addition of a solution of 2-fluoro-5-methoxybenzaldehyde(24.5 g, 159 mmol) in THF (50 mL). The resulting suspension was allowed to warm to ambient temperature. After 18 h, H₂O (200 mL) was added and the solution was extracted with EtOAc. The organics were combined, washed with brine, dried over MgSO₄, and concentrated to afford a white waxy solid. The material was then purified by Kügelrohr distillation to give the title compound, 24.3 g (86%): mp 56-57 °C; ¹H NMR (300 MHz, CDCl₃) δ 3.79 (s, 3H), 5.98 (d, *J* = 16.1 Hz, 1H), 6.85-6.90 (m, 1H), 6.91-6.93 (m, 1H), 6.99 (t, *J* = 6.5 Hz, 1H), 7.43 (d, *J* = 16.1 Hz, 1H); MS (isobutane - DCI) *m/e* 177; Analysis calc'd for C₁₀H₈NOF: C, 67.79; H, 4.55; N, 7.91; found: C, 67.41; H, 4.44; N, 7.86.
(b) **(*trans*)-2-(2-Fluoro-5-methoxyphenyl)cyclopropane carbonitrile:** To a suspension of NaH (1.73 g, 72 mmol) in DMSO (40 mL) was added solid trimethylsulfoxonium iodide (15.9 g, 72 mmol) in small portions. After the foaming had subsided (40 min), a solution of (*trans*)-3-(2-fluoro-5-methoxyphenyl)-2-propenenitrile (4.26 g, 24 mmol) in DMSO (10 mL) was added dropwise, maintaining the temperature between 35-40 °C. Stirring was continued for 18 h at room temperature, followed by the dropwise addition of saturated NH₄Cl (100 mL) and extraction with ethyl acetate. The organics were combined, washed with brine, dried (K₂CO₃), and concentrated to give a red oil which was then purified by silica gel chromatography (CH₂Cl₂/hexane ,60:40) to afford the title compound as a clear oil (46% yield): ¹H NMR (300 MHz, CDCl₃) δ 0.82-0.88 (m, 2H), 1.65-1.75 (m, 1H), 2.55-2.62 (m, 1H), 3.75 (s, 3H), 6.50-6.55 (m, 1H), 6.60-6.70 (m, 1H), 6.92 (t,*J*=6.5 Hz, 1H).

### Step ii

(c) **(*trans*)-2-(2-Fluoro-5-methoxyphenyl)cyclopropanemethanamine:**
   A suspension of (*trans*)-2-(2-fluoro-5-methoxyphenyl)cyclopropane carbonitrile (5.0 g, 26 mmol), PtO₂ (200 mg), and CHCl₃ (10 mL) in EtOH (65 mL) was hydrogenated in a Parr apparatus at 55 psi for 3 h. The catalyst was filtered through a plug of celite and the solvents were removed. The resulting hydrochloride salt was then partitioned between CH₂Cl₂ and 10% K₂CO₃. The organic layer was separated, dried over K₂CO₃, and concentrated to afford the title compound as the free amine, 3.8 g (74%): ¹H NMR (250 MHz, CDCl₃) δ 0.79-0.82 (m, 1H), 0.90-0.95 (m, 1H), 1.49 (br s, 2H), 1.70-1.76 (m, 1H), 1.82-1.86 (m, 1H), 2.60-2.74 (m, 2H), 3.75 (s, 3H), 6.35-6.42 (m, 1H), 6.50-6.62 (m, 1H), 6.85-6.90 (m, 1H).
(d) **(*trans*)-N-[[2-(2-Fluoro-5-methoxyphenyl)cyclopropyl]methyl]-2-methylpropanamide:**
   To a magnetically stirred solution of (*trans*)-2-(2-fluoro-5-methoxyphenyl)cyclopropanemethanamine(600 mg, 3.1 mmol), Et₃N (909 mg, 9.0 mmol), in dry dichloromethane (15 mL) at 0 °C was added isobutyryl chloride (352 mg, 3.3 mmol) dropwise. The resulting suspension was then allowed to warm to room temperature and stirred for 18 h. The solvents were removed and the residue was taken into EtOAc (100mL) and washed sequentially with H₂O, 5% citric acid, 5% K₂CO₃, brine, and dried over K₂CO₃. Concentration by rotary evaporation yielded a crude oil which was then purified by Kügelrohr distillation. Recrystallization of the resultant solid from Et₂O/hexane (1:1) gave 380 mg (47% yield) of the title compound: mp 93-94 °C; ¹H NMR (300 MHz, CDCl₃) δ 0.82-0.88 (m, 1H), 0.97-1.03 (m, 1H), 1.13 (d, *J* = 8.1 Hz, 6H), 1.14-1.24 (m, 1H), 1.85-1.91 (m, 1H), 2.25-2.30 (m, 1H), 2.93-3.02 (m, 1H), 3.52 (dt, *J* = 7.8, 13.8 Hz, 1H), 3.72 (s, 3H), 5.74 (br s, 1H), 6.41-6.44 (m, 1H), 6.59-6.64 (m, 1H), 6.91 (t, *J* = 6.5 Hz, 1H); IR (NaCl Film) 3298, 2962, 1642, 1546, 1502, 1428 cm⁻¹; MS (isobutane - DCI) *m/e* 253; Analysis calc'd for C₁₅H₂₀NO₂F: C, 67.90; H, 7.60; N, 5.28; found: C, 68.18; H, 7.77; N, 5.24.

### Example 2 (Process 2)

### Preparation of (trans)-N-[[2-(3-Methoxyphenyl)cyclopropyl]methyl] butanamide

### Step i

(a)(b) **(*trans*)-N-Methoxy-N-methyl-3-(3-methoxyphenyl)-2-propenamide:**
   A solution of 3-methoxycinnamic acid (75.0 g, 0.42 mol) in thionyl chloride (250 mL) was heated at reflux for 1 h. The majority of the the thionyl chloride was then distilled off and the resulting residue was treated with dichloromethane and residual thionyl chloride was removed by codistillation to give 3-methoxycinnamic acid chloride of sufficient purity to be used without further purification. Pyridine (186 mL, 2.3 mol) and N,O-dimethylhydroxylamine hydrochloride (45.17 g, 0.46 mol) were added to a 0 °C solution of 3-methoxycinnamic acid chloride(0.42 mol) in dry dichloromethane (500 mL). The solution was stirred for 18 h at room temperature, diluted with dichloromethane (200 mL), and washed sequentially with 1N HCl, saturated sodium bicarbonate, and brine. It was then dried over MgSO₄ and concentrated in vacuo to give the title compound as a dark oil (88.43 g, 95%): ¹H NMR (300 MHz, CDCl₃) δ 3.25 (s, 3H), 3.68 (s, 3H), 3.84 (s, 3H), 6.82-6.86 (m, 1H), 7.00 (d, *J* = 16.1 Hz, 1H), 7.10 (br s, 1H), 7.15-7.20 (m, 1H), 7.32 (t, *J* = 6.6 Hz, 1H), 7.72 (d, *J* = 16.0 Hz, 1H).
(c) **(*trans*)-N-Methoxy-N-methyl-2-(3-methoxyphenyl) cyclopropanecarboxamide:** To a suspension of NaH (16.27 g, 0.68 mol) in DMSO (375 mL) was added solid trimethylsulfoxonium iodide (149.16 g, 0.68 mol) in small portions. After the foaming had subsided (40 min), a solution of (*trans*)-N-methoxy-N-methyl-3-(3-methoxyphenyl)-2-propenamide (50 g, 0.23 mol) in DMSO (50 mL) was added dropwise, maintaining the temperature between 35-40 °C. Stirring was continued for 18 h at room temperature, followed by the dropwise addition of saturated NH₄Cl (400 mL) and extraction with ethyl acetate. The organics were combined, washed with brine, dried (K₂CO₃), and concentrated to give a red oil which was then purified by Kügelrohr distillation (130 °C, 0.5 mm Hg) to afford the title compound as a white wax (52.47 g, 100%): ¹H NMR (300 MHz, CDCl₃) δ 0.82-0.88 (m, 1H), 1.20-1.30 (m, 1H), 1.59-1.62 (m, 1H), 2.40-2.45 (m, 1H), 3.22 (s, 3H), 3.68 (s, 3H), 3.79 (s, 3H), 6.61-6.72 (m, 3H), 7.18 (t, *J* = 6.0 Hz, 1H).

### Step ii

(d) **(*trans*)-2-(3-Methoxyphenyl)cyclopropanecarboxaldehyde:** To a rapidly stirred suspension of LiAlH₄ (7.74 g, 204 mmol) in THF (800 mL) at -45 °C was added a solution of the (*trans*)-N-methoxy -N- methyl-2-(3-methoxyphenyl)cyclopropanecarboxamide (40 g, 171 mmol) in THF (100 mL) maintaining the temperature below -40 °C by dropwise addition. After addition the cooling bath was removed and the reaction was allowed to warm to 5 °C, then immediately recooled to -45 °C. Potassium hydrogen sulfate (40 g, 300 mmol) in H₂O (120 mL) was cautiously added dropwise, the temperature maintained below -30 °C throughout. After addition the cooling bath was removed and the suspension was stirred at room temperature for 30 min. The mixture was filtered through celite and the filter cake was washed with ether. The combined filtrates were then washed with cold 1N HCl, 5% K₂CO₃, brine , and dried over MgSO₄. Concentration by rotary evaporation yielded the title compound as a clear oil (29.9 g, 99%): ¹H NMR (300 MHz , CDCl₃) δ 1.49-1.58 (m, 1H), 1.65-1.73 (m, 1H), 2.10-2.19 (m, 1H), 2.58-2.67 (m, 1H), 3.80 (s, 3H), 6.66-6.78 (m, 3H), 7.21 (t, *J* = 6.6 Hz, 1H), 9.32 (d, *J*=2.0 Hz, 1H).

### Step iii

(e)(f) **(*trans*)-[2-(3-Methoxyphenyl)cyclopropanemethanamine:**
A solution of (*trans*)-2-(3-methoxyphenyl)cyclopropanecarboxaldehyde (31.0 g, 176 mmol), hydroxylamine hydrochloride (38.57 g, 555 mmol), ethanol (200 mL), water (120 mL), and 10 N NaOH (55 mL, 555 mmol) was heated at reflux (steam bath) for 18 h. The solution was cooled to room temperature, diluted with water (1 L), washed sequentially with 1 N HCl, H₂O, brine, and dried over K₂CO₃. Concentration by rotary evaporation produced 30.98 g (92%) of the oxime which was used directly in the next step. The oxime (30.98 g, 162 mmol) was digested with THF (50 mL) and added dropwise to a -45 °C suspension of LiAlH₄ (9.2 g, 242 mmol), in THF (300 mL) maintaining the temperature below -40 °C. The reaction was then allowed to come to ambient temperature, stirred for 4 h, and recooled to -45 °C. Potassium hydrogen sulfate (55 g, 404 mmol) in H₂O (200 mL) was then cautiously added dropwise. The cooling bath was removed and the suspension was stirred at room temperature for 30 min. The resulting paste was then filtered through celite, and the filter cake was washed with Et₂O. The combined filtrates were extracted with 1N HCl, the acid extracts were made basic (50% NaOH), and then extracted with dichloromethane. The organics were combined, washed with brine, dried (K₂CO₃), and concentrated in vacuo to give the title compound as a clear oil (17.62 g, 56%, two steps): ¹H NMR (300 MHz , CDCl₃) δ 0.80-0.95 (m, 2H), 1.31-1.35 (m, 1H), 1.70-1.79 (m, 1H), 2.28 (br s, 2H), 2.70-2.74 (m, 2H), 3.79 (s, 3H), 6.60-6.71 (m, 3H), 7.17 (t, *J* = 6.4 Hz, 1H).

### Step iv

(g) **(*trans*)-N-[[2-(3-Methoxyphenyl)cyclopropyl]methyl] butanamide:** To a magnetically stirred solution of (*trans*)-[2-(3-methoxyphenyl)cyclopropanemethanamine (1.02 g, 5.8 mmol) and Et₃N (1.60 mL, 11.5 mmol) in dry dichloromethane (20 mL) at 0 °C was added butyryl chloride (0.69 g, 6.4 mmol) dropwise. The resulting suspension was allowed to warm to room temperature and stirred for 4 h. The solvents were removed and the residue was digested with EtOAc (100mL) and washed sequentially with H₂O, 5% citric acid, 5% K₂CO₃, brine, and dried over K₂CO₃. Concentration by rotary evaporation yielded a crude product which was then purified by flash chromatography (silica gel, 40% EtOAc/hexane) to afford 1.01 g (71%) of the title compound: ¹H NMR (300 MHz, CDCl₃) δ 0.85-0.95 (m, 5H), 1.22-1.31 (m, 1H), 1.62 (q,*J* = 7.1 Hz, 2H), 1.73-1.77 (m, 1H), 2.13 (t, *J* = 7.7 Hz, 2H), 3.15-3.36 (m, 2H), 3.76 (s, 3H), 5.59 (s, 1H), 6.55-6.69 (m, 3H), 7.15 (t, *J* = 6.5 Hz, 1H); IR (NaCl Film) 3302, 2862, 1732, 1644, 1464 cm⁻¹; MS (isobutane - DCI) *m/e* 247; Analysis calc'd for C₁₅H₂₁NO₂: C, 72.84; H, 8.56; N, 5.66; found: C, 72.71; H, 8.50; N, 5.62.

### Example 3 (Process 3)

### 3a. Preparation of (+)-(trans)-N-[[2-(3-Methoxyphenyl)cyclopropyl]methyl]butanamide

### Step i

(a) **(+)-(*trans*)-2-(3-Methoxyphenyl)cyclopropanemethanol :** To a suspension of LAH (0.65 g, 17.1 mmol) in THF (45 mL) at -45 °C was added a solution of (2'S)-N-(1S,2S)-2-(3-methoxyphenyl)cyclopropanecarbonyl] bornane-10',2'-sultam (3.36 g, 8.6 mmol), prepared according to the method of Vallgårda, J.; Appelberg, U.; Csöregh, I.; and Hacksell, U. (*J. Chem. Soc. Perkin Trans. I*, pages 461-470, **1994**), in THF (20 mL) dropwise. The cooling bath was then removed and the reaction was allowed to warm to room temperature, followed by immediate recooling to -45 °C. Potassium hydrogen sulfate (3.9 g, 29 mmol) in H₂O (15 mL) was cautiously added, allowing the temperature to rise to -5 °C. The resulting paste was stirred at room temperature for 1 h, filtered through celite, and the filter cake was washed with Et₂O. The combined filtrates were washed with cold (0 °C) 1 N HCl, 5% K₂CO₃, brine, dried (K₂CO₃), and concentrated to give a crude wax. Trituration with hexane, filtration of the precipitated chiral auxiliary, and subsequent concentration of the filtrate afforded the title compound as a clear oil (1.33 g, 87%): ¹H NMR (300 MHz , CDCl₃) δ 0.90-1.00 (m, 2H), 1.21-1.25 (m, 1H), 1.52-1.59 (br s, 1H), 1.78-1.85 (m, 1H), 3.60 (dd, *J* = 5.0, <1 Hz, 2H), 3.80 (s, 3H), 6.60-6.72 (m, 3H), 7.20 (t, *J* = 7.5 Hz, 1H); [a]_{D}²⁰ 55.5 °C (c=1, CH₂Cl₂).

### Step ii

(b)(c) **(+)-(*trans*)-1-(Azidomethyl)-2-(3-methoxyphenyl)cyclopropane:** To a solution of (+)-(*trans*)-2-(3-methoxyphenyl)cyclopropanemethanol (1.33 g, 7.5 mmol) and triethylamine (1.57 mL, 11.3 mmol) in dichloromethane (25 mL) at 0 °C was added methanesulfonyl chloride (950 mg, 8.3 mmol) dropwise. After addition was complete the ice bath was removed and stirring was continued for 30 min. The solution was treated with dichloromethane (100 mL), washed with H₂O, saturated NaHCO₃, and dried over K₂CO₃. Concentration by rotary evaporation gave 1.78 g of a crude oil. The mesylate was taken into DMF (25 mL), treated with sodium azide (980 mg, 15 mmol) and allowed to stir at room temperature for 18 h. The mixture was concentrated and the residue was taken into Et₂O, washed with H₂O, brine, and the solvents removed by rotary evaporation to produce 1.11 g (73%, two steps) of a clear oil: ¹H NMR (300 MHz , CDCl₃) δ 0.85-0.92 (m, 2H), 1.41-1.45 (m, 1H), 1.80-1.83 (m, 1H), 3.25-3.38 (m, 1H), 3.48-3.58 (m, 1H), 3.83 (s, 3H), 6.70-6.78 (m, 3H), 7.21 (t, *J* = 7.8 Hz, 1H).
(d) **(+)-(*trans*)-2-(3-Methoxyphenyl)cyclopropanemethanamine:** To a stirred suspension of LAlH₄ (455 mg, 12 mmol) in THF (20 mL) at -30 °C was added a solution of (+)-(*trans*)-1-(azidomethyl)-2-(3-methoxyphenyl)cyclopropane (1.11 g, 5.5 mmol) in THF (10 mL). The temperature was maintained below -30 °C, throughout. After addition was complete the suspension was allowed to warm to room temperature and stirred for 4 h. The reaction was cooled to -45 °C and a solution of KHSO₄ (2.6 g) in H₂O (20mL) was cautiously added dropwise. The suspension was stirred at room temperature for 30 min, filtered through celite, and the filter cake was washed well with Et₂O. The filtrates were combined, extracted with 1N HCl and the acidic layers were basified (30%NaOH). The basic solution was extracted with CH₂Cl₂, dried (K₂CO₃), and concentrated to afford 400 mg (41%) of the title compound as a clear oil: ¹H NMR (300 MHz, CDCl₃) δ 0.86-0.95 (m, 2H), 1.24-1.31 (m, 1H), 1.72-1.79 (m, 1H), 2.10-2.40 (br s, 2H), 2.70-2.74 (m, 2H), 3.79 (s, 3H), 6.60-6.72 (m, 3H), 7.15 (t, *J* = 7.8 Hz, 1H).

### Step iii

(e) **(+)-(*trans*)-N-[[2-(3-Methoxyphenyl)cyclopropyl]methyl] butanamide:** To a magnetically stirred solution of (+)-(*trans*)-2-(3-methoxyphenyl)cyclopropanemethanamine(400 mg, 2.3 mmol) and Et₃N (1.1 mL, 7.9 mmol) in dry dichloromethane (15 mL) at 0 °C was added butyryl chloride (269 mg, 2.5 mmol) dropwise. The resulting suspension was allowed to come to room temperature and stirred for 18 h. The solvents were removed and the residue was digested with EtOAc (100mL) and washed sequentially with H₂O, 5% citric acid, 5% K₂CO₃, brine, and dried over K₂CO₃. Concentration by rotary evaporation yielded a crude product which was then purified by flash chromatography (silica gel, 1% MeOH/CH₂Cl₂) to afford 310 mg (56% ) of the title compound as a clear oil: ¹H NMR (300 MHz, CDCl₃) δ 0.86-0.95 (m, 5H), 1.22-1.31 (m, 1H), 1.54-1.83 (m, 3H), 2.08 (t, *J* = 7.7 Hz, 2H), 3.16-3.36 (m, 2H), 3.76 (s, 3H), 5.59 (br s, 1H), 6.55-6.76 (m, 3H), 7.17 (t, *J* = 7.8 Hz, 1H); IR (NaCl Film) 3296, 2962, 1644, 1604, 1550, 1494 cm⁻¹; MS (isobutane - DCI) *m/e* 247; [a]_{D}²⁰ +57.2 ° (c=1, CH₂Cl₂); Analysis calc'd for C₁₅H₂₁NO₂·0.15 H₂O: C, 72.05; H, 8.59; N, 5.60; found: C, 71.97; H, 8.59; N, 5.60.
3b. **(-)-(*trans*)-N-[[2-(3-Methoxyphenyl)cyclopropyl]methyl] butanamide:** The title compound was synthesized by the above method from (2'R)-N-(1R,2R)-2-(3-methoxyphenyl)cyclopropanecarbonyl] bornane-10',2'-sultam, prepared according to the method of Vallgårda, J.; Appelberg, U.; Csöregh, I.; and Hacksell, U. (*J. Chem. Soc. Perkin Trans. I,* pages 461-470, **1994**): ¹H NMR (300 MHz, CDCl₃) δ 0.86-0.95 (m, 5H), 1.22-1.31 (m, 1H), 1.54-1.83, (m, 3H), 2.08 (q, *J* = 7.7 Hz, 2H), 3.16-3.36 (m, 2H), 3.76 (s, 3H), 5.59 (br s, 1H), 6.55-6.76 (m, 3H), 7.17 (t, *J* = 7.8 Hz, 1H); IR (NaCl Film) 3294, 2962, 1644, 1604, 1550, 1494 cm⁻¹; MS (isobutane - DCI) m/e 247.

### Example 4 (Process 4)

### 4a. Preparation of (trans)-N-[[2-(2-Iodo-5-methoxyphenyl)cyclopropyl]methyl] butanamide

To a stirred solution of (*trans*)-N-[[2-(3-methoxyphenyl) cyclopropyl]methyl] butanamide (120 mg, 0.49 mmol) and Tl(OAc)₃ (522 mg, 1.5 mmol) in CCl₄ (10 mL) was added a solution of I₂ (139 mg, 0.55 mmol) in CCl₄ (20 mL). The resulting suspension was heated at reflux for 18 h. The suspension was cooled to ambient temperature, filtered through a medium pore glass frit, and the filtrate washed with saturated sodium thiosulfate, brine, and dried (K₂CO₃). Concentration by rotary evaporation gave 150 mg of an orange oil which was purified by chromatography (silica gel, 40% EtOAc/hexane) to afford 108 mg (60%) of the title compound as a white solid: mp 84-86 °C; ¹H NMR (300 MHz, CDCl₃) δ 0.87-1.01 (m, 5H), 1.07-1.23 (m, 1H), 1.59-1.71 (m, 2H), 1.84-1.90 (m, 1H), 2.16 (t, *J* = 7.8 Hz, 2H), 3.16-3.25 (m, 1H), 3.46-3.53 (m, 1H), 3.73 (s, 3H), 5.83 (br s, 1H), 6.45-6.50 (m, 2H), 7.66 (d, *J* = 8.5 Hz, 1H); IR (NaCl Film) 3304, 2962, 1636, 1556, 1444, 1416 cm⁻¹; MS (isobutane - DCI) *m/e* 373; Analysis calc'd for C₁₅H₂₀NO₂I: C, 48.27; H, 5.40; N, 3.75; found: C, 48.24; H, 5.41; N, 3.53.

### 4b. Preparation of (trans)-N-[[2-(2-Iodo-5-fluorophenyl)cyclopropyl]methyl] butanamide

To a stirred solution of (*trans*)-N-[[2-(3-fluorophenyl) cyclopropyl]methyl] butanamide (1.0 g, 4.3 mmol), Tl(OAc)₃ (2.5 g, 6.5 mmol), and TFA (15 mL) in acetonitrile (15 mL) was added a solution of NaI (705 mg, 4.7 mmol) in H₂O (2 mL). The resulting suspension was heated at 55 °C for 12 h. The suspension was cooled to ambient temperature, filtered through a medium pore glass frit, and the filtrate washed with saturated sodium thiosulfate, brine, and dried (K₂CO₃). Concentration by rotary evaporation gave 1.26 g of an orange oil which was purified by chromatography (silica gel, 35% EtOAc/hexane) to afford 371 mg (25%) of the title compound as a white solid: mp 66-68 °C; ¹H NMR (300 MHz, CDCl₃) δ 0.91-0.99 (m, 5H), 1.00-1.15 (m, 1H), 1.70 (q, *J* = 7.5 Hz, 2H), 1.89-1.92 (m, 1H), 2.17 (t, *J* = 7.2 Hz, 2H), 3.28-3.32 (m, 1H), 3.43-3.50 (m, 1H), 5.67 (br s, 1H), 6.59-6.67 (m, 2H), 7.71-7.76 (m, 1H); IR (NaCl Film) 3417, 3294, 2962, 1638, 1554, 1448, 1412 cm⁻¹; MS (ESI) *m/e* 361; Analysis calc'd for C₁₄H₁₇NOFI: C, 46.55; H, 4.74, N, 3.88; found: C, 46.57; H, 4.68; N, 3.85.

### Example 5 (Process 5)

### Preparation of (trans)-N-[[2-(2,4-Dibromo-5-methoxyphenyl) cyclopropyl]methyl] butanamide

To a stirred solution of N-[[2-(3-methoxyphenyl)cyclopropyl]methyl] butanamide (190 mg, 0.77 mmol), and Tl(OAc)₃ (881 mg, 2.3 mmol) in CCl₄ (20 mL) at 0 °C was added a solution of Br₂ (238 mg, 1.5 mmol) in CCl₄ (10 mL) dropwise. After addition was complete the suspension was filtered through a medium pore glass frit and the filtrate washed with saturated sodium thiosulfate, brine, dried (K₂CO₃) and concentrated to give a clear oil. Purification by chromatography (silica gel, 35% EtOAc/hexane) afforded 185 mg (62%) of the title compound as a white solid: m.p. 114-115 °C; ¹H NMR (300 MHz, CDCl₃) δ 0.90-1.02 (m, 5H), 1.04-1.16 (m, 1H), 1.60-1.69 (m, 2H), 1.89-1.93 (m, 1H), 2.16 (t, *J* = 7.8 Hz, 2H), 3.04-3.16 (m, 1H), 3.49-3.54 (m, 1H), 3.82 (s, 3H), 5.77 (br s, 1H), 6.44 (s, 1H), 7.66 (s, 1H); IR (NaCl Film) 3290, 2960, 1632, 1550, 1474, 1442 cm⁻¹; MS *m/e* 405; Analysis calc'd for C₁₅H₁₉NO₂Br₂: C, 44.47; H, 4.73; N, 3.46; found: C, 44.59; H, 4.65; N, 3.16.

### Example 6 (Process 6)

### Preparation of (trans)-N-[[2-(3-Methoxyphenyl)cyclopropyl]methyl]-N-(phenylmethyl)butanamide

A magnetically stirred solution of (*trans*)-N-[2-[(3-methoxyphenyl)cyclopropyl]methyl] butanamide (1.0 g, 4.0 mmol) and NaH (105 mg, 4.4 mmol) in DMF (10 mL) was treated with benzyl bromide (750 mg, 4.4 mmol). The suspension was stirred for 18 h at room temperature, diluted with Et₂O (100 mL), and quenched with H₂O (100 mL). The layers were separated and the organic phase was washed with H₂O, brine, dried (K₂CO₃) and concentrated to give a crude oil which was purified by flash chromatography (silica gel, 45% EtOAc/Hexane) to afford 580 mg (43%) of the title compound as a clear oil: ¹H NMR (300 MHz, CDCl₃) δ 0.78-0.98 (m, 4H), 1.17-1.33 (m, 1H), 1.60-1.79 (m, 4H), 2.27-2.44 (m, 2H), 3.17-3.33 (m, 1H), 3.51-3.61 (m, 1H), 3.77 (br s, 3H), 4.46-4.82 (m, 2H), 6.50-6.72 (m, 3H), 7.10-7.34 (m, 6H); IR (NaCl Film) 2962, 1644, 1604, 1454, 1210 cm⁻¹; MS (isobutane - DCI) *m/e* 337; Analysis calc'd for C₂₂H₂₇NO₂·0.1 H₂O: C, 77.88; H, 8.08; N, 4.13; found: C, 77.87; H, 8.12; N, 3.89.

### Example 7 (Process 7)

### Preparation of (trans)-N-[[2-[3-(2-Propenyloxy)phenyl]cyclopropyl]methyl] butanamide

### Step i

(a). **(*trans*)-N-[[2-(3-Hydroxyphenyl)cyclopropyl]methyl] butanamide:** To a stirred solution of (*trans*)-N-[[2-(3-methoxyphenyl)cyclopropyl]methyl] butanamide (3.50 g, 14.2 mmol) in dichloromethane (50 mL) at -78 °C was added BBr₃ (28.4 mL, 1 N in CH₂Cl₂, 28.4 mmol) dropwise. After addition was complete, the cooling bath was removed and stirring was continued for 18 h. The solution was poured over ice/H₂O (100 mL) and extracted with 2.5 N NaOH. The basic extracts were combined, washed with dichloromethane, and acidified (conc. HCl). The acidic solution was extracted with dichloromethane, the organics were combined, washed with brine, dried (MgSO₄), and concentrated to afford 2.49 g (75%) of the title compound: ¹H NMR (300 MHz, DMSO-d₆) δ 0.72-0.84 (m, 5H), 1.09-1.15 (m, 1H), 1.42-1.54 (m, 2H), 1.63-1.69 (m, 1H), 2.02 (t, *J* = 5.0 Hz, 2H), 2.99-3.11 (m, 2H), 6.39-6.50 (m, 3H), 6.98 (t, *J* = 7.8 Hz, 1H), 7.91 (s, 1H), 9.19 (br s, 1H); IR (NaCl Film) 3300, 2964, 1642, 1585, 1542, 1466 cm⁻¹; MS (isobutane - DCI) *m/e* 233; Analysis calc'd for C₁₄H₁₉NO₂·0.1 H₂O: C, 71.52; H, 8.23; N, 5.96; found: C, 71.53; H, 8.32; N, 5.92.

### Step ii

(b). **(*trans*)-N-[[(2-Propen-1-yloxyphenyl)cyclopropyl]methyl] butanamide:**
To a rapidly stirred solution of (*trans*)-N-[[2-(3-hydroxyphenyl)cyclopropyl]methyl] butanamide (0.8 g, 3.4 mmol) and KOH (210 mg, 3.74 mmol) in ethanol (15 mL) was added allyl iodide (622 mg, 3.7 mmol). After stirring for 18 h, the suspension was diluted with Et₂O (100 mL), and washed with H₂O, 2N NaOH, brine, dried (K₂CO₃), and concentrated to a give a crude residue. The resulting material was purified by chromatography (silica gel, 40% EtOAc/hexane) to afford 320 mg (34%) of the title compound: ¹H NMR (300 MHz, CDCl₃) δ 0.70-0.94 (m, 5H), 1.12-1.32 (m, 1H), 1.58-1.84 (m, 3H), 2.20 (t, *J* = 6.0 Hz, 2H), 3.15-3.36 (m, 2H), 4.48 (d,*J*=5.6 Hz, 2H), 5.24 (d,*J*=10.0 Hz, 1H), 5.40 (d,*J* = 17.2 Hz, 1H), 5.56 (br s, 1H), 5.96-6.07 (m, 1H), 6.57-6.70 (m, 3H), 7.13 (t, *J* = 7.2 Hz, 1H); IR (NaCl Film) 3296, 2962, 1644, 1607, 1548, 1494 cm⁻¹; MS *m/e* 273; Analysis calc'd for C₁₇H₂₃NO₂ 0.20(H₂O): C, 73.72; H, 8.52; N, 5.06; found: C, 73.77; H, 8.63; N, 5.00.

### Example 8 (Process 8)

### Preparation of (trans)-N-[[2-[3-(Methylethoxy)phenyl]cyclopropyl]methyl] butanamide

To a rapidly stirred solution of (*trans*)-N-[[2-(3-hydroxyphenyl)cyclopropyl]methyl] butanamide (390 mg, 1.7 mmol), triphenylphosphine (498 mg, 1.9 mmol) and isopropanol (150 mg, 2.5 mmol) in THF (10 mL) at 0 °C was added diethyl azodicarboxylate ( 331 mg, 1.9 mmol) in one portion. After stirring for 2 h, the suspension was diluted with Et₂O (100 mL), washed with H₂O, 2 N NaOH, and brine, dried (K₂CO₃) and concentrated to a give a crude wax. The resulting material was purified by chromatography (silica gel, 35% EtOAc/hexane) to afford 144 mg (31%) of the title compound: ¹H NMR (300 MHz, CDCl₃) δ 0.85-0.94 (m, 5H), 1.22-1.24 (m, 1H), 1.29 (d, *J*=6.7 Hz, 6H), 1.58-1.68 (m, 2H), 1.70-1.77 (m, 1H), 2.14 (t, *J*=6.0 Hz, 2H), 3.14-3.36 (m, 2H), 4.44-4.54 (m, 1H), 5.81 (br s, 1H), 6.53-6.67 (m, 3H), 7.13 (t, *J*=7.2 Hz, 1H); IR (NaCl Film) 3294, 2974, 1644, 1610, 1550, 1492 cm⁻¹; MS *m/e* 275; Analysis calc'd for C₁₇H₂₅NO₂: C, 74.14; H, 9.15; N, 5.09; found: C, 73.77; H, 8.99; N, 4.82.

### Example 9 (Process 9)

### 9a. Preparation of (-)-(trans)-N-[[2-[5-Methoxy-2-phenylethynyl)phenyl] cyclopropyllmethyl] butanamide

(a) **(-)-(*trans*)-N-[[2-(5-Methoxy-2-phenylethynyl)phenyl] cyclopropyl]methyl] butanamide:** A stirred suspension of (*trans*)-N-[[2-(2-iodo-5-methoxyphenyl) cyclopropyl] methyl] butanamide (400 mg, 1.1 mmol), phenyl acetylene (133 mg, 1.3 mmol), and Pd (Ph₃P)₄ (63 mg, 0.05 mmol) in triethylamine (10 mL) was heated at reflux for 18 h, cooled to ambient temperature, and diluted with Et₂O (100 mL), washed with H₂O, brine, dried (K₂CO₃), and concentrated in vacuo to give 400 mg of a red oil. Purification by chromatography (silica gel, 30% EtOAc/Hexane) afforded 220 mg of a white solid (58%): mp 107-108 °C; ¹H NMR (300 MHz, CDCl₃) δ 0.78 (t, *J* = 7.5 Hz, 3H), 0.94-0.98 (m, 1H), 1.14-1.16 (m, 2H), 1.40-1.56 (m, 2H), 1.78-1.93 (m, 2H), 2.25-2.31 (m, 1H), 2.80-2.99 (m, 1H), 3.74-3.83 (m, 1H), 3.79 (s, 3H), 5.84 (br s, 1H), 6.41 (s, 1H), 6.71 (d, *J* = 4 Hz, 1H), 7.16-7.61 (m, 6H); IR (NaCl Film) 3310, 2960, 2214, 1638, 1544, 1502, 1428 cm⁻¹; MS (ESI) *m/e* 347; Analysis calc'd for C₂₃H₂₅NO₂: C, 79.51; H, 7.25, N, 4.03; found: C, 79.28; H, 7.21; N, 3.78.

### 9b. Preparation of (-)-(trans)-N-[[2-(5-Methoxy-2-phenylethyl)phenyl] cyclopropyl]methyl] butanamide

(b) **(-)(*trans*)-N-[[2-(5-methoxy-2-phenylethyl)phenyl] cyclopropyl]methyl] butanamide:** A suspension of (-)-(*trans*)-N-[[2-(5-methoxy-2-phenylethynyl)phenyl] cyclopropyl]methyl] butanamide (100 mg, 0.28 mmol), 10% Pd/C (50 mg) in ethanol (25 mL) was shaken under hydrogen (Parr apparatus, 50 psi) for 18 h. The suspension was filtered through celite and concentrated in vacuo to afford a clear oil (100 mg, 100%); ¹H NMR (300 MHz, CDCl₃) δ 0.90-0.94 (m, 5H), 1.25-1.30 (m, 1H), 1.60-1.66 (m, 1H), 1.71-1.79 (m, 2H), 2.05-2.15 (m, 2H), 2.88-2.98 (m, 4H), 3.20-3.35 (m, 2H), 3.77 (s, 3H), 5.57 (br s, 1H), 6.47 (s, 1H), 6.66-6.68 (m, 1H), 7.05 (d, *J* = 4 Hz, 1H), 7.17-7.32 (m, 5H); IR (NaCl Film) 3294, 2960, 1644, 1548, 1498, 1454 cm⁻¹; MS (ESI) *m/e* 351; Analysis calc'd for C₂₃H₂₉NO₂ 0.25(H₂O): C, 77.60; H, 8.41, N, 3.58; found: C, 77.48; H, 8.41; N, 3.58.

### Example 10 (Process 10)

### Preparation of (-)-(trans)-N-[[2-(2-Cyano-5-methoxyphenyl] cyclopropyl]methyl] butanamide

To a 100 mL round bottom flask fitted with a reflux condenser equipped with a calcium chloride drying tube was added (*trans*)-N-[[2-(2-iodo-5-methoxyphenyl)cyclopropyl]methyl] butanamide (220 mg, 0.6 mmol), powdered copper(I) cyanide (106 mg, 1.2 mmol), and anhydrous pyridine (2 mL). The mixture was then heated at 185 °C for 18 h. The reaction was quenched by the addition of 50% aqueous ammonia solution (30 mL), followed by extraction with toluene (3 x 50 mL). The organics were combined, washed with H₂O, 1N HCl, brine, and dried over MgSO₄. Filtration and concentration in vacuo gave 310 mg of a crude oil which was further purified by chromatography (silica gel, 45% EtOAc/ hexane) to afford a clear oil (120 mg, 74%): ¹H NMR (300 MHz, CDCl₃) δ 0.93 (t, *J* = 6.0 Hz, 3H), 0.96-0.99 (m, 1H), 1.10-1.16 (m, 1H), 1.20-1.28 (m, 1H), 1.64-1.71 (m, 2H), 2.03-2.10 (m, 1H), 2.20 (t, *J* = 7.8 Hz, 2H), 2.72-2.79 (m, 1H), 3.82 (s, 3H), 3.85-3.92 (m, 1H), 6.27 (br s, 1H), 6.56 (s, 1H), 6.73 (d, *J* = 8.4 Hz, 1H), 7.53 (d, *J* = 8.7 Hz, 1H); IR (NaCl Film) 3300, 2964, 2218, 1646, 1606, 1564, cm⁻¹; MS (ESI) *m/e* 272; Analysis calc'd for C₁₆H₂₀N₂O₂ 0.25(H₂O): C, 69.41; H, 7.46, N, 10.12; found: C, 69.41; H, 7.46; N, 10.00.

### Example 11 (Process 11)

### Preparation of (-)-(trans)-N-[[2-(2-Phenyl-5-methoxyphenyl] cyclopropyl]methyl] butanamide:

To a stirred suspension of (*trans*)-N-[[2-(2-iodo-5-methoxyphenyl) cyclopropyl] methyl] butanamide (210 mg, 0.56 mmol), barium hydroxide (265 mg, 0.84 mmol), and H₂O (1 mL) in DME (6 mL) was added an admix of Pd(Ph₃P)₄ (12 mg, 0.01 mmol) and phenyl boronic acid (75 mg, 0.62 mmol) in one portion. The resulting mixture was then heated at reflux for 18 h, diluted with toluene, washed with H₂O, brine, dried over K₂CO₃ and concentrated in vacuo to give a red wax. Purification by chromatography (silica gel, 35% EtOAc/ hexane) afforded 145 mg (85%) of clear oil: ¹H NMR (300 MHz, CDCl₃) δ 0.69-0.76 (m, 1H), 0.89 (t, *J* = 7.2 Hz, 3H), 0.93-0.98 (m, 1H), 1.01-1.08 (m, 1H), 1.51-1.64 (m, 2H), 1.79-1.86 (m, 1H), 2.01 (t, *J* = 7.2 Hz, 2H), 2.78-2.86 (m, 1H), 3.21-3.30 (m, 1H), 3.79 (s, 3H), 5.04 (br s, 1H), 6.50 (s, 1H), 6.74 (d, *J* = 8.4 Hz, 1H), 7.12 (d, *J* = 8.5 Hz, 1H), 7.30-7.43 (m, 5H); IR (NaCl Film) 3296, 2962, 1644, 1610, 1550, 1482, cm⁻¹; MS (ESI) *m/e* 323; Analysis calc'd for C₂₁H₂₅NO₂ 0.5(H₂O): C, 75.87; H, 7.88, N, 4.21; found: C, 75.95; H, 7.86; N, 4.15.

### Example 12 (Process 12)

### Preparation of (±)-(trans)-[2,2-Difluoro-3-(3-methoxyphenyl)-cyclopropylmethyl] butanamide

### Step i

(a) **(±)-(*trans*)-3-(3-Methoxyphenyl)-prop-2-en-1-ol:** A solution of 3-methoxycinnamic acid (50.24 g, 281 mmol) in 400 mL of anhydrous tetrahydrofuran was added to a magnetically stirred suspension of sodium borohydride (12.80 g, 338 mmol) in 500 mL of anhydrous tetrahydrofuran at room temperature. The suspension was stirred until gas evolution ceased, cooled to 0 °C, and a solution of iodine (35.77 g, 141 mmol) in anhydrous tetrahydrofuran (500 mL) was added over 1 h. The suspension was stirred for 3 h and treated dropwise with a 4N solution of hydrochloric acid (100 mL). The resultant suspension was extracted with diethyl ether (3 x 500 mL), and the combined organic portions washed with 3 N NaOH (3 x 500 mL), brine (500 mL), dried (MgSO₄), filtered, and concentrated in vacuo. Kügelrohr distillation (0.5 mm) gave the product as a clear liquid (23.12 g, 50%): ¹H NMR (300 MHz, CDCl₃) δ 3.82 (s, 3H), 4.34 (d, *J* = 6 Hz, 2H), 6.37 (dt,*J* = 15,6 Hz, 1H), 6.60 (d,*J* = 15 Hz, 1H), 6.80 (dd,*J* = 8,*J* = 1 Hz, 1H), 6.93 (t,*J* = 1 Hz, 1H), 7.00 (d, *J* = 8 Hz, 1H), 7.24 (t, *J* = 8 Hz, 1H).

### Step ii

(b) **(±)-(*trans*)-Acetic acid (3-(3-methoxyphenyl) allyl ester:** A magnetically stirred solution of (±)-(*trans*)-3-(3-Methoxyphenyl)-prop-2-en-1-ol (22.35 g, 136 mmol) in anhydrous pyridine (115 mL) at 0 °C was treated dropwise with acetic anhydride (16.0 mL, 170 mmol). After addition was complete the ice bath was removed, the solution was allowed to warm to room temperature, and stirred for 24 h. The solution was concentrated in vacuo and the residue digested with diethyl ether (300 mL), washed with 1 N HCl (2 x 300 mL), 5% NaHCO₃ (200 mL), water (200 mL), brine (200 mL), dried over MgSO₄, filtered, and concentrated in vacuo to a clear liquid. Flash chromatography (5% diethyl ether / hexane elution) and Kügelrohr distillation (0.5 mm) of the resultant liquid gave the product as a clear liquid (16.95 g, 60%): ¹H NMR (300 MHz, CDCl₃) δ 2.11 (s, 3H), 3.81 (s, 3H), 4.73 (d, *J* = 6 Hz, 2H), 6.28 (dt, *J* = 15, Hz, 1H), 6.63 (d, *J* = 15 Hz, 1H), 6.82 (dd, *J* = 8,1 Hz, 1H), 6.92 (t, *J* = 1 Hz, 1H), 6.99 (d, *J* = 8 Hz, 1H), 7.24 (t, *J* = 8 Hz, 1H).

### Step iii

(c) **(±)-(*trans*)-Acetic acid-2,2-difluoro-3-(3-methoxyphenyl)cyclopropyl methyl ester.** A solution of (±)-(*trans*)-Acetic acid (3-(3-methoxyphenyl) allyl ester (1.63 g, 7.90 mmol) in anhydrous diglyme was heated at reflux and subsequently treated dropwise over 1 h with a solution of sodium chlorodifluoroacetate (9.02 g, 59.2 mmol) in anhydrous diglyme (25 mL). The suspension was cooled to room temperature and poured over 200 mL of ice. The resultant suspension was extracted with diethyl ether (4 x 200 mL) and the combined organic extracts were washed with water (3 x 400 mL), brine (400 mL), dried (MgSO₄), filtered, and concentrated in vacuo. Kügelrohr distillation (0.5 mm) gave 1.59 g (79%) of the product as a clear oil: ¹H NMR (300 MHz, CDCl₃) δ 2.10 (s, 3H), 2.24 (ddt, *J* = 15,7.5,7 Hz, 1 H), 2.63 (dd, *J* = 15,7.5 Hz, 1 H), 1H), 3.80 (s, 3H), 4.19-4.25 (m, 4.31-4.37 (m, 1H), 6.74-6.83 (m, 3H), 7.25 (t, *J* = 8 Hz, 1H).

### Step iv

(d) **(±)-(*trans*)-[2,2-Difluoro-3-(3-methoxyphenyl)cyclopropyl] methanol:** A magnetically stirred solution of potassium hydroxide (2.39 g, 42.6 mmol) in 3 :1 methanol / tetrahydrofuran (88 mL) was treated with (±)-(*trans*)-acetic acid-2,2-difluoro-3-(3-methoxyphenyl)cyclopropyl methyl ester (4.16 g, 16.2 mmol). The solution was stirred for 2 h and concentrated in vacuo. The residue was digested with diethyl ether (200 mL) and water (200 mL), the layers separated, the aqueous layer further extracted with diethyl ether (2 x 200 mL), the organic extracts washed with saturated NaHCO₃ solution (300 mL), water (300 mL), brine (300 mL), dried (MgSO₄), filtered, and concentrated in vacuo to give 3.42 g (99%) of a clear oil that was used without further purification: ¹H NMR (300 MHz, CDCl₃) δ 1.60 (br s, 1H), 2.15-2.36 (m, 1 H), 1H), 2.60 (ddd, *J* = 15, 7.5, 1.5 Hz, 3.81 (s, 3H), 3.83-3.98 (m, 2H), 6.77 (t, *J* = 1 Hz, 1H), 6.81 (d, *J* = 8 Hz, 1H), 6.82 (dd, *J* = 8, 1 Hz, 1H), 7.26 (t, *J* = 8 Hz, 1H).

### Step v

(e)(f)(g) **(±)-(*trans*)-[2,2-Difluoro-3-(3-methoxyphenyl)cyclopropyl] methanamine:** A magnetically stirred solution of the alcohol (3.27 g, 15.3 mmol), triethylamine (3.14 g, 31.0 mmol), and CH₂Cl₂ (50 mL) at 0 °C under nitrogen was treated dropwise over 15 min with methanesulfonyl chloride (2.8 g, 24.4 mmol). The solution was stirred 30 min, diluted with CH₂Cl₂ (200 mL), and washed sequentially with water (200 mL) and saturated NaHCO₃ (200 mL), dried (K₂CO₃), filtered, and concentrated in vacuo at 5 °C. The residue was digested with anhydrous dimethylformamide (50 mL), treated with sodium azide (1.96 g, 30.1 mmol), and stirred 14 h. The resultant solution was poured into water (500 mL), extracted with diethyl ether (4 x 250 mL), the combined extracts washed with water (4 x 500 mL), brine (2 x 300 mL), dried (MgSO₄), filtered, and concentrated in vacuo (caution!) to a clear oil. The oil was digested with anhydrous diethyl ether (40 mL) and added dropwise to a magnetically stirred suspension of lithium aluminum hydride (1.16 g, 30.6 mmol) in anhydrous diethyl ether (60 mL) at -30 °C. The suspension was allowed to warm to room temperature, stirred for 3 h, recooled to -30 °C, and treated dropwise with a solution of KHSO₄ (2.6 g, 19 mmol) in water (20 mL). The resultant suspension was allowed to warm to room temperature, stirred 1 h, and filtered through celite with diethyl ether elution (400 mL). The layers were separated and the organic layer extracted with 1N HCl (3 x 100 mL), the combined aqueous extracts made basic with 50% NaOH, and extracted with CH₂Cl₂ (3 x 150 mL). The organic portion was washed with brine (200 mL), dried (K₂CO₃), filtered, and concentrated in vacuo to give 1.23 g (38%) of a clear oil: ¹H NMR (300 MHz, CDCl₃) δ 1.42 (br s, 2H), 1.97-2.08 (m, 1 H), 2.44-2.52 (m, 1H), 2.91-3.10 (m, 2 H), 3.80 (s, 3H), 6.76 (t, *J* = 1 Hz, 1H), 6.81 (d, *J* = 8 Hz, 1H), 6.81 (dd, *J* = 8,1 Hz, 1H), 7.23 (t, *J* = 8 Hz, 1H).

### Step vi

(h) **(±)-(*trans*)-[2,2-Difluoro-3-(3-methoxyphenyl)cyclopropylmethyl] butanamide:** A magnetically stirred solution of (±)-(*trans*)-[2,2-Difluoro-3-(3-methoxyphenyl)-cyclopropyl] methanamine (672 mg, 3.16 mmol) and triethylamine (1.5 mL) in anhydrous CH₂Cl₂ (20 mL) was treated dropwise with a solution of butyryl chloride (370 mg, 3.47 mmol) in anhydrous CH₂Cl₂ (3 mL). The solution was stirred for 48 h and concentrated in vacuo. The residue was digested with CH₂Cl₂ (100 mL) and washed with 1 N HCl (100 mL), 1 N NaOH (100 mL), brine (100 mL), dried (K₂CO₃), filtered, and concentrated in vacuo. Flash chromatography (2:1 hexanes/ethyl acetate elution) gave the product as a clear oil that solidified to a waxy solid on standing: ¹H NMR (300 MHz, CDCl₃) δ 0.92 (t, *J* = 7.4 Hz, 3H), 1.64 (sextet, J=7.4 Hz, 2H), 2.09-2.21 (m, 1H), 2.16 (t, *J* = 7.4 Hz, 2H), 2.51 (dd, *J* = 13.8, 7.4 Hz, 1H), 3.19-3.22 (m, 1 H), 1H), 3.76 (s, 3H), 3.77-3.85 (m, 5.90 (br s, 1H), 6.70 (s, 1H), 6.75 (d, *J* = 8 Hz, 1H), 6.77 (dd, *J* = 8, 1 Hz, 1H), 7.20 (t, *J* = 8 Hz, 1H); IR (NaCl Film) 3302, 2960, 1644, 1552, 1288, 1266, 1250, 1160 cm⁻¹; MS (isobutane - DCI) *m/e* 282 (M-H⁻); Analysis calc'd for C₁₅H₁₉NO₂F₂: C, 63.59; H, 6.76; N, 4.94; found: C, 63.59; H, 6.83; N, 4.85.

### Example 13 (Process 13)

### Preparation of (trans)-N-[[2-(2-Butyrylamino-5-methoxyphenyl)cyclopropyl]methyl] butanamide

### Step i

**(a) (*trans*)-N-[[2-(2-Amino-5-methoxyphenyl)cyclopropane] methyl] amine:** A Parr bottle was charged with a suspension of (*trans*)-2-(2-nitro-5-methoxyphenyl)cyclopropane carboxaldehyde oxime (1.0 g, 4.2 mmol), 10% Pd/C (100 mg) and HOAc (25 mL) was shaken under hydrogen (50 psi) for 18 h. The material was then filtered through celite, digested with H₂O (300 mL) and made basic with 10N NaOH. The product was then extracted with CH₂Cl₂, the extracts were combined, dried over K₂CO₃, and concentrated in vacuo to afford 800 mg (100%) of a red oil: ¹H NMR (300 MHz, CDCl₃) 8 0.78-0.85 (m, 1H), 1.34-1.39 (m, 1H), 1.59-1.69 (m, 1H), 1.77-1.83 (m, 1H), 1.88 (br s, 2H), 3.10-3.38 (m, 2H), 3.66 (s, 3H), 6.39 (d, *J* = 6.0 Hz, 1H), 6.50-6.59 (m, 1H), 6.61 (br s, 2H), 6.78 -6.97 (m, 1H).

### Step ii

**(b) (*trans*)-N-[[2-(2-Butyryl amino-5-methoxyphenyl)cyclopropyl]methyl]-butanamide:** To a magnetically stirred solution of (*trans*)-N[[2-(2-amino-5-methoxyphenyl)cyclopropane]methyl]amine (400 mg, 2.2 mmol) and Et₃N (0.86 mL, 6.6 mmol) in dry CH₂Cl₂ (15 mL) at 0 °C was added butyryl chloride (256 mg, 2.4 mmol) dropwise. The resulting suspension was allowed to come to room temperature and stirred for 18 h. Concentration by rotary evaporation yielded a crude product which was then purified by flash chromatography (silica gel, 30% EtOAc/hexane) to afford 250 mg (44% ) of a clear oil: ¹H NMR (300 MHz, CDCl₃) δ 0.75-1.04 (m, 8H), 1.50-1.76 (m, 5H), 2.10 (t, *J* = 8.1 Hz, 2H), 2.45 (t, *J* = 8.0 Hz, 2H), 2.68-2.78 (m, 1H), 3.69 (s, 3H), 3.72-3.78 (m, 2H), 6.27 (d, *J* = 2.0 Hz, 1H), 6.36 (br s, 1H), 6.63 (dd, *J* = 7.8, 2.7 Hz, 1H), 7.50 (d, *J* = 8.7 Hz, 1H), 7.89 (br s, 1H); IR (NaCl Film) 3290, 2961, 1647, 1534, 1425 cm^{-1;} MS (ESI) *m/e* 332; Analysis calc'd for C₁₉H₂₈N₂O₃·0.4 H₂O: C, 67.19; H, 8.54; N, 8.24; found: C, 67.58; H, 8.19; N, 7.77.

### Example 14 (Process 14)

### Preparation of (-)-(trans)-N-[1-[2-(3-Methoxyphenyl)cyclopropyl]ethyl] butanamide (Isomer A)

### Step i

(a) **(-)-(*trans*)-1-[2-(3-Methoxyphenyl)cyclopropyl] ethanol (Isomer A) and (-)(*trans*)-1-[2-(3-Methoxyphenyl)cyclopropyl] ethanol (Isomer B):**
Dimethyl sulfoxide (3.98 mL, 56 mmol) was added dropwise to a solution of oxalyl chloride (2M in CH₂Cl₂, 19.5 mL, 39 mmol) in methylene chloride (20 mL) at -78 °C. A solution of (-)-(*trans*)[2-(3-Methoxyphenyl)cyclopropyl] methanol (5.0 g, 28 mmol) in dichloromethane (10 mL) was added dropwise. The solution was stirred for 30 min, triethylamine (16.2 mL, 112 mmol) was slowly added, and the resulting suspension allowed to warm to room temperature. The suspension was stirred for 30 min, diluted with CH₂Cl₂ (100 mL), washed with water, dried over K₂CO₃, and concentrated in vacuo to afford 5.8 g of the aldehyde, which was used without purification The aldehyde was digested with THF (10 mL) and added dropwise to a solution of methyl magnesium bromide (3M in Et₂O, 23.3 mL, 70 mmol) in THF (25 mL) at 0 °C. The solution was stirred at 0 °C for 2 h after which 2N HCl (20 mL) in EtOAc (250 mL) was added. The resultant suspension was then washed with H₂O, brine, dried (K₂CO₃), and concentrated in vacuo to give 5.5 g of a crude oil. Purification by silica gel chromatography (25% EtOAc/hexane) afforded two diastereomers, (isomer A) 1.66 g, 1st band, and (isomer B) 1.64 g, 2nd band: (isomer A) R_{f} = 0.45(25% EtOAc/hexanes); ¹H NMR (300 MHz, CDCl₃) δ 0.84-0.94 (m, 2H), 1.19-1.27 (m, 1H), 1.30 (d, *J* = 6.3 Hz, 3H), 1.83-2.02 (m, 1H), 3.32-3.38 (m, 1H), 3.77 (s, 3H), 6.60-6.73 (m, 3H), 7.12 (t, *J* = 7.8 Hz, 1H); [a]_{D}²⁰ -49.0 ° (c=1, CH₂Cl₂). (isomer B) R_{f} = 0.40 (25% EtOAc/hexanes); ¹H NMR (300 MHz, CDCl₃) δ 0.93-1.00 (m, 2H), 1.21-1.29 (m, 1H), 1.32 (d, *J* = 6.3 Hz, 3H), 1.75-1.78 (m, 1H), 3.33-3.38 (m, 1H), 3.77 (s, 3H), 6.59-6.69 (m, 3H), 7.15 (t, *J* = 7.8 Hz, 1H); [a]_{D}²⁰ -55.3° (c=1, CH₂Cl₂).

### Step ii

**(b) (-)-(*trans*)-2-[1-[2-(3-methoxyphenyl)cyclopropyl]ethyl]isoindole-1,3-dione (isomerA):**
Diethyl azodicarboxylate (1.79 g, 10.3 mmol) was added dropwise to a stirred solution of (-)-(*trans*)-1-[2-(3-methoxyphenyl)cyclopropyl]ethanol (isomer A) (1.66 g, 8.6 mmol), triphenyl phosphine (2.69 g, 10.3 mmol), and phthalimide (1.52 g, 10.3 mmol) in THF (75 mL) at 0 °C. The reaction was allowed to warm to ambient temperature and stirred for 18 h. The solvent was removed in vacuo and the resulting residue was digested with EtOAc (150 mL) and washed sequentially with 1N HCl, 1N NaOH, and brine. Drying over K₂CO₃, filtration, and concentration in vacuo afforded 2.6 g (94%) of the product as a waxy white solid: ¹H NMR (300 MHz, CDCl₃) δ 0.80-0.92 (m, 2H), 1.48-1.60 (m, 4H), 1.81-1.88 (m, 1H), 1.91-2.00 (m, 1H); 3.76 (s, 3H), 6.58-6.70 (m, 3H), 7.15 (t, *J* = 7.8 Hz, 1H), 7.24-7.80 (m, 4H).

### Step iii

(c) **(-)-(*trans*)-1-[2-(3-Methoxyphenyl)cyclopropyl]ethylamine (isomer A):**
A solution of (-)-(*trans*)-2-[1-[2-(3-methoxyphenyl)cyclopropyl]ethyl] isoindole-1,3-dione (isomer A) (2.6 g, 8.1 mmol) and hydrazine (827 mg, 25.8 mmol) in ethanol (75 mL) was stirred at room temperature for 18 h. The resultant white paste was diluted with ethanol (100 mL), treated with 10 N HCl (10 mL), and stirred for 1 h. The solution was treated with EtOAc (200 mL), and extracted with 1 N HCl. The acidic extracts were combined, washed with Et₂O, basified (10 N NaOH), and extracted with CH₂Cl₂. The organics were dried over K₂CO₃ and concentrated in vacuo to give 1.1 g of a crude oil. Purification by silica gel chromatography (10% MeOH/CH₂Cl₂) afforded a clear oil (600 mg, 40%): ¹H NMR (300 MHz, CDCl₃) δ 0.85-0.98 (m, 2H), 1.10-1.20 (m, 1H), 1.25 (d, *J* = 6.0 Hz, 3H), 1.70-1.79 (m, 1H), 2.30 (br s, 2H), 2.48-2.52 (m, 1H), 3.75 (s, 3H), 6.58-6.75 (m, 3H), 7.15 (t, *J* = 7.0 Hz, 1H).

### Step iv

(d) **(-)-(*trans*)-N-[1-[2-(3-Methoxyphenyl)cyclopropyl]ethyl] butanamide (isomer A):** To a magnetically stirred solution of (-)-(*trans*)-1-[2-(3-methoxyphenyl)cyclopropyl]ethylamine(isomer A) (200 mg, 1.1 mmol) and Et₃N (0.46 mL, 3.3 mmol) in dry dichloromethane (15 mL) at 0 °C was added butyryl chloride (127 mg, 1.2 mmol) dropwise. The resulting suspension was allowed to warm to room temperature and stirred for 4 h. The solvent was removed in vacuo and the residue was digested with EtOAc (100 mL), washed sequentially with H₂O, 5% citric acid, 5% K₂CO₃, brine, and dried over K₂CO₃. Filtration and concentration in vacuo yielded a crude product which was then purified by flash chromatography (silica gel, 40% EtOAc/hexane) to afford 100 mg (39%) of the title compound: R_{f} = 0.75 (10% MeOH/CHCl₃); ¹H NMR (300 MHz, CDCl₃) δ 0.85-0.97 (m, 4H), 1.04-1.17 (m, 2H), 1.24 (d, *J* = 6.6 Hz, 3H), 1.61-1.73 (m, 2H), 1.77-1.83 (m, 1H), 2.19 (t, *J* = 7.8 Hz, 2H), 3.54-3.68 (m, 1H), 3.77 (s, 3H), 5.59 (s, 1H), 6.57-6.71 (m, 3H), 7.15 (t, *J* = 7.6 Hz, 1H); IR (NaCl Film) 3291, 2963, 1640, 1543, 1455 cm⁻¹; MS (ESI) *m/e* 261; Analysis calc'd for C₁₆H₂₃NO₂: C, 73.53; H, 8.87; N, 5.36; found: C, 73.48; H, 8.86; N, 5.25.

### Example 15 (Process 15)

### Preparation of (trans)-N-[[2-[3-(Methylethoxy)phenyl]cyclopropyl]methyl] butanamide

A solution of (*trans*)-N-[[2-(3-hydroxyphanyl)cyclopropyl]methyl] butanamide (342 mg, 1.47 mmol) and sodium hydroxide (58 mg, 1.45 mmol) in anhydrous methanol (10 mL) was concentrated in vacuo and the residue powdered under anhydrous toluene (3 mL). The suspension was treated with a solution of ethylene carbonate (259 mg, 2.94 mmol) in anhydrous toluene (5 mL). The resultant mixture was heated at reflux for 12 h, cooled to room temperature, and treated with 3 N NaOH (100 mL), ice (50 g), and diethyl ether (100 mL). The aqueous layer was separated and extracted with fresh diethyl ether (2 x 100 mL). The combined organic extracts were washed with water (200 mL), brine (200 mL), dried (K₂CO₃), filtered, and concentrated in vacuo. Flash chromatography of the resultant oil (1:2 hexane/ethyl acetate) gave 178 mg (44%) of the compound as a clear oil: ¹H NMR (300 MHz, CDCl₃) δ 0.84-0.92 (m, 2H), 0.91 (t, *J* = 7.4 Hz, 2H), 1.21-1.32 (m, 1H), 1.63 (sextet, *J* = 7.4 Hz, 2H), 1.72-1.78 (m, 1H), 2.13 (t, *J* = 7.4 Hz, 2H), 2.30 (br s, 1H), 3.16-3.34 (m, 2H), 3.91 (t, *J* = 4.5 Hz, 2H), 4.03 (t, *J* = 4.5 Hz, 2H), 5.75 (br s, 1H), 6.58 (t, *J* = 1.5 Hz, 1H), 6.63 (d, *J* = 8.0 Hz, 1H), 6.68 (ddd, *J*=8.0, 1.5, 1.5 Hz, 1 H), 1H), 7.13 (t, *J* = 8.0 Hz, IR (NaCl Film) 3300, 3076, 2962, 1644, 1610, 1582, 1552 cm⁻¹; MS *m/e* 276 (M-H⁻) ; Analysis calc'd for C₁₆H₂₃NO₃: C, 69.29; H, 8.36; N, 5.05; found: C, 68.99; H, 8.39; N, 4.93.

### Example 16 (Process 16)

### Preparation of (-)-(trans)-N-[[2-(3-Methoxyphenyl)cyclopropyl]ethyl]butanamide

### Step i

(**a) (-)-(*trans*)-1-(Cyanomethyl)-2-(3-methoxyphenyl)cyclopropane:** To a magnetically stirred solution of (-)-(*trans*)-2-(3-methoxyphenyl)cyclopropane methanol (1.60 g, 8.8 mmol) and triethylamine (1.9 mL, 13.2 mmol) in dichloromethane (150 mL) at 0 °C was added methanesulfonyl chloride (1.21 g, 10.6 mmol) dropwise. After addition was complete the ice bath was removed and stirring was continued for 1 h. The solution was treated with dichloromethane (150 mL), washed with saturated NaHCO₃, and dried over K₂CO₃. Concentration in vacuo gave 1.78 g of a crude oil which was utilized without further purification.
   The mesylate was taken into DMF (100 mL), treated with sodium cyanide (862 mg, 17.6 mmol), and stirred on a steam bath for 4 h. The solution was concentrated in vacuo, the residue was taken into Et₂O (100 mL), washed with H₂O, brine, and the solvent removed in vacuo to produce 1.41 g (85%,) of a clear oil: ¹H NMR (300 MHz, CDCl₃) δ 0.90-1.09 (m, 2H), 1.23-1.30 (m, 1H), 1.81-1.92 (m, 1H), 2.52-2.60 (m, 2H), 3.78 (s, 3H), 6.58-6.72 (m, 3H), 7.19 (t, *J* = 8.0 Hz, 1H).
**(b) (-)-(*trans*)-2-(3-Methoxyphenyl)cyclopropane ethylamine:** To a stirred suspension of LiAlH₄ (650 mg, 17 mmol) in THF (50 mL) at -45 °C was added a solution of (-)-(*trans*)-1-(cyanomethyl)-2-(3-methoxyphenyl)cyclopropane (1.41 g, 7.5 mmol) in THF (25 mL). The temperature was maintained below -30 °C, throughout. After addition was complete, the suspension was allowed to warm to room temperature and stirred for 4 h. The suspension was recooled to -45 °C and 1N HCl (50 mL) was cautiously added dropwise. The suspension was stirred at room temperature for 30 min, filtered through celite, and the filter cake was washed well with Et₂O. The filtrates were combined, extracted with 1N HCl, and the acidic layers were basified (30% NaOH). The basic solution was extracted with CH₂Cl₂, dried (K₂CO₃), and concentrated in vacuo to afford 500 mg (39%) of a clear oil: ¹H NMR (300 MHz, CDCl₃) δ 0.75-0.82 (m, 1H), 0.88-0.96 (m, 1H), 1.00-1.11 (m, 1H), 1.45-1.70 (m, 3H), 2.62 (br s, 2H), 2.88 (t, *J* = 7.5 Hz, 2H), 3.77 (s, 3H), 6.52-6.70 (m, 3H), 7.18 (t, *J* = 8.0 Hz, 1H).
**(c) (-)-(*trans*)-N-[[2-(3-Methoxyphenyl)cyclopropyl]ethyl] butanamide:** To a magnetically stirred solution of (-)-(*trans*)-2-(3-methoxyphenyl) cyclopropane ethylamine (400 mg, 2.2 mmol) and Et₃N (0.86 mL, 6.6 mmol) in dry dichloromethane (15 mL) at 0 °C was added butyryl chloride (256 mg, 2.4 mmol) dropwise. The resulting suspension was allowed to come to room temperature and stirred for 18 h. Concentration in vacuo yielded a crude product which was then purified by flash chromatography (silica gel, 30% EtOAc/hexane) to afford 250 mg (44% ) of a clear oil: ¹H NMR (300 MHz, CDCl₃) δ 0.70-0.81 (m, 1H), 0.88-1.01 (m, 5H), 1.54-1.68 (m, 5H), 2.12 (t, *J* = 7.2 Hz, 2H), 3.29-3.36 (m, 2H), 3.77 (s, 3H), 5.75 (br s, 1H), 6.54-6.71 (m, 3H), 7.14 (t, *J* = 7.8 Hz, 1H); IR (NaCl Film) 3295, 2962, 1644, 1604, 1551, 1494 cm⁻¹; MS (ESI) *m/e* 261; [a]_{D}²⁰ -66.3° (c=1, CH₂Cl₂); Analysis calc'd for C₁₆H₂₃NO₂·0.20 H₂O: C, 72.53; H, 8.90; N, 5.29; found: C, 72.67; H, 9.03; N, 5.29.

### Example 17 (Process 17)

### Preparation of (trans)-N-[[3-(3-methoxyphenyl)-2,2-dimethyl-1-cyclopropyl]methyl]butanamide.

### Step i

**(a) (*trans*)-3-(3-Methoxypheny)-2,2-dimethyl-cyclopropane carboxylic acid ethyl ester:** To a stirred solution of isopropyltriphenylphosphonium iodide (50 g, 116 mmol) in THF (250 mL) at 0 °C was added butyllithium (2.3 M in hexane, 42.2 mL, 115.6 mmol) dropwise, maintaining the temperature below 5 °C throughout. The solution was stirred for 1 h, treated dropwise with a solution of cinnamic acid ethyl ester (18.33 g, 89 mmol) in THF (50 mL), allowed to warm to room temperature, and stirred for 4 h. The resulting suspension was quenched with saturated NH₄Cl (200mL), and extracted with ethyl acetate. The organic extracts were combined, washed with brine, dried (K₂CO₃), and concentrated to give a light brown solid. This solid was then purified by Kügelrohr distillation (110 °C,0.5 mm) to give 13.0 g (59%) of a clear oil: ¹H NMR (300 MHz, CDCl₃) δ 0.95 (s, 3H), 1.12 (t, *J* = 8.5 Hz, 3H), 1.38 (s, 3H), 1.95 (d, *J* = 6.0 Hz, 1H), 2.62 (d, *J* = 6.0 Hz, 1H), 3.80 (s, 3H), 4.12 (q, *J* = 7.0 Hz, 2H), 6.65-6.68 (m, 2H), 7.18-7.26 (m, 2H).

### Step ii

**(b) (*trans*)-(3-(3-Methoxyphenyl)-2,2-dimethyl-cyclopropyl) methanol:** To a suspension of LiAlH₄ (4.21 g, 111 mmol) in THF (200 mL) at -45 °C was added a solution of (*trans*)-3-(3-methoxypheny)-2,2-dimethyl-cyclopropane carboxylic acid ethyl ester (12.5 g, 50.4 mmol) in THF (20 mL) dropwise. The cooling bath was then removed and the reaction was allowed to come to room temperature followed by immediate recooling to -45 °C. A solution of KHSO₄ (24 g, 177 mmol) in H₂O (50 mL) was cautiously added allowing the temperature to rise to -5 °C. The resulting paste was stirred at room temperature for 1 h, filtered through celite, and the filter cake was washed with Et₂O. The combined filtrates were washed with cold (0 °C) 1 N HCl (3x), 5% K₂CO₃ (1x), brine (1x), dried (K₂CO₃), and concentrated to give 10.1 g (97%) of a clear oil: ¹H NMR (300 MHz, CDCl₃) δ 0.85 (s, 3H), 1.25 (s, 3H), 1.78 (d, *J* = 6.0 Hz, 1H), 3.62-3.72 (m, 3H), 3.79 (s, 3H), 3.82 (dd, *J* = 7,10 Hz, 1H), 6.70-6.78 (m, 2H), 7.20 (t, *J*= 7.5 Hz, 1H), 7.35 (m, 1H).

### Step iii

**(c) (*trans*)-3-(3-Methoxyphenyl)-2,2-dimethyl-cyclopropyl)methyl) azide:**
To a solution of (*trans*)-(3-(3-methoxyphenyl) -2,2-dimethyl-cyclopropyl) methanol (10.0 g, 48.5 mmol) and triethylamine (10.1 mL,72.8 mmol), in dichloromethane (100 mL) at 0 °C was added methanesulfonyl chloride (6.11 g, 53.4 mmol) dropwise. After addition was complete the ice bath was removed and stirring was continued for 30 min. The reaction was then diluted with dichloromethane (100 mL), washed with H₂O, saturated NaHCO₃, and dried over K₂CO₃. Concentration in vacuo gave 13.09 g of a colored oil suitable for use in the next step.
The above mesylate was taken into DMF (100 mL), treated with sodium azide (3.78 g, 58.2 mmol), and stirred at room temperature for 18 h. The mixture was concentrated and the residue was taken into Et₂O, washed with H₂O (1x), brine(1x), and the solvents removed in vacuo to produce 10.1 g of a clear oil suitable for use without further purification: ¹H NMR (300 MHz, CDCl₃) δ 0.85 (s, 3H), 1.30 (s, 3H), 1.39-1.42 (m, 1H), 1.80 (d, *J* = 6.0 Hz, 1H), 3.30 (dd, *J* = 8,10 Hz, 1H), 3.51 (dd, *J* = 6.0, 8.3 Hz, 1H), 3.80 (s, 3H), 6.68-6.79 (m, 3H), 7.20 (t, *J* = 7.5 Hz, 1H).

### Step iv

**(d) (*trans*)-(3-(3-Methoxyphenyl)-2,2-dimethyl-cyclopropyl)methyl) amine:**
A stirred suspension of LiAlH₄ (4.80 g, 126.6 mmol) in THF (100 mL) at -30 °C was treated with a solution of (*trans*)-(3-(3-methoxyphenyl)-2,2-dimethyl-cyclopropyl)methyl) azide (6.48 g, 30.14 mmol) in THF (10 mL), maintaining the temperature below -30 °C. After addition was complete the suspension was allowed to come to room temperature and stirred for 4 h. The suspension was cooled to -45 °C and a solution of KHSO₄ (16 g) in H₂O (20 mL) was added dropwise (caution !). The resulting suspension was stirred at room temperature for 30 min, filtered through celite, and the filter cake was washed well with Et₂O. The filtrates were combined, extracted with 1 N HCl (2x), and the acidic layers were basified (30% NaOH). The basic solution was extracted with CH₂Cl₂ (3x), dried (K₂CO₃), and concentrated to afford 2.80 g (47%) of a clear oil: ¹H NMR (300 MHz, CDCl₃) δ 0.82 (s, 3H), 1.22 (s, 3H), 1.60-1.68 (m, 4H), 2.75-2.92 (m, 2H), 3.78 (s, 3H), 6.68-6.75 (m, 3H), 7.15 (t, *J* = 7.5 Hz, 1H).

### Step v

**(e) (*trans*)-N-[[3-(3-methoxyphenyl)-2,2-dimethyl-1-cyclopropyl]methyl] butanamide:** Acylation of (3-(3-methoxyphenyl)-2,2-dimethylcyclopropyl)methyl) amine (620 mg, 3.0 mmol) as in Example 1 was accomplished with butyryl chloride (0.34 mL, 3.3 mmol) to give 550 mg (67%) of a clear oil: ¹H NMR (300 MHz, CDCl₃) δ 0.80 (s, 3H), 0.90 (t, *J* = 7.4 Hz, 3H), 1.23 (s, 3H), 1.23-1.30 (m, 1H), 1.58-1.71 (m, 3H), 2.14 (t, *J* = 7.3 Hz, 2H), 3.39 (t, *J* = 5.4 Hz, 2H), 3.76 (s, 3H), 5.50 (br s , 1H), 6.65-6.71 (m, 3H), 7.15 (t, *J* = 7.8 Hz, 1H); IR (NaCl Film) 3294, 2964, 1642, 1550, 1490, 1456, 1274 cm⁻¹; MS *m/e* 275.39; Analysis calc'd for C₁₇H₂₅NO₂: C, 74.14; H, 9.15; N, 5.09; found: C, 73.95; H, 9.21; N, 5.11.

Table 1 shows chemical data for compounds of Formula I wherein X is OCH₃, R is hydrogen, G is methylene and the other substituents are as indicated below.

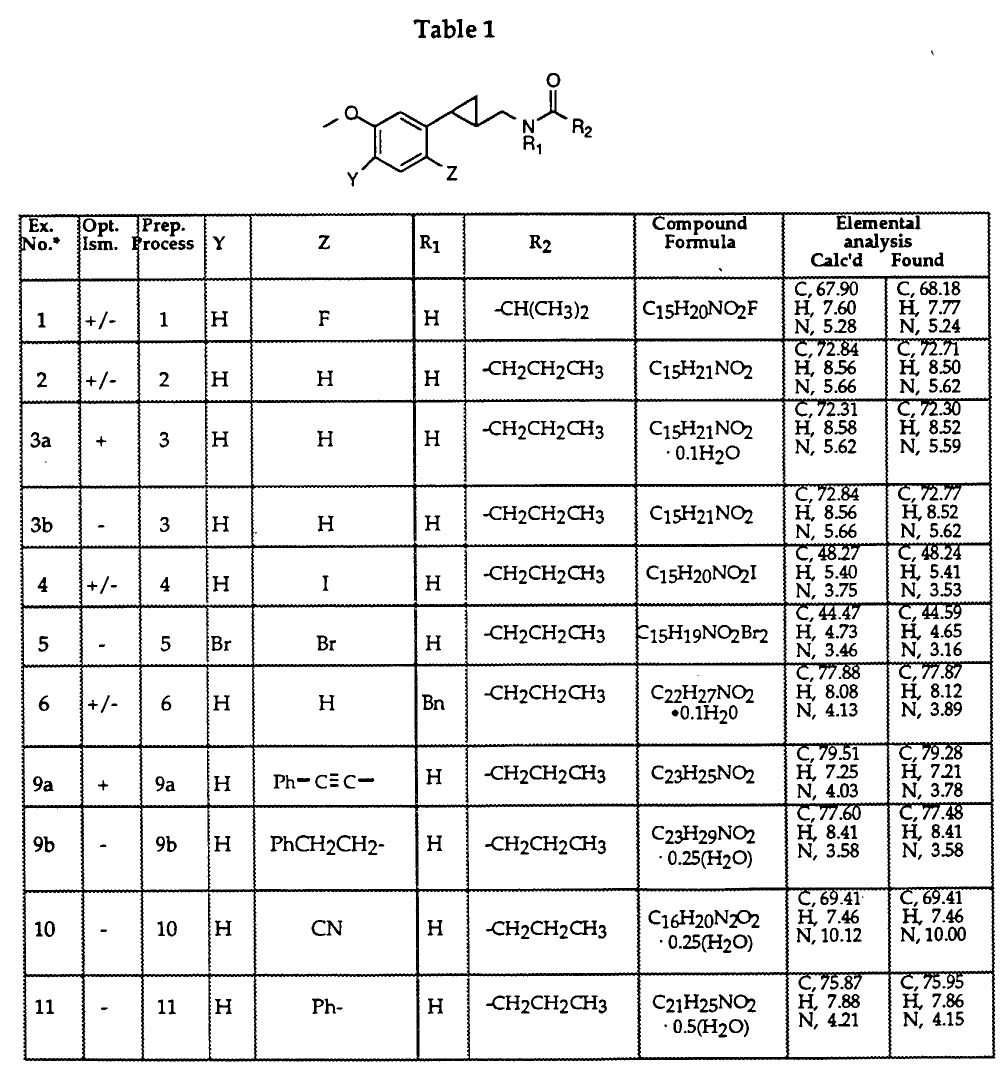

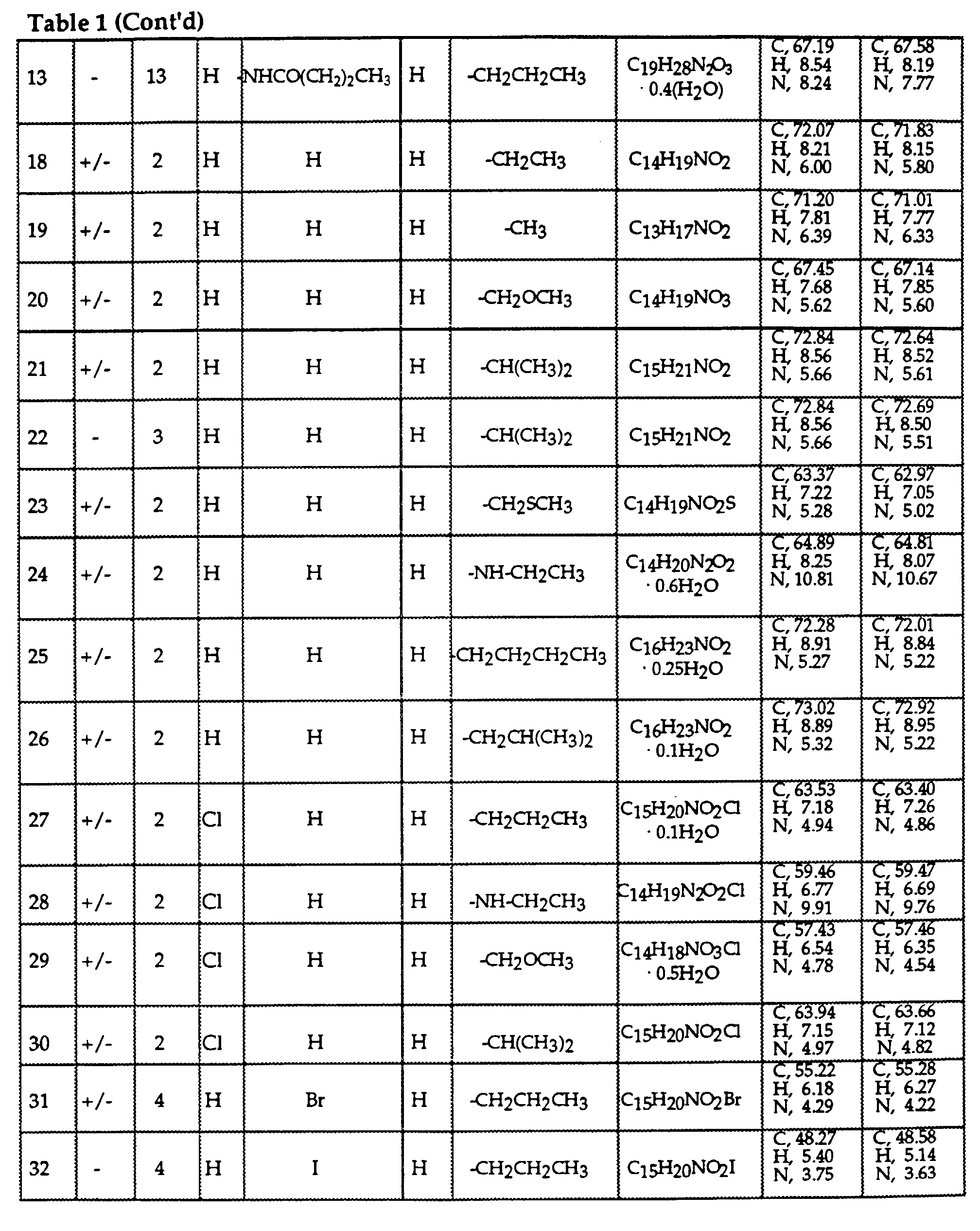

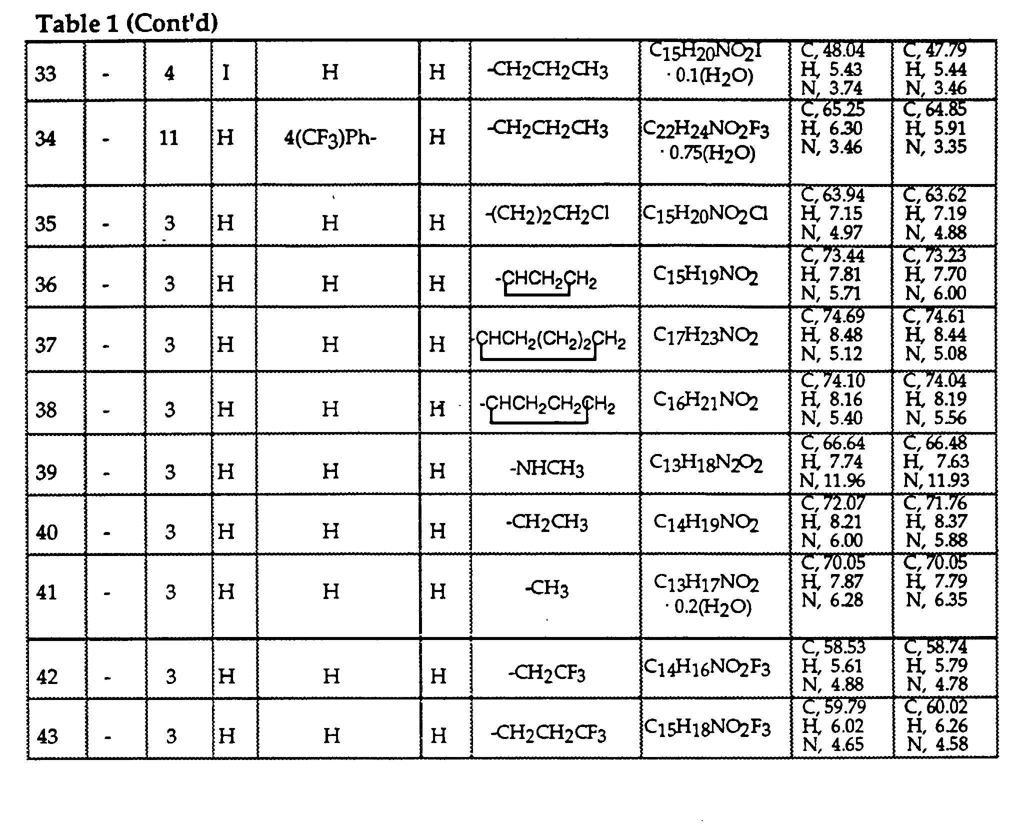

Table 2 gives chemical data for the Formula I compounds wherein R₁ and R are hydrogen, G is methylene and R₂, X ,Y, and Z are defined in the table.

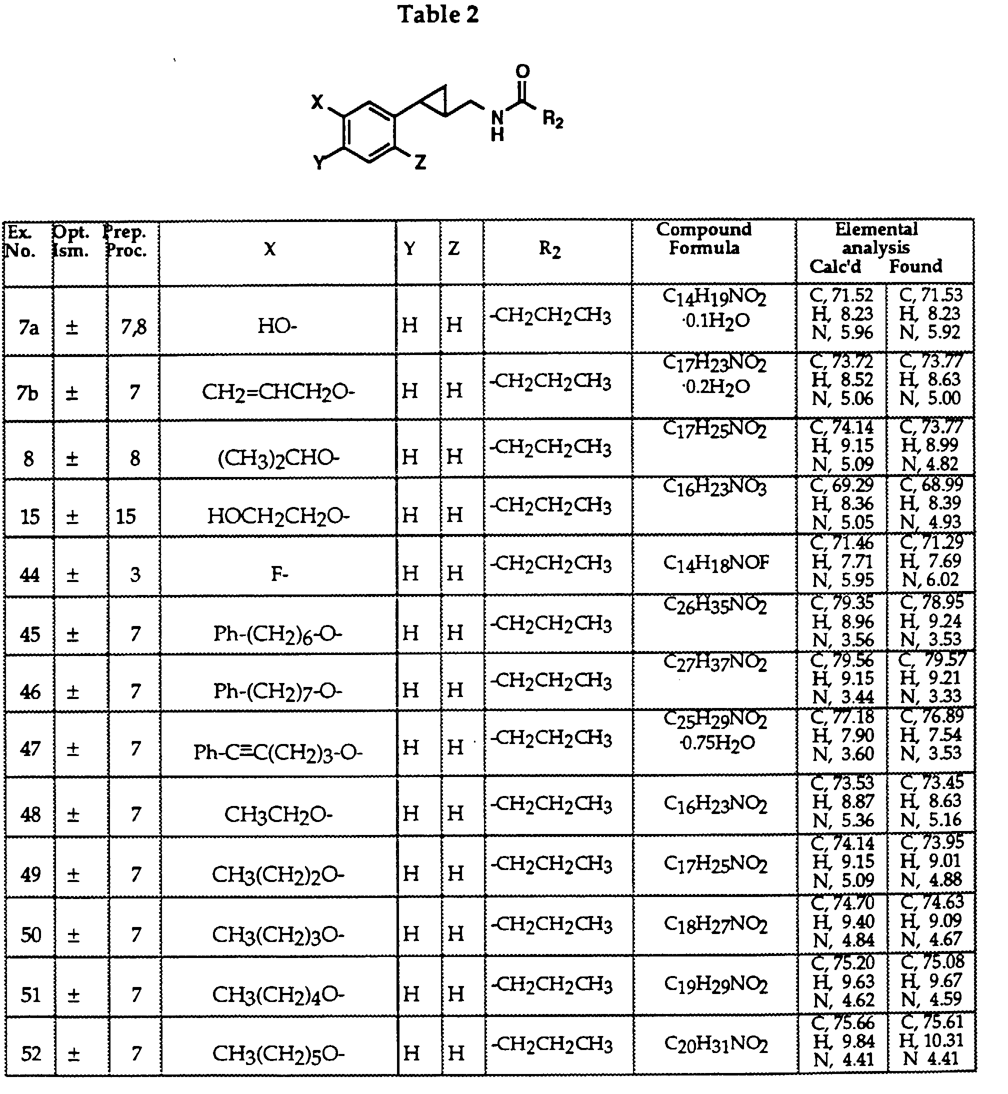

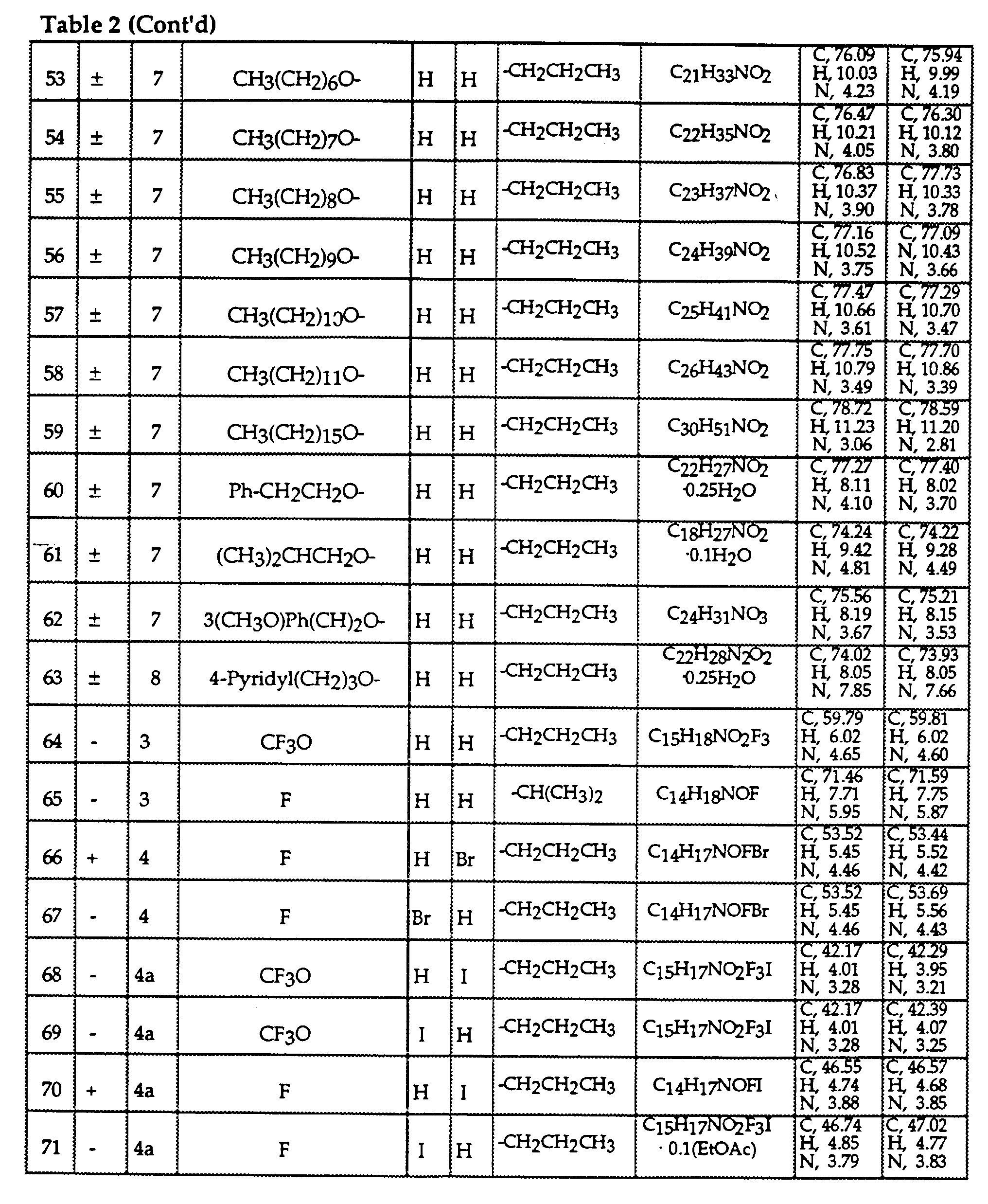

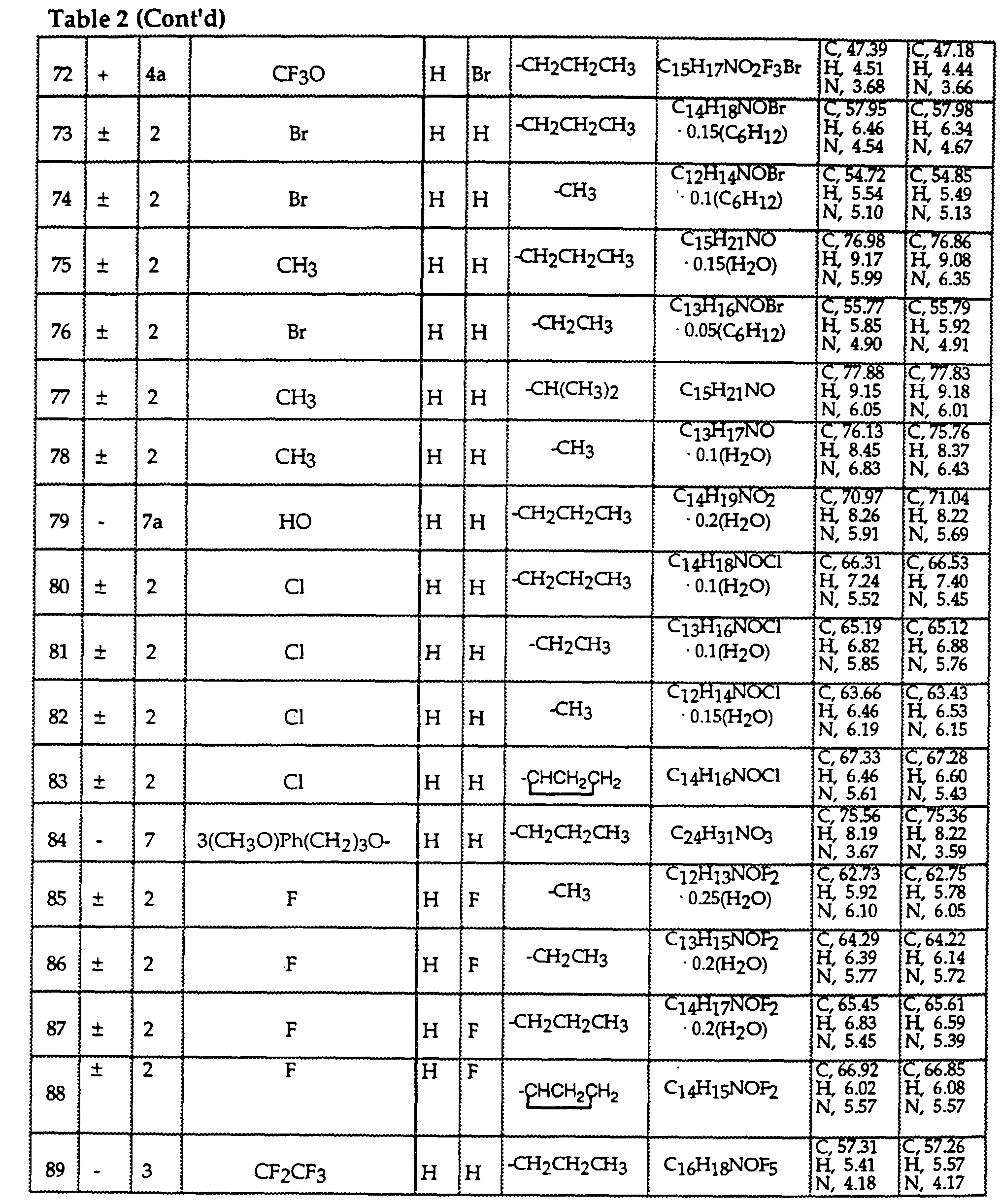

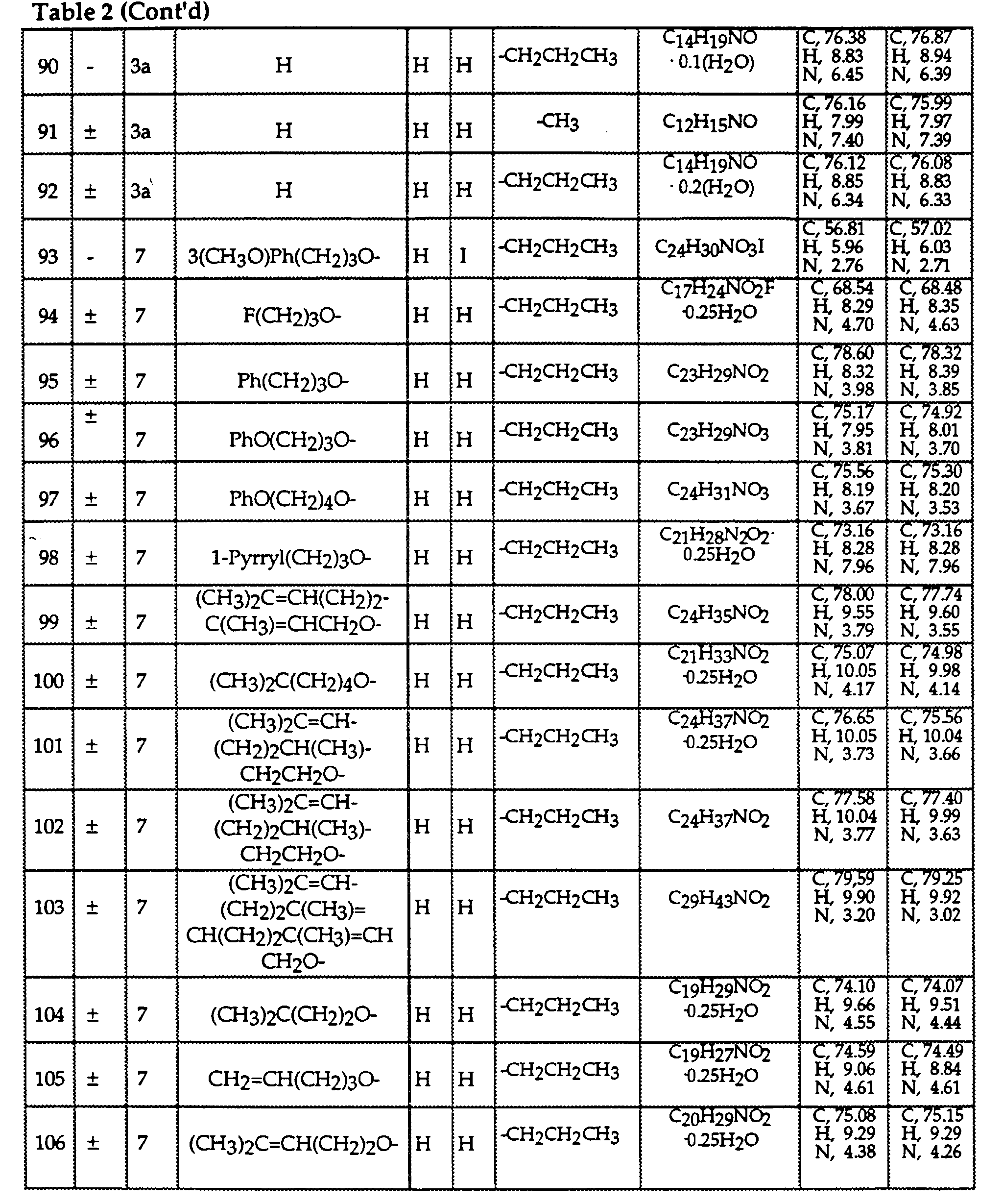

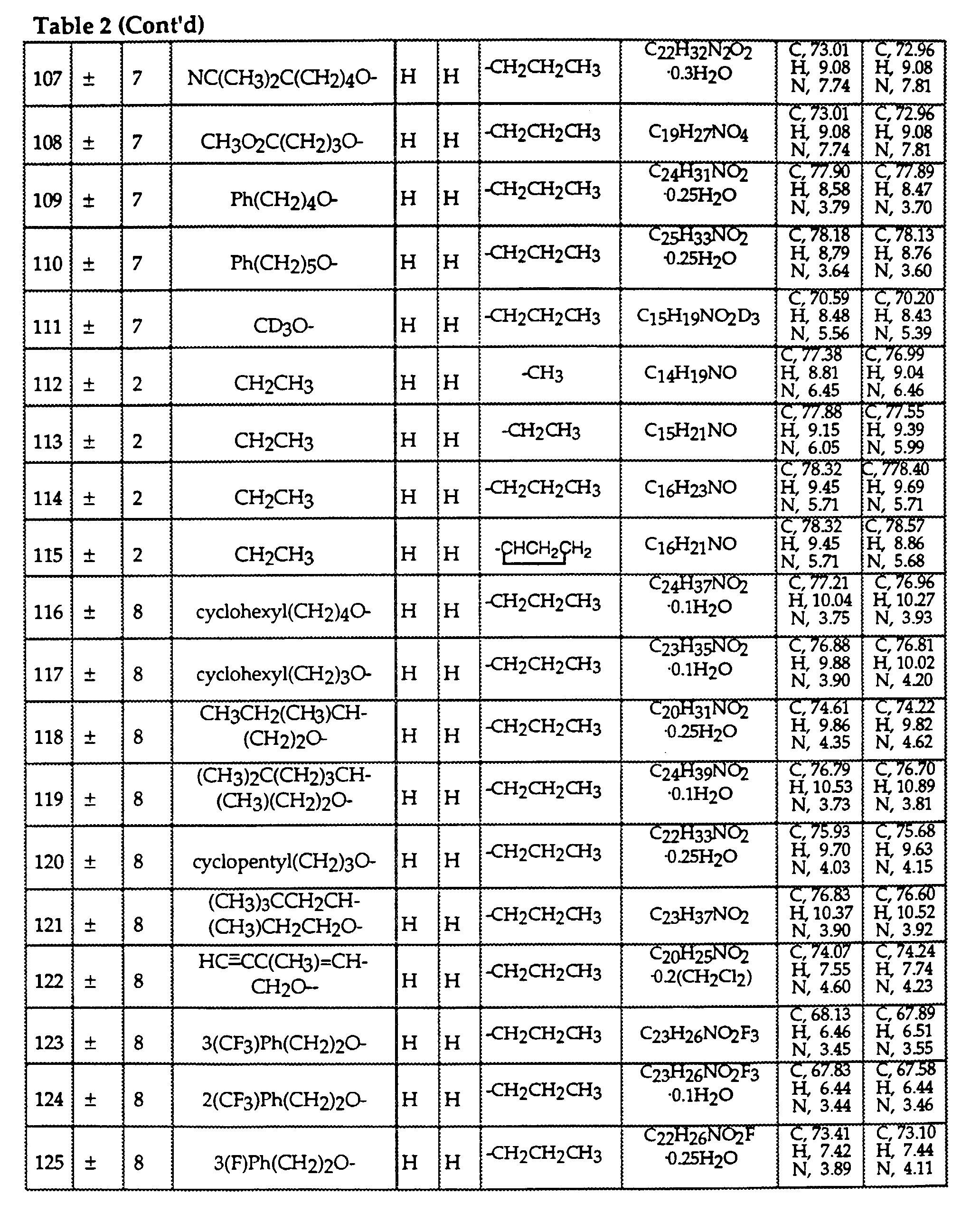

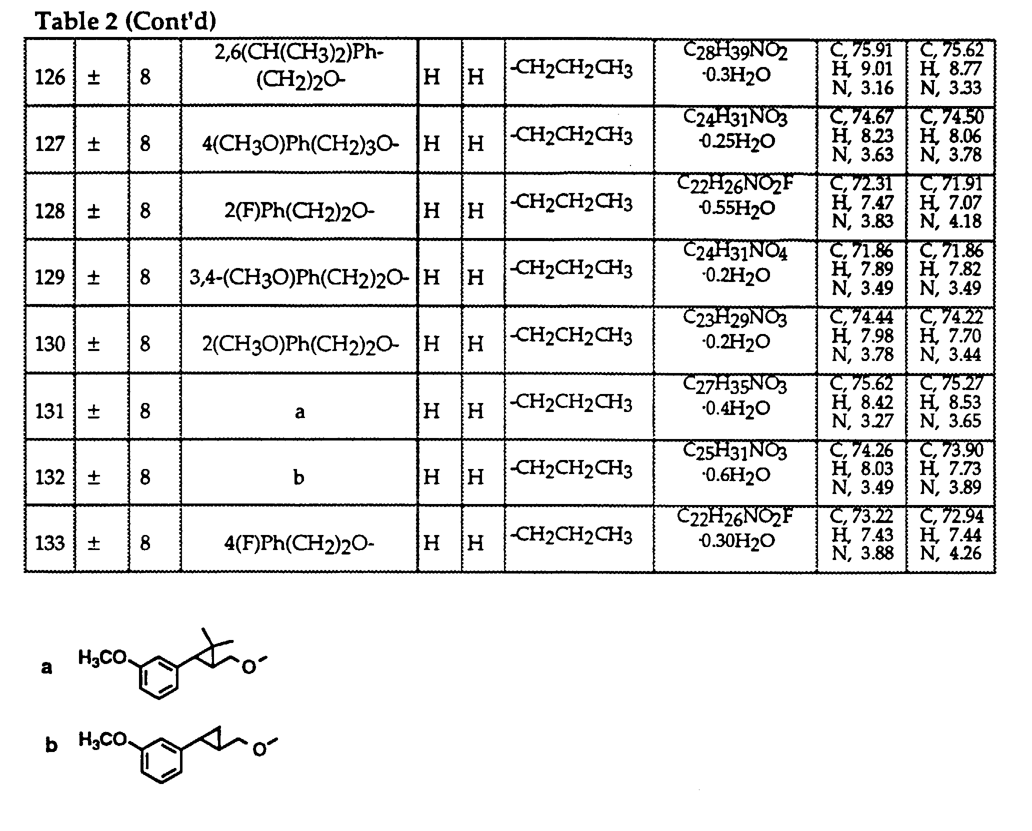

Table 3 shows chemical data for compounds of Formula I wherein X is OCH₃, Y and Z are H, and R₁ is H. The substituents R, G, and R₂ are as indicated in the table below.

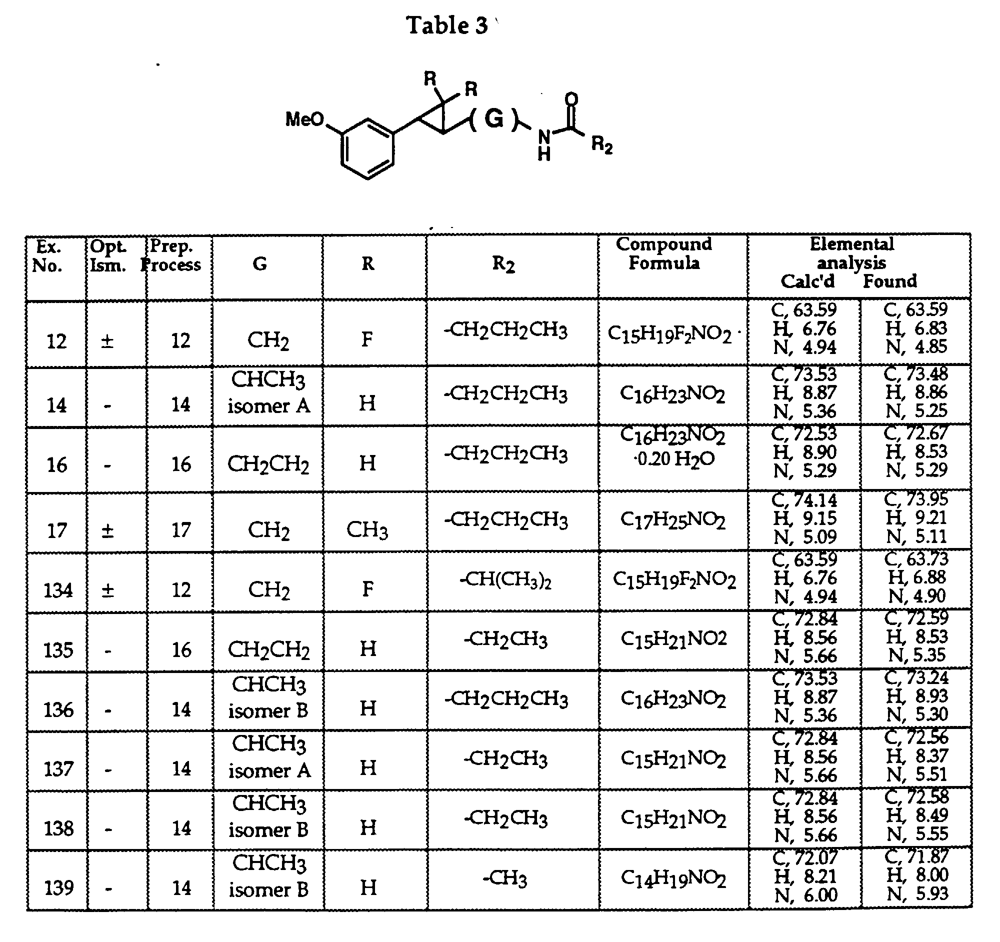

### Preparation of Compounds of Formula II from a 2-cycloalkenone:

### Example 140

### Mixture of cis- and trans-N-[3-(3-methoxyphenyl)-cyclopentyl]butanamide

A solution of 2-cyclopentenone (8.21 g, 0.1 mol) in THF (50 ml) was added dropwise to the Grignard reagent formed from 3-bromo anisole (18.7 g, 0.1 mol) and magnesium metal (2.5 g) in THF (200 ml). The solution was stirred for 18 hr, and then quenched with the slow addition of 3N HCl (75 ml). The mixture was stirred for 1 hr and diluted with ethyl ether (200 ml). The organic layer was separated, extracted twice with water, and twice with brine. The organic solution was then concentrated to an amber oil that was chromatographed on silica gel using CH₂Cl₂/hexanes mixture as the eluent to give the desired cyclopentanone (2.0 g, 10.5%, IR: 1734 cm⁻¹).

A solution of the above cyclopentanone (1.9 g, 10 mmol), hydroxylamine hydrochloride (2.8 g, 40 mmol), and sodium hydroxide (4 ml of 10 N, 40 mmol) in ethanol (150 ml), was refluxed for 18 hr. The mixture was cooled and then hydrogenated at 60 psi for 4 hr over Raney nickel (4 g). The mixture was filtered and the filtrate was concentrated in vacuo. The residue was dissolved in acetonitrile (100 ml) and HCl (2 ml of 12 N), and the solution was concentrated *in vacuo*. The residue was triturated with acetonitrile to give a gummy solid that recrystallized from ethanol to give the amine hydrochloride a white powder (0.5 g, 26.2%).

A solution of the amine salt intermediate (0.5 g, 2.62 mmol) in pyridine (10 ml) was cooled in an ice bath as butyryl chloride (0.363 ml, 3.5 mmol) was added. The reaction mixture was stirred for 4 hr at room temperature, and then concentrated. The residue was dissolved in methylene chloride (50 ml) and extracted twice with 1N HCl (30 ml). The organic layer was concentrated and the crude product was chromatographed on silica gel using methylene chloride/ethyl acetate as the eluent to give a mixture of cis- and trans-N-[3-(3-methoxyphenyl)-cyclopentyl]-butanamide as a clear oil (0.3 g, 43.8 %). Calc'd for C₁₆H₂₃NO₃: C, 74.53; H, 8.87; N, 5.36. Found: C, 73.18; H, 8.79; N, 5.36. MS (isobutane-DCI): 262 (M+H). IR (film): 3288, 2960, 1638, 1548, 1264, 698 cm⁻¹.

### Preparation of Compounds of Formula II from a 3-alkoxy-cycloalkenone:

### Example 141

### (cis)-N-[3-(3-Methoxyphenyl)cyclohexyl]butanamide

A solution of 3-(2-propyloxy)-2-cyclohexenone (28.0 g, 0.182 mol) in THF (100 ml) was added dropwise to the Grignard reagent formed from 3-bromo-anisole (37.4 g, 0.2 mol) and magnesium metal (6.08 g, 0.25 mol) in THF (400 ml). The solution was stirred for 3 hr, and then quenched with the slow addition of 3N HCl (150 ml). The mixture was stirred for 16 hr and diluted with ethyl ether (400 ml). The organic layer was separated, extracted twice with water (100 ml), and twice with brine. The organic solution was then concentrated to an amber oil that was Kügelrohr distilled to give the intermediate enone as a pale amber oil (35.1 g, 95.5%). PMR (CDCl₃, 300 MHz): δ 2.17 (m, 2H), 2.50 (t, 2H), 2.77 (t, 2H), 3.85 (s, 3H), 6.41 (s, 1H), 6.97 (d of d, 1H), 7.06 (t, 1H), 7.15 (d, 1H), 7.30 (t, 1H). IR (film): 1663 cm⁻¹.

A mixture of the above amber oil (35.1 g, 0.174 mol), ethylene glycol (15 g, 0.242 mol), and p-toluene sulfonic acid (0.5 g) in toluene (200 ml) was heated to reflux under a Dean-Stark trap for 18 hr. Another portion of p-toluene sulfonic acid (0.5 g) was added and the heating continued 24 hr. The solution was cooled and extracted twice with saturated sodium carbonate (100 ml), twice with water, and twice with brine. The organic layer was concentrated to a brown oil that was Kügelrohr distilled to give the intermediate ketal as a clear oil that was taken on. PMR (CDCl₃, 300 MHz): δ 1.85 (t, 2H), 2.4-2.5 (m, 2H), 2.66 (bs, 2H), 3.84 (s, 3H), 4.04 (s, 4H), 6.17 (m, 1H), 6.80 (d of d, 1H), 8.95 (t, 1H), 7.00 (d, 1H), 7.25 (t, 1H).

A solution of the intermediate oil in ethanol (200 ml) was hydrogenated for 18 hr at 60 psi over 10% Pd/C (1 g). The mixture was filtered, and the filtrate was stirred with 3N HCl (75 ml) for 4 hr. The solution was concentrated and extracted three times with ethyl acetate (200 ml). The organic extracts were dried with brine and concentrated. The oily residue was Kügelrohr distilled to the intermediate ketone as a clear oil (29.97 g, 95%). PMR (CDCl₃, 300 MHz): δ 1.73-1.95 (m, 2H), 2.05-2.2 (m, 2H), 2.33-2.63 (m, 4H), 2.95-3.07 (m, 1H), 3.87 (s, 3H), 6.70-6.85 (overlapping, 3H), 7.20-7.30 (m, 1H). IR (film): 1711 cm⁻¹.

A solution of the above ketone (10.86 g, 53.24 mmol) in ethanol (150 ml) was added to a mixture of hydroxylamine sulfate (12.31 g, 0.15 mol) and 10N sodium hydroxide (15 ml) and then heated to reflux for 6 hr. The mixture was cooled and then hydrogenated at 60 psi over Raney nickel (2 g) for 16 hr. The mixture was filtered and the filtrate concentrated to a gummy residue. The residue was partitioned between ethyl acetate and water. The organic layer was separated, extracted twice with water (75 ml), and then three times with 3N HCl (100 ml total). The HCl extracts were made basic with 50% sodium hydroxide, and then extracted three times with ethyl acetate (100 ml). The organic extracts were dried over brine and concentrated. The residue was dissolved in acetonitrile (50 ml) and 12N HCl was added dropwise to give the amine hydrochloride intermediate as a white precipitate that was filtered and air dried (2.54 g, 19.8%).

A solution of the amine salt intermediate (0.53 g, 2.59 mmol) in pyridine (10 ml) was cooled in an ice bath as butyryl chloride (0.33 mL, 3.2 mmol) was added via a syringe. The reaction mixture was stirred for 2 hr at room temperature, and then concentrated. The residue was dissolved in methylene chloride (30 ml) and extracted twice with 1N HCl (30 ml). The organic layer was concentrated and the crude product was chromatographed on silica gel using methylene chloride/ethyl acetate as the eluent to give (±)-cis-N-[3-(3-methoxyphenyl)-cyclohexyl]-butyramide as a clear oil (0.45 g, 62.7%).
Calc'd for C₁₇H₂₅NO₂ • 0.2 H₂O: C, 73.18; H, 9.18; N, 5.02.
Found: C, 73.27; H, 9.17; N, 5.01.
MS (isobutane-DCI): 276 (M+H).
IR (film): 3286, 2932, 1640, 1548, 1268, 698 cm⁻¹.
CMR (CDCl₃): δ 13.68, 19.25, 25.05, 32.93, 32.98, 38.93, 41.10, 43.12, 48.43, 55.12, 111.18, 112.70, 119.11, 129.31, 147.87, 159.60, 171.98 ppm.

### Example 142

### (±)-cis -N-[3-(3-methoxyphenyl)-cyclohexyl]-2-methyl-propanamide

This compound was prepared in a manner similar to that given in Example 2, and was isolated as a white powder (0.49 g, 68.3 %, mp: 89-92°C).
Calc'd for C₁₇H₂₅NO₂ • 0.09 H₂O: C, 73.71; H, 9.16; N, 5.06.
Found: C, 73.78; H, 9.16; N, 5.01.
MS (isobutane-DCI): 276 (M+H).
IR (film): 3290, 2930, 1642, 1544, 1236, 698 cm⁻¹.
CMR (CDCl₃): δ 19.60, 19.67, 25.08, 32.91, 32.96, 35.78, 41.17, 43.16, 48.31, 55.15, 111.23, 112.73, 119.14, 129.33, 147.91, 159.66, 175.97 ppm.

### Example 143

### (±)-cis-N-[3-(3-methoxyphenyl)cyclohexyl]acetamide

This compound was prepared in a manner similar to that given in Example 2, and was isolated as a clear oil (0.25 g, 38.8 %).
Calc'd for C₁₅H₂₁NO₂ • 0.1 H₂O: C, 72.29; H, 8.59; N, 5.60.
Found: C, 72.31; H, 8.58; N, 5.62.
MS (isobutane-DCI): 248 (M+H).
IR (film): 3284, 2932, 1652, 1554, 1270, 698 cm⁻¹.
CMR (CDCl₃): δ 23.56, 25.07, 32.93, 33.01, 40.95, 43.12, 48.71, 55.15, 111.23, 112.73,119.13,129.34,147.84,159.65,169.08 ppm.

### Example 144

### (cis)-N-Ethyl-N'-[3-(3-methoxyphenyl)cyclohexyl]urea

This compound was prepared in a manner similar to that given in Example 2, and was isolated as a clear oil (0.36 g, 49.2 %).
Calc'd for C₁₆H₂₄N₂O₂ • 0.35 H₂O: C, 67.98; H, 8.81; N, 9.91.
Found: C, 67.92; H, 9.01; N, 9.99.
MS (isobutane-DCI): 277 (M+H).
IR (film): 2930, 1632, 1570, 1268, 1156, 1048 cm⁻¹.
CMR (CDCl₃): δ 15.39, 25.15, 33.07, 33.58, 35.35, 41.61, 43.25, 49.67, 55.12, 111.13, 112.13, 119.13, 129.30, 147.93, 157.67, 159.58 ppm.

### Example 145 (Measurement of Melatonergic Binding)

The melatonergic binding of the compounds of Formula I was determined by a modification of the method of Reppert, S. M. et al. (*Neuron*, Volume 13, pages 1177-1185, 1994). Compounds with IC₅₀ values of 600 nM or less are termed active. The reagents, membranes, and other parameters used in the assay are described below:
Reagents:
(a) 50 mM Tris buffer containing 12.5 mM MgCl₂, and 2 mM EDTA (pH 7.4 at 37 ^{°}C).
(b) Wash buffer: 20 mM Tris base containing 2 mM MgCl₂ (pH 7.4 at room temperature).
(c) 6-Chloromelatonin (10⁻⁵ M final concentration).
(d) 2-[¹²⁵I]-Iodomelatonin (100 pM final concentration). (Source: NEN) Membrane preparation: The cDNA (human ML_{1A}) in pcDNAI was introduced into COS-1 cells by the DEAE-dextran method. Three days later, after media removal, the plates were washed with buffered saline; the cells removed using Hank's balanced salt solution and pelleted. The supernant was discarded and the pellets frozen. For membrane homogenates, the pellets were thawed and resuspended in TME buffer, Tris base, MgCl₂, EDTA (pH 7.4 at 37 °C), and supplemented with aprotinin, leupeptin, and phenylmethylsulfonyl fluoride. The cells were homogenized and centrifuged; the resulting pellet resuspended in TME and frozen. At assay, the small aliquot was thawed on ice and resuspended in TME buffer. Incubation: 37 ^{°}C for 1 hour. Reaction is terminated by filtration.

Table 4 presents the melatonergic activity for Formula I compounds wherein X is OCH₃, R is hydrogen, G is methylene, and the other substituents are as indicated in the table.

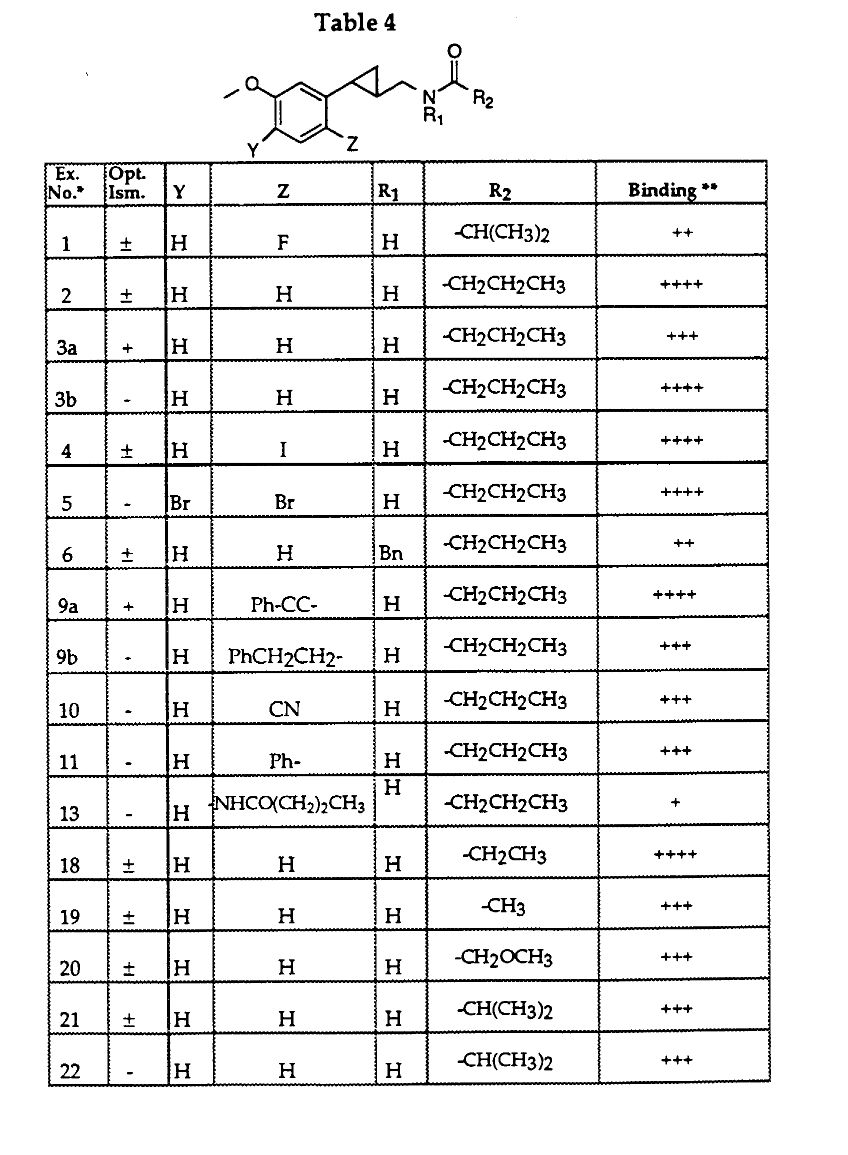

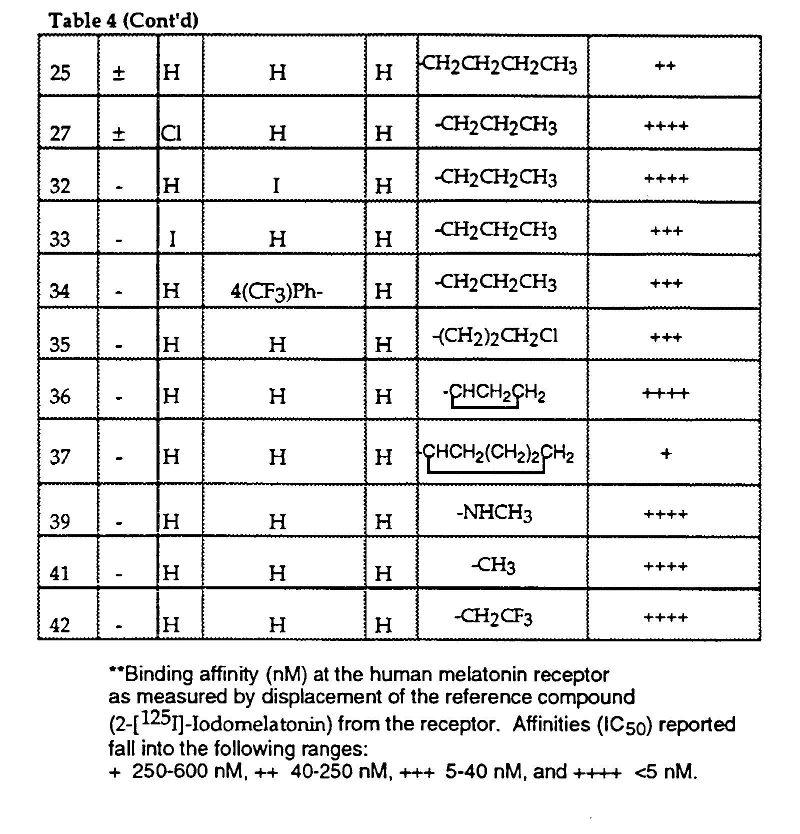

Table 5 gives the melatonergic activity for the Formula I compounds wherein R₁ and R are hydrogen, G is methylene and R₂ ,X ,Y, and Z are defined in the table.

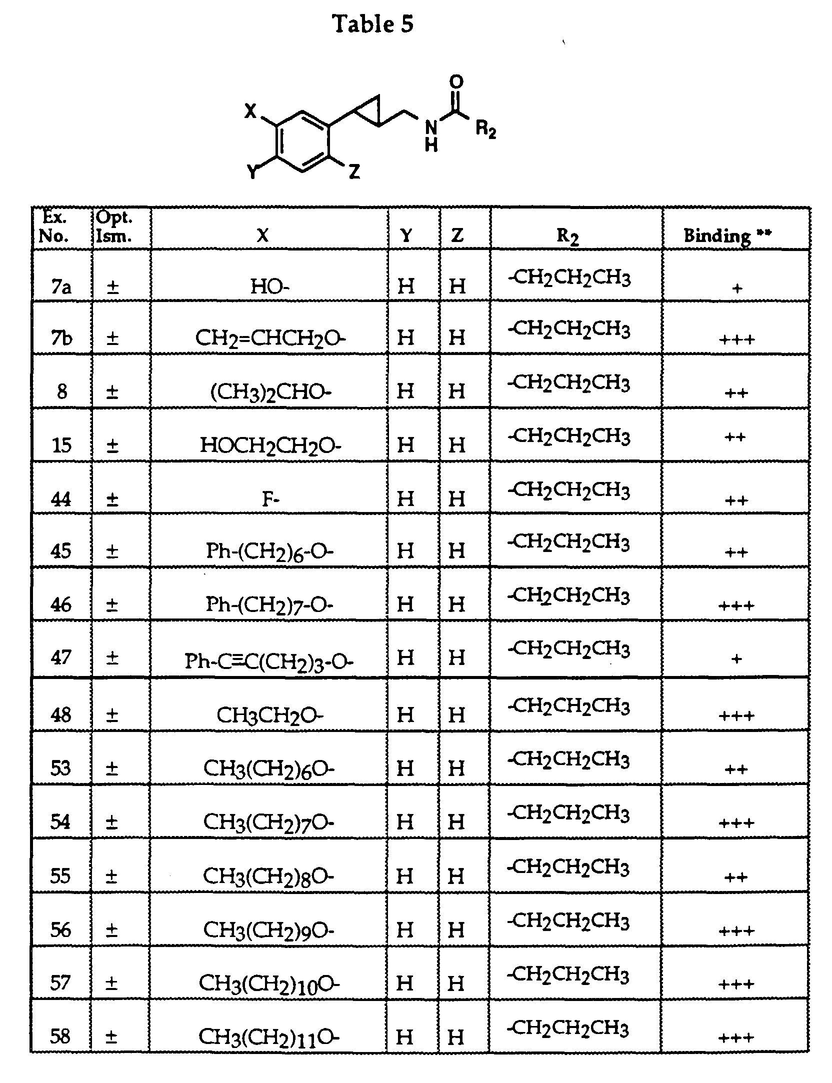

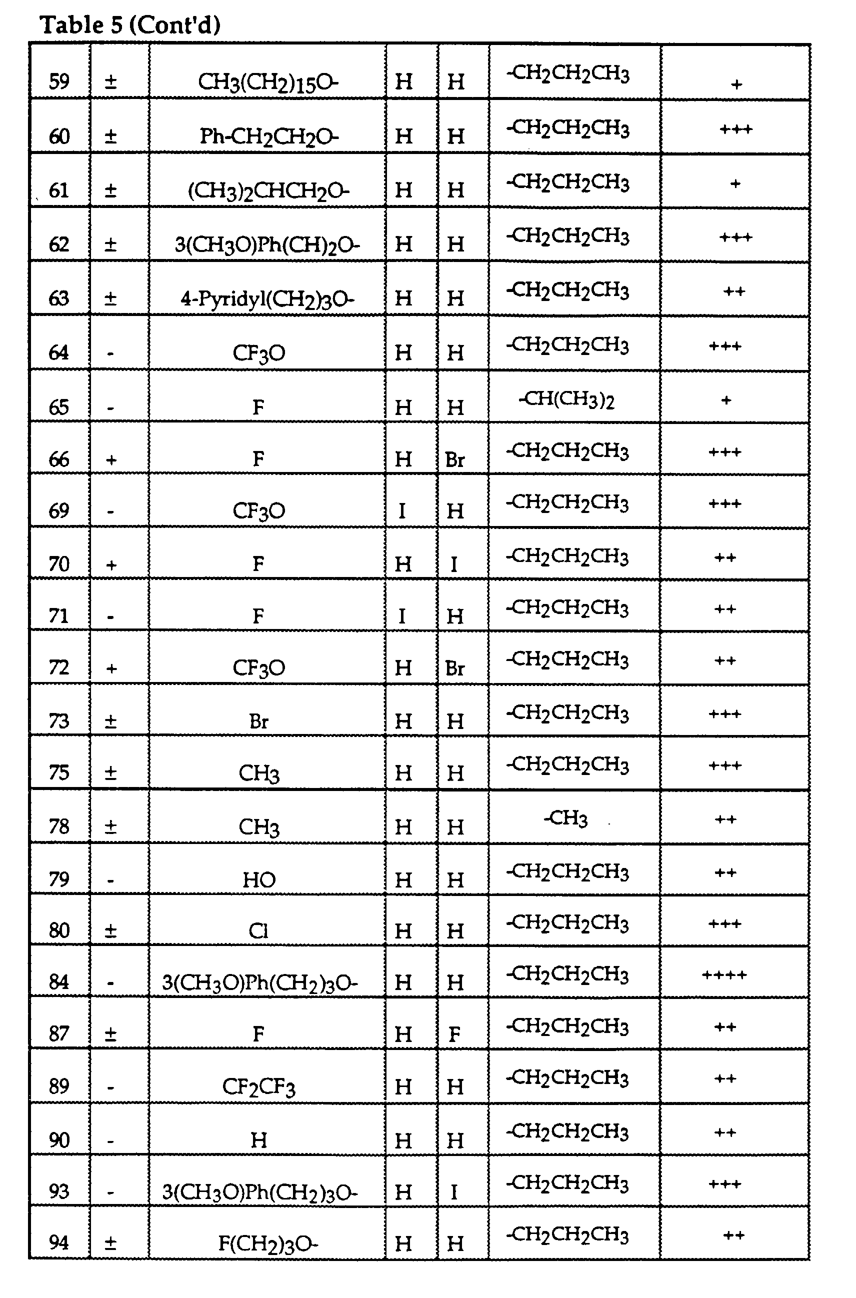

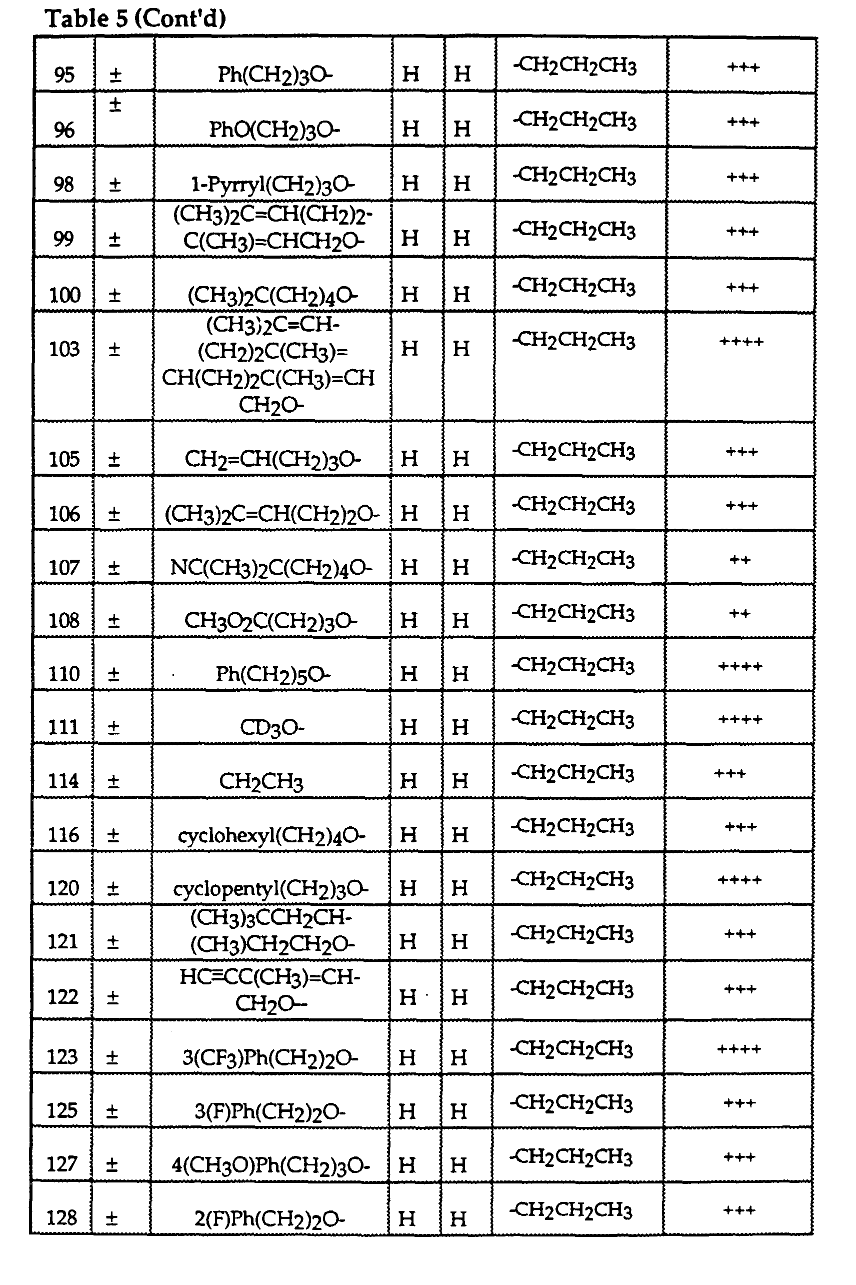

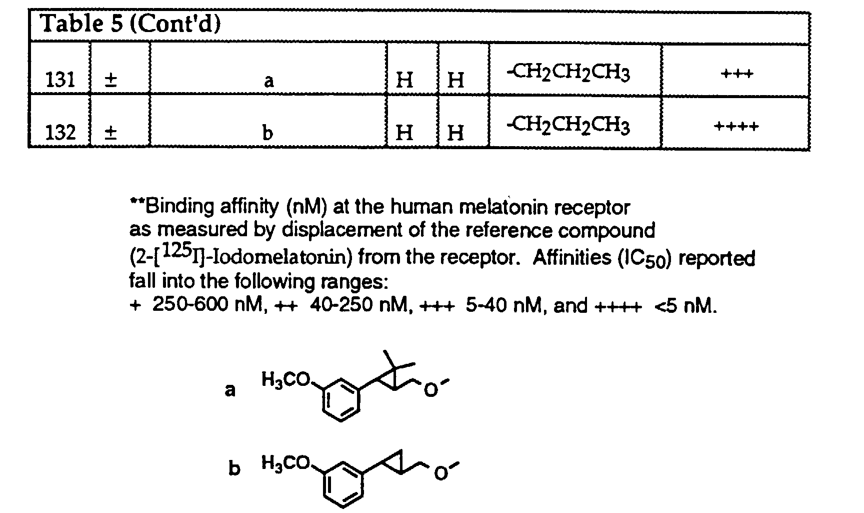

Table 6 presents yhe melatonergic activity for compounds of Formula I wherein X is OCH₃, Y and Z are H, and R₁ is H. The substituents R, G, and R₂ are as indicated in the table below.

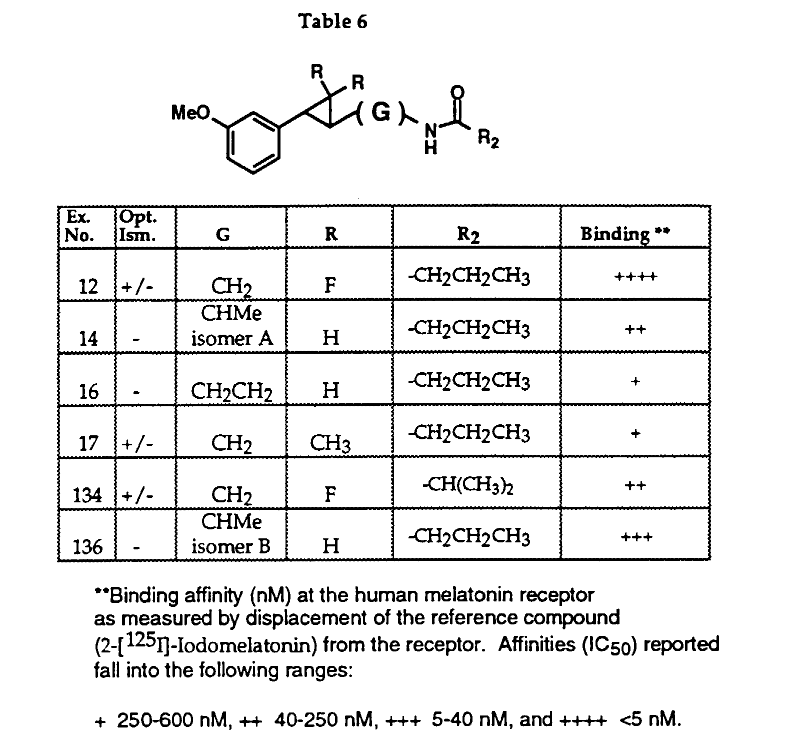

Reasonable variations, such as those which would occur to a skilled artisan, can be made herein without departing from the scope of the invention.

## Claims

1. A melatonergic compound of Formula I or II: 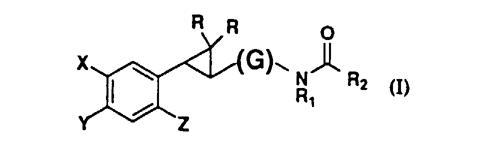 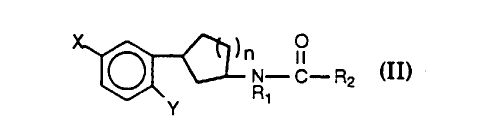 wherein:
X is halogen, hydrogen, C₁₋₄ alkyl or OR₅, wherein R₅ is hydrogen, C₁₋₄ perfluoroalkyl, C₁₋₄ fluoroalkyl, C₁₋₄ perdeuteroalkyl, C₁₋₂₀ alkyl, C₄₋₂₀ cycloalkylalkyl, C₂₋₂₀ carbonitriloalkyl, C₃₋₂₂ alkoxycarbonylalkyl, C₃₋₂₀ alkenyl, C₃₋₂₀ alkynyl, optionally substituted C₉₋₂₀ phenylalkyl, optionally substituted C₉₋₂₀ phenylalkenyl, optionally substituted C₉₋₂₀ phenylalkynyl, C₂₋₂₀ hydroxyalkyl, optionally substituted C₈₋₂₀ phenyloxyalkyl, C₇₋₂₀ pyridylalkyl, or C₆₋₂₀ pyrrylalkyl wherein the optional substituents are on the phenyl ring and are selected from halogen, trifluoromethyl and C₁₋₄ alkoxy;
Y is hydrogen or halogen;
Z is hydrogen, halogen, cyano, optionally substituted phenyl, optionally substituted C₇₋₂₀ phenylalkyl, optionally substituted C₈₋₂₀ phenylalkynyl, or C₂₋₂₀ alkamido wherein the optional substituents are on the phenyl ring and are selected from halogen, trifluoromethyl and C₁₋₄ alkoxy;
R, in both cases, is hydrogen, halogen, or C₁₋₄ alkyl;
G is a divalent methylene, ethylene, or C₁₋₄ alkmethylene moiety;
R₁ is hydrogen, C₁₋₄ alkyl or benzyl;
R₂ is C₁₋₆ alkyl, C₂₋₆ alkenyl, C₃₋₆ cycloalkyl, C₂₋₄ alkoxyalkyl, C₁₋₄ trifluoromethylalkyl, C₂₋₈ alkylthioalkyl or NR₃R₄, wherein R₃ and R₄ are independently selected from hydrogen and C₁₋₄ alkyl, but R₃ and R₄ cannot both be hydrogen; and
n is 1 or 2.

2. The compound of Claim 1 wherein R₁ is hydrogen; R₂ is C₁₋₆ alkyl, C₂₋₆ alkenyl or C₃₋₆cycloalkyl; Y and Z are hydrogen and X is methoxy.

3. The compound of Claim 2 selected from the group consisting of:
(*trans*)-N-[[2-(3-Methoxyphenyl)cyclopropyl]methyl] butanamide;
(-)(*trans*)-N-[[2-(3-Methoxyphenyl)cyclopropyl]methyl] butanamide;
(*trans*)-N-[[2-(3-Methoxyphenyl)cyclopropyl]methyl] propanamide;
(+)-(*trans*)-N-[[2-(3-Methoxyphenyl)cyclopropyl]-methyl] butanamide;
(*trans*)-N-[[2-(3-Methoxyphenyl)cyclopropyl]methyl] acetamide; and
(-)-(*trans*)-N-[[2-(3-Methoxyphenyl)cyclopropyl]methyl]-2-methylpropanamide.

4. The compound of Claim 2 wherein one or more of X, Y and Z is halogen.

5. The compound of Claim 4 selected from the group consisting of:
(*trans*)-N-[[2-(4-Chloro-3-methoxyphenyl)cyclopropyl]methyl] butanamide;
(*trans*)-N-[[2-(2-Brom-5-methoxyphenyl)cyclopropyl]methyl] butanamide;
(*trans*)-N-[[2-(2,4-Dibromo-5-methoxyphenyl)- cyclopropyl]methyl] butanamide;
(*trans*)-N-[[2-(2-Iodo-5-methoxyphenyl)cyclopropyl]methyl] butanamide; and
(-)-(*trans*)-N-[[2-(2-Iodo-5-methoxyphenyl)cyclopropyl]methyl] butanamide.

6. The compound of Claim 1 wherein R₁ is hydrogen, R₂ is alkyl, alkenyl or cycloalkyl, X and Z are hydrogen and R₅ is C₂₋₂₀ alkyl, C₉₋₂₀ aralkyl or C₃₋₂₀ alkenyl.

7. The compound of Claim 6 selected from the group consisting of:
(*trans*)-N-[[2-(3-[(Ethoxyphenyl)cyclopropyl]methyl] butanamide;
(*trans*)-N-[[2-[(3-Octyloxy)phenyl]cyclopropyl]methyl] butanamide;
(*trans*)-N-[[2-[(3-Decyloxy)phenyl]cyclopropyl]methyl] butanamide;
(*trans*)-N-[[2-[(3-Undecyloxy)phenyl]cyclopropyl]methyl] butanamide;
(*trans*)-N-[[2-[(3-Dodecyloxy)phenyl]cyclopropyl]methyl] butanamide;
(*trans*)-N-[[2-[3-(Heptyloxy)phenyl]cyclopropyl]methyl] butanamide;
(*trans*)-N-[[2-[3-(Nonyloxy)phenyl]cyclopropyl]methyl] butanamide;
(*trans*)-N-[[2-[3-(2-Propenyloxy)phenyl]cyclopropyl]methyl] butanamide;
(*trans*)-N-[[2-[3-(6-Phenylhexyl)oxy]phenyl]cyclopropyl]methyl] butanamide;
(*trans*)-N-[[2-[3-[(7-Phenylheptyl)oxy]phenyl]cyclopropyl]methyl] butanamide;
(*trans*)-N-[[2-[3-[(2-Phenylethyl)oxy]phenyl]cyclopropyl]methyl] butanamide; and
(*trans*)-N-[[2-[3-[3-(3-Methoxyphenyl)propoxy]phenyl]cyclopropyl]methyl] butanamide.

8. Use of a compound of Claim 1 for manufacturing a medicament for treating a circadian rhythm-related disorder in a patient.

9. A pharmaceutical composition for treating circadian rhythm-related disorders comprising a suitable amount of a pharmaceutically acceptable carrier and a therapeutic amount of a compound of Claim 1.

10. A radioligand comprising a radiochemically substituted compound of Claim 1.

11. A radioligand comprising a radioiodinated compound derived from a compound of Claim 1.

12. The compound of Claim 1 selected from the group consisting of:
(*trans*)-N-[[2-(3-Chlorophenyl)cycloprop-1-yl]methyl] butanamide;
(*trans*)-N-[[2-(3-Bromophenyl)cycloprop-1-yl]methyl] butanamide;
(-)-(*trans*)-N-[[2-(3-Fluorophenyl)cyclopropyl]methyl] butanamide;
(*trans*)-N-[[2-(2,5-Difluorophenyl)cycloprop-1-yl]methyl] butanamide;
(*trans*)-N-[[2-(2-Bromo-5-fluorophenyl)cycloprop-1-yl]methyl] butanamide;
(*trans*)-N-[[2-(3-Methylphenyl)cycloprop-1-yl]methyl] butanamide;
(*trans*)-N-[2-[(3-Ethylphenyl)cycloprop-1-yl]methyl] butanamide; and
(*trans*)-N-[[2-[3-(Trifluoromethoxy)phenyl]cycloprop-1-yl]methyl] butanamide,
(cis)-N-[3-(3-methoxyphenyl)cyclohexyl]acetamide;
(cis)-N-[3-(3-methoxyphenyl)cyclohexyl]-2-methyl-propanamide;
(cis)-N-[3-(3-methoxyphenyl)cyclohexyl]butanamide;
(cis)-N-Ethyl-N'-[3-(3-methoxyphenyl)cyclohexyl]urea; and
N-[3-(3-methoxyphenyl)cyclopentyl]butanamide.

13. A compound according to Claim 1 for use as a therapeutic agent.

## Revendications

1. Composé mélatonergique de formule I ou II: 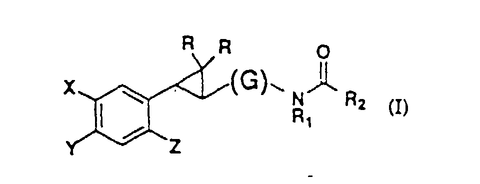 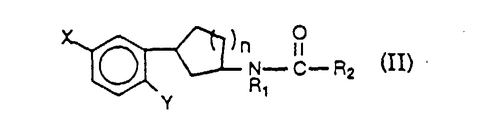 où :
X est un halogène, un hydrogène, un alkyle en C₁₋₄ ou OR₅, où R₅ est un hydrogène, un perfluoroalkyle en C₁₋₄, un fluoroalkyle en C₁₋₄, un perdeutéroalkyle en C₁₋₄, un alkyle en C₁₋₂₀, un cycloalkylalkyle en C₄₋₂₀, un carbonitriloalkyle en C₂₋₂₀, un alcoxycarbonylalkyle en C₃₋₂₂, un alcényle en C₃₋₂₀, un alcynyle en C₃₋₂₀, un phénylalkyle en C₉₋₂₀ optionnellement substitué, un phénylalcényle en C₉₋₂₀ optionnellement substitué, un phénylalcynyle en C₉₋₂₀ optionnellement substitué, un hydroxyalkyle en C₂₋₂₀, un phényloxyalkyle en C₈₋₂₀ optionnellement substitué, un pyridylalkyle en C₇₋₂₀, ou un pyrrylalkyle en C₆₋₂₀ où les substituants optionnels sont sur le cycle phényle et sont sélectionnés parmi un halogène, un trifluorométhyle et un alcoxy en C₁₋₄ ;
Y est un hydrogène ou un halogène ;
Z est un hydrogène, un halogène, un cyano, un phényle optionnellement substitué, un phénylalkyle en C₇₋₂₀ optionnellement substitué, un phénylalcynyle en C₈₋₂₀ optionnellement substitué, ou un alcamido en. C₂₋₂₀ où les substituants optionnels sont sur le cycle phényle et sont sélectionnés parmi un halogène, un trifluorométhyle et un alcoxy en C₁₋₄ ;
R, dans les deux cas, est un hydrogène, un halogène, ou un alkyle en C₁₋₄ ;
G est un méthylène divalent, un éthylène, ou une fraction alkméthylène en C₁₋₄ ;
R₁ est un hydrogène, un alkyle en C₁₋₄ ou un benzyle ;
R₂ est un alkyle en C₁₋₆, un alcényle en C₂₋₆, un cycloalkyle en C₃₋₆, un alcoxyalkyle en C₂₋₄, un trifluorométhylalkyle en C₁₋₄, un alkylthioalkyle en C₂₋₈ ou NR₃R₄, où R₃ et R₄ sont indépendamment sélectionnés parmi un hydrogène et un alkyle en C₁₋₄, mais R₃ et R₄ ne peuvent pas être tous deux un hydrogène ; et
n vaut 1 ou 2.

2. Composé selon la revendication 1 où R₁ est un hydrogène ; R₂ est un alkyle en C₁₋₆, un alcényle en C₂₋₆ ou un cycloalkyle en C₃₋₆ ; Y et Z sont un hydrogène et X est un méthoxy.

3. Composé selon la revendication 2 sélectionné dans le groupe consistant en :
le (*trans*)-N-[[2-(3-Méthoxyphényl)cyclopropyl]méthyl]butanamide ;
le (-)-(*trans*)-N-[[2-(3-Méthoxyphényl)cyclopropyl]méthyl]butanamide ;
le (*trans*)-N-[[2-(3-Méthoxyphényl)cyclopropyl]méthyl]propanamide ;
le (+)-(*trans*)-N-[[2-(3-Méthoxyphényl)cyclopropyl]méthyl]butanamide ;
le (*trans*)-N-[[2-(3-Méthoxyphényl)cyclopropyl]méthyl]acétamide ; et
le (-)-(*trans*)-N-[[2-(3-Méthoxyphényl)cyclopropyl]méthyl]-2-méthylpropanamide.

4. Composé selon la revendication 2, où un ou plus de X, Y et Z est un halogène.

5. Composé selon la revendication 4 sélectionné dans le groupe consistant en :
le (*trans*)-N-[[2-(4-Chloro-3-méthoxyphényl)cyclopropyl]méthyl]butanamide ;
le (*trans*)-N-[[2-(2-Bromo-5-méthoxyphényl)cyclopropyl]méthyl]butanamide ;
le (*trans*)-N-[[2-(2,4-Dibromo-5-méthoxyphényl)cyclopropyl]méthyl] butanamide ;
le (*trans*)-N-[[2-(2-lodo-5-méthoxyphényl)cyclopropyl]méthyl]butanamide ; et
le (-)-(*trans*)-N-[[2-(2-lodo-5-méthoxyphényl)cyclopropyl]méthyl]butanamide.

6. Composé selon la revendication 1 où R₁ est un hydrogène, R₂ est un alkyle, un alcényle ou un cycloalkyle, X et Y sont un hydrogène et R₅ est un alkyle en C₂₋₂₀, un aralkyle en C₉₋₂₀ ou un alcényle en C₃₋₂₀.

7. Composé selon la revendication 6 sélectionné dans le groupe consistant en :
le (*trans*)-N-[[2-(3-[Ethoxyphényl)cyclopropyl]méthyl]butanamide ;
le (*trans*)-N-[[2-[(3-Octyloxy)phényl)cyclopropyl]méthyl]butanamide ;
le (*trans*)-N-[[2-[(3-Décyloxy)phényl)cyclopropyl]méthyl]butanamide ;
le (*trans*)-N-[[2-[(3-Undécyloxy)phényl)cyclopropyl]méthyl]butanamide ;
le (*trans*)-N-[[2-[(3-Dodécyloxy)phényl)cyclopropyl]méthyl]butanamide ;
le (*trans*)-N-[[2-[(3-Heptyloxy)phényl)cyclopropyl]méthyl]butanamide ;
le (*trans*)-N-[[2-[(3-Nonyloxy)phényl)cyclopropyl]méthyl]butanamide ;
le (*trans*)-N-[[2-[(3-Propényloxy)phényl)cyclopropyl]méthyl]butanamide ;
le (trans)-N-[[2-[(3-(6-Phénylhexyl)oxy]phényl]cyclopropyl]méthyl] butanamide ;
le (*trans*)-N-[[2-[(3-[(7-Phénylheptyl)oxy]phényl]cyclopropyl]méthyl] butanamide ;
le (*trans*)-N-[[2-[(3-[(2-Phényléthyl)oxy]phényl]cyclopropyl]méthyl] butanamide ; et
le (*trans*)-N-[[2-[3-[3-(3-Méthoxyphényl)propoxy]phényl)cyclopropyl]méthyl] butanamide.

8. Utilisation d'un composé selon la revendication 1 pour la fabrication d'un médicament pour traiter un désordre relié au rythme circadien chez un patient.

9. Composition pharmaceutique pour traiter des désordres reliés au rythme circadien comprenant une quantité appropriée d'un porteur pharmaceutiquement acceptable et une quantité thérapeutique d'un composé selon la revendication 1.

10. Radioligand comprenant un composé radiochimiquement substitué selon la revendication 1.

11. Radioligand comprenant un composé radioiodé dérivé d'un composé selon la revendication 1.

12. Composé selon la revendication 1, sélectionné dans le groupe consistant en :
le (*trans*)-N-[[2-(3-Chlorophényl)cycloprop-1-yl]méthyl]butanamide ;
le (*trans*)-N-[[2-(3-Bromophényl)cycloprop-1-yl]méthyl]butanamide ;
le (-)-(*trans*)-N-[[2-(3-Fluorophényl)cyclopropyl]méthyl]butanamide ;
le (*trans*)-N-[[2-(2,5-(Difluorophényl)cycloprop-1-yl]méthyl]butanamide ;
le (*trans*)-N-[[2-(2-Bromo-5-fluorophényl)cycloprop-1-yl]méthyl]butanamide ,
le (*trans*)-N-[[2-(3-Méthylphényl)cycloprop-1-yl]méthyl]butanamide ;
le (*trans*)-N-[[2-[(3-Ethylphényl)cycloprop-1-yl]méthyl]butanamide ; et
le (*trans*)-N-[[2-[3-(Trifluorométhoxy)phényl]cycloprop-1-yl]méthyl] butanamide,
le (cis)-N-[3-(3-méthoxyphényl)cyclohexyl]acétamide ;
le (cis)-N-[3-(3-méthoxyphényl)cyclohexyl]-2-méthyl-propanamide ;
le (cis)-N-[3-(3-méthoxyphényl)cyclohexyl]butanamide ;
la (cis)-N-Ethyl-N'-[3-(3-méthoxyphényl)cyclohexyl]urée; et
le N-[3-[3-méthoxyphényl)cyclopentyl]butanamide.

13. Composé selon la revendication 1 pour utilisation en tant qu'agent thérapeutique.

## Patentansprüche

1. Melatonerge Verbindung der Formel I oder II: 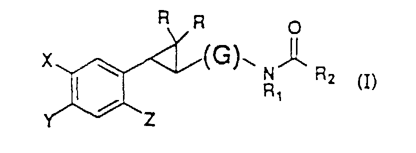 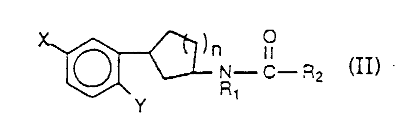 in der
X ein Halogenatom, ein Wasserstoffatom, einen C₁₋₄-Alkylrest oder einen Rest OR₅ darstellt, wobei R₅ ein Wasserstoffatom, einen C₁₋₄-Perfluoralkyl-, C₁₋₄-Fluoralkyl-, C₁₋₄-Perdeuteroalkyl-, C₁₋₂₀-Alkyl-, C₄₋₂₀-Cycloalkylalkyl-, C₂₋₂₀-Carbonitriloalkyl-, C₃₋₂₂-Alkoxycarbonylalkyl-, C₃₋₂₀-Alkenyl-, C₃₋₂₀-Alkinyl-, gegebenenfalls substituierten C₉₋₂₀-Phenylalkyl-, gegebenenfalls substituierten C₉₋₂₀-Phenylalkenyl-, gegebenenfalls substituierten C₉₋₂₀-Phenylalkinyl-, C₂₋₂₀-Hydroxyalkyl-, gegebenenfalls substituierten C₈₋₂₀-Phenyloxyalkyl-, C₇₋₂₀-Pyridylalkyl- oder C₆₋₂₀-Pyrrylalkylrest bedeutet, wobei sich die möglichen Substituenten am Phenylring befinden und aus einem Halogenatom, einer Trifluormethylgruppe und einem C₁₋₄-Alkoxyrest ausgewählt sind;
Y ein Wasserstoff- oder Halogenatom darstellt;
Z ein Wasserstoffatom, ein Halogenatom, eine Cyanogruppe, eine gegebenenfalls substituierte Phenylgruppe, einen gegebenenfalls substituierten C₇₋₂₀-Phenylalkylrest, einen gegebenenfalls substituierten C₈₋₂₀-Phenylalkinylrest oder einen C₂₋₂₀-Alkamidorest bedeutet, wobei sich die möglichen Substituenten am Phenylring befinden und aus einem Halogenatom, einer Trifluormethylgruppe und einem C₁₋₄-Alkoxyrest ausgewählt sind;
R in beiden Fällen ein Wasserstoffatom, ein Halogenatom oder einen C₁₋₄-Alkylrest darstellt;
G eine divalente Methylen-, Ethylen- oder C₁₋₄-Alkmethyleneinheit bedeutet;
R₁ ein Wasserstoffatom, einen C₁₋₄-Alkylrest oder eine Benzylgruppe darstellt;
R₂ einen C₁₋₆-Alkyl-, C₂₋₆-Alkenyl-, C₃₋₆-Cycloalkyl-, C₂₋₄-Alkoxyalkyl-, C₁₋₄-Trifluormethylalkyl-, C₂₋₈-Alkylthioalkylrest oder einen Rest NR₃R₄ bedeutet, wobei R₃ und R₄ unabhängig voneinander aus einem Wasserstoffatom und einem C₁₋₄-Alkylrest ausgewählt sind, aber R₃ und R₄ nicht beide Wasserstoffatome darstellen können; und
n 1 oder 2 ist.

2. Verbindung des Anspruchs 1, wobei R₁ ein Wasserstoffatom darstellt, R₂ einen C₁₋₆-Alkyl-, C₂₋₆-Alkenyl- oder C₃₋₆-cycloalkylrest bedeutet, Y und Z Wasserstoffatome darstellen und X eine Methoxygruppe bedeutet.

3. Verbindung des Anspruchs 2, ausgewählt aus der Gruppe:
(*trans*)-N-[[2-(3-Methoxyphenyl)cyclopropyl]methyl]butanamid;
(-)-(*trans*)-N-[[2-(3-Methoxyphenyl)cyclopropyl]methyl ]butanamid;
(*trans*)-N-[[2-(3-Methoxyphenyl)cyclopropyl]methyl]propanamid;
(+)-(*trans*)-N-[[2-(3-Methoxyphenyl)cyclopropyl]methyl]butanamid;
(*trans*)-N-[[2-(3-Methoxyphenyl)cyclopropyl]methyl]acetamid und
(-)-(*trans*)-N-[[2-(3-Methoxyphenyl)cyclopropyl]methyl]-2-methylpropanamid.

4. Verbindung des Anspruchs 2, wobei einer oder mehrere der Reste X, Y und Z Halogenatome darstellen.

5. Verbindung des Anspruchs 4, ausgewählt aus der Gruppe:
(*trans*)-N-[[2-(4-Chlor-3-methoxyphenyl)cyclopropyl]methyl]butanamid;
(*trans*)-N-[[2-(2-Brom-5-methoxyphenyl)cyclopropyl]methyl]butanamid;
(*trans*)-N-[[2-(2,4-Dibrom-5-methoxyphenyl)cyclopropyl]methyl]butanamid;
(*trans*)-N-[[2-(2-Jod-5-methoxyphenyl)cyclopropyl]methyl]butanamid und
(-)-(*trans*)-N- [[2-(2-Jod-5-methoxyphenyl)cyclopropyl]methyl]butanamid.

6. Verbindung des Anspruchs 1, wobei R₁ ein Wasserstoffatom darstellt, R₂ einen Alkyl-, Alkenyl- oder Cycloalkylrest bedeutet, X und Z Wasserstoffatome darstellen und R₅ einen C₂₋₂₀-Alkyl-, C₉₋₂₀-Aralkyl- oder C₃₋₂₀-Alkenylrest bedeutet.

7. Verbindung des Anspruchs 6, ausgewählt aus der Gruppe:
(*trans*)-N-[[2-[(3-Ethoxy)phenyl]cyclopropyl]methyl]butanamid;
(*trans*)-N-[[2-[(3-Octyloxy)phenyl]cyclopropyl]methyl]butanamid;
(*trans*)-N-[[2-[(3-Decyloxy)phenyl]cyclopropyl]methyl]butanamid;
(*trans*)-N-[[2-[(3-Undecyloxy)phenyl]cyclopropyl]methyl]butanamid;
(*trans*)-N-[[2-[(3-Dodecyloxy)phenyl]cyclopropyl]methyl]butanamid;
(*trans*)-N-[[2-[(3-Heptyloxy)phenyl]cyclopropyl]methyl]butanamid;
(*trans*)-N-[[2-[(3-Nonyloxy)phenyl]cyclopropyl]methyl]butanamid;
(*trans*)-N-[[2-[3-(2-Propenyloxy)phenyl]cyclopropyl]methyl]butanamid;
(*trans*)-N-[[2-[3-[(6-Phenylhexyl)oxy]phenyl]cyclopropyl]methyl]butanamid;
(*trans*)-N-[[2-[3-[(7-Phenylheptyl)oxy]phenyl]cyclopropyl]methyl]butanamid;
(*trans*)-N-[[2-[3-[(2-Phenylethyl)oxy]phenyl]cyclopropyl]methyl]butanamid und
(*trans*)-N-[[2-[3-[3-(3-Methoxyphenyl)propoxy]phenyl]cyclopropyl]methyl]butanamid.

8. Verwendung einer Verbindung des Anspruchs 1 zur Herstellung eines Medikamentes zur Behandlung einer den Tagesrhythmus betreffenden Störung bei einem Patienten.

9. Arzneimittel zur Behandlung von den Tagesrhythmus betreffenden Störungen, das eine geeignete Menge eines pharmazeutisch verträglichen Trägers und eine therapeutische Menge einer Verbindung des Anspruchs 1 umfaßt.

10. Radioligand, der eine radiochemisch substituierte Verbindung des Anspruchs 1 umfaßt.

11. Radioligand, der eine radiojodierte Verbindung umfaßt, die von einer Verbindung des Anspruchs 1 abgeleitet ist.

12. Verbindung des Anspruchs 1, ausgewählt aus der Gruppe:
(*trans*)-N-[[2-(3-Chlorphenyl)cycloprop-1-yl]methyl]butanamid;
(*trans*)-N-[[2-(3-Bromphenyl)cycloprop-1-yl]methyl]butanamid;
(-)-(*trans*)-N-[[2-(3-Fluorphenyl)cyclopropyl]methyl]butanamid;
(*trans*)-N-[[2-(2,5-Difluorphenyl)cycloprop-1-yl]methyl]butanamid;
(*trans*)-N-[[2-(2-Brom-5-fluorphenyl)cycloprop-1-yl]methyl]butanamid;
(trans)-N-[[2-(3-Methylphenyl)cycloprop-1-yl]methyl]butanamid;
(*trans*)-N-[[2-(3-Ethylphenyl)cycloprop-1-yl]methyl]butanamid;
(*trans*)-N-[[2-[3-(Trifluormethoxy)phenyl]cycloprop-1-yl]methyl]butanamid;
(*cis*)-N-[3-(3-Methoxyphenyl)cyclohexyl]acetamid;
(*cis*)-N-[3-(3-Methoxyphenyl)cyclohexyl]-2-methylpropanamid;
(*cis*)-N-[3-(3-Methoxyphenyl)cyclohexyl]butanamid;
(*cis*)-N-Ethyl-N'-[3-(3-methoxyphenyl)cyclohexyl]harnstoff und
N-[3-(3-Methoxyphenyl)cyclopentyl]butanamid.

13. Verbindung nach Anspruch 1 zur Verwendung als therapeutischer Wirkstoff.
